# EUROPEAN PATENT SPECIFICATION

(11) **EP 3 180 034 B1**
(45) Date of publication and mention of the grant of the patent: **20.04.2022**
(21) Application number: 15832025.9
(22) Date of filing: 11.08.2015
(51) Int. Cl.: A61K 38/46, C12N 15/113, C07K 14/44, A61P 21/00

(54) **PREVENTION OF MUSCULAR DYSTROPHY BY CRISPR/CAS9-MEDIATED GENE EDITING**
VORBEUGUNG VON MUSKELDYSTROPHIE DURCH CRISPR/CAS9-VERMITTELTE GENEDITIERUNG
PRÉVENTION DE LA DYSTROPHIE MUSCULAIRE PAR ÉDITION DE GÈNE MÉDIÉE PAR CRISPR/CAS9

(30) Priority: 11.08.2014 US 201462035584 P
(43) Date of publication of application: 21.06.2017
(73) Proprietor: The Board of Regents of The University of Texas System, Austin, TX 78701 (US)
(72) Inventor: OLSON, Eric N., Dallas, TX 75390 (US); LONG, Chengzu, Dallas, TX 75390 (US); MCANALLY, John R., Dallas, TX 75390 (US); SHELTON, John M., Dallas, TX 75390 (US); BASSEL-DUBY, Rhonda, Dallas, TX 75390 (US)
(74) Representative: Potter Clarkson
(86) International application number: PCT/US2015/044639
(87) International publication number: WO 2016/025469

(56) References cited:
- US-A1- 2012 301 456
- US-A1- 2014 179 770
- HONGMEI?LISA LI ET AL: "Precise Correction of the Dystrophin Gene in Duchenne Muscular Dystrophy Patient Induced Pluripotent Stem Cells by TALEN and CRISPR-Cas9", STEM CELL REPORTS, vol. 4, no. 1, 1 January 2015 (2015-01-01) , pages 143-154, XP055233644, United States ISSN: 2213-6711, DOI: 10.1016/j.stemcr.2014.10.013
- Ousterout: "Genetic Correction of Duchenne Muscular Dystrophy by Multiplex CRISPR/Cas9-Based Gene Editing", Molecular Therapy, 1 May 2014 (2014-05-01), pages S196-S197, XP055457315, United States DOI: https://doi.org/10.1016/S1525-0016(16)3552 2-8 Retrieved from the Internet: URL:http://www.cell.com/molecular-therapy- family/molecular-therapy/pdf/S1525-0016(16 )35522-8.pdf [retrieved on 2018-03-07]
- LONG ET AL.: 'Prevention of muscular dystrophy in mice by CRISPR/Cas9-mediated editing of germline DNA' SCIENCE vol. 345, no. 6201, 05 September 2014, pages 1184 - 1188, XP055159130
- NATHWANI ET AL.: 'Long-term safety and efficacy following systemic administration of a self- complementary AAV vector encoding human FIX pseudotyped with serotype 5 and 8 capsid proteins.' MOL THER vol. 19, no. 5, 2011, pages 876 - 885, XP055405392
- DOENCH ET AL.: 'How to Design Your gRNA for CRISPR Genome Editing' ADDGENE'S BLOG, POST 10 April 2014, pages 1 - 9, XP055405399 Retrieved from the Internet: <URL:http://blog.addgene.org/how-to-design- your-gma-for-crispr-genome-editing> [retrieved on 2015-09-15]
- Patrick d. Hsu ET AL: "Development and Applications of CRISPR-Cas9 for Genome Engineering", CELL, vol. 157, no. 6, 1 June 2014 (2014-06-01), pages 1262-1278, XP055529223, AMSTERDAM, NL ISSN: 0092-8674, DOI: 10.1016/j.cell.2014.05.010
- Kotterman ET AL: "Engineering adeno-associated viruses for clinical gene therapy", Nat Rev Genet, vol. 15, no. 7, 1 July 2014 (2014-07-01), pages 445-451, XP055317129, DOI: 10.1038/nrg3742
- John Doench: "Addgene's Blog / Post How to Design Your gRNA for CRISPR Genome Editing", , 10 April 2014 (2014-04-10), pages 1-9, XP055405399, Addgene's blog post Retrieved from the Internet: URL:http://blog.addgene.org/how-to-design- your-gma-for-crispr-genome-editing [retrieved on 2017-09-11]

### SEQUENCE LISTING

<110> THE BOARD OF REGENTS OF THE UNIVERSITY OF TEXAS SYSTEM
<120> PREVENTION OF MUSCULAR DYSTROPHY BY CRISPR/CAS9-MEDIATED GENE EDITING
<130> UTFD.P2838WO
<140> Unknown
   <141> 2015-08-11
<150> US 62/035,584
   <151> 2014-08-11
<160> 182
<170> PatentIn version 3.5
<210> 1
   <211> 21
   <212> DNA
   <213> Mus musculus
<400> 1
   ttgaaagagc aacaaaatgg c 21
<210> 2
   <211> 21
   <212> DNA
   <213> Artificial sequence
<220>
   <223> Synthetic oligonucleotide
<400> 2
   ttgaaagagc aataaaatgg c 21
<210> 3
   <211> 30
   <212> DNA
   <213> Artificial sequence
<220>
   <223> Synthetic oligonucleotide
<400> 3
   agttctttga aagagcaata aaatggcttc 30
<210> 4
   <211> 30
   <212> DNA
   <213> Artificial sequence
<220>
   <223> Synthetic oligonucleotide
<400> 4
   agttctttaa aggagcagca gaatggcttc 30
<210> 5
   <211> 21
   <212> DNA
   <213> Artificial sequence
<220>
   <223> Synthetic oligonucleotide
<400> 5
   ttaaaggagc agcagaatgg c 21
<210> 6
   <211> 33
   <212> DNA
   <213> Artificial sequence
<220>
   <223> Synthetic oligonucleotide
<400> 6
   agttctttga aagagcaaca aaatggcttc aac 33
<210> 7
   <211> 33
   <212> DNA
   <213> Mus musculus
<400> 7
   gttgaagcca ttttgttgct ctttcaaaga act 33
<210> 8
   <211> 30
   <212> RNA
   <213> Artificial sequence
<220>
   <223> Synthetic oligonucleotide
<400> 8
   ucuuugaaag agcaacaaaa guuuuauuuu 30
<210> 9
   <211> 33
   <212> DNA
   <213> Artificial sequence
<220>
   <223> **Synthetic oligonucleotide**
<400> 9
   agttctttaa aggagcagca gaatggcttc aac 33
<210> 10
   <211> 33
   <212> DNA
   <213> Artificial sequence
<220>
   <223> Synthetic oligonucleotide
<220>
   <221> misc_feature
   <222> (16)..(16)
   <223> n is a, c, g, or t
<220>
   <221> misc_feature
   <222> (19)..(23)
   <223> n is a, c, g, or t
<220>
   <221> misc_feature
   <222> (25)..(33)
   <223> n is a, c, g, or t
<400> 10
   agttctttga aagagnaann nnntnnnnnn nnn 33
<210> 11
   <211> 78
   <212> DNA
   <213> Artificial sequence
<220>
   <223> Synthetic oligonucleotide
<400> 11
   agttctttga aagagcaact tctatttaat tttgaggctc tgcaaagttc tttaaaggag 60
   aagcagaatg gcttcaac 78
<210> 12
   <211> 29
   <212> DNA
   <213> Artificial sequence
<220>
   <223> Synthetic oligonucleotide
<400> 12
   agttctttga aagagaaaat ggcttcaac 29
<210> 13
   <211> 28
   <212> DNA
   <213> Artificial sequence
<220>
   <223> Synthetic oligonucleotide
<400> 13
   agttctttga aagagaaatg gcttcaac 28
<210> 14
   <211> 35
   <212> DNA
   <213> Artificial sequence
<220>
   <223> Synthetic oligonucleotide
<400> 14
   agttctttga aagagcaacc caaaatggct tcaac 35
<210> 15
   <211> 30
   <212> DNA
   <213> Artificial sequence
<220>
   <223> Synthetic oligonucleotide
<400> 15
   agttctttga aagagcaaaa tggcttcaac 30
<210> 16
   <211> 33
   <212> DNA
   <213> Artificial sequence
<220>
   <223> Synthetic oligonucleotide
<400> 16
   agttctttga aagagcaacg aaatggcttc aac 33
<210> 17
   <211> 33
   <212> DNA
   <213> Artificial sequence
<220>
   <223> Synthetic oligonucleotide
<400> 17
   agttctttga aagagcaaca aaacggcttc aac 33
<210> 18
   <211> 33
   <212> DNA
   <213> Artificial sequence
<220>
   <223> Synthetic oligonucleotide
<400> 18
   agttctttga aagagcaata aaatggcttc aac 33
<210> 19
   <211> 6
   <212> PRT
   <213> Artificial sequence
<220>
   <223> Synthetic oligonucleotide
<400> 19
   Ser Ser Leu Lys Glu Gln
   1               5
<210> 20
   <211> 11
   <212> PRT
   <213> Mus musculus
<400> 20
   Ser Ser Leu Lys Glu Gln Gln Asn Gly Phe Asn
   1               5                   10
<210> 21
   <211> 10
   <212> PRT
   <213> Artificial sequence
<220>
   <223> Synthetic oligonucleotide
<400> 21
   Ser Ser Leu Lys Glu Gln Asn Gly Phe Asn
   1 5 10
<210> 22
   <211> 21
   <212> DNA
   <213> Artificial sequence
<220>
   <223> Synthetic oligonucleotide
<400> 22
   agttctttga atggcttcaa c 21
<210> 23
   <211> 24
   <212> DNA
   <213> Artificial sequence
<220>
   <223> Synthetic oligonucleotide
<400> 23
   agttctttgc aaaatggctt caac 24
<210> 24
   <211> 18
   <212> DNA
   <213> Artificial sequence
<220>
   <223> Synthetic oligonucleotide
<400> 24
   agttctttga aagaacct 18
<210> 25
   <211> 30
   <212> DNA
   <213> Artificial sequence
<220>
   <223> Synthetic oligonucleotide
<400> 25
   agttctttga aagagcaaaa tggcttcaac 30
<210> 26
   <211> 33
   <212> DNA
   <213> Artificial sequence
<220>
   <223> Synthetic oligonucleotide
<400> 26
   agttctttga aagagtaata aaatggcttc aac 33
<210> 27
   <211> 48
   <212> DNA
   <213> Artificial sequence
<220>
   <223> Synthetic oligonucleotide
<400> 27
   ttgcaaaaac ccaaaatatt ttagctccta ctcagactgt tactctgg 48
<210> 28
   <211> 48
   <212> DNA
   <213> Artificial sequence
<220>
   <223> Synthetic oligonucleotide
<400> 28
   ccagagtaac agtctgagta ggagctaaaa tattttgggt ttttgcaa 48
<210> 29
   <211> 13
   <212> DNA
   <213> Artificial sequence
<220>
   <223> Synthetic oligonucleotide
<400> 29
   aaccccacag gtc 13
<210> 30
   <211> 13
   <212> DNA
   <213> Artificial sequence
<220>
   <223> Synthetic oligonucleotide
<400> 30
   gacctgtggg gtt 13
<210> 31
   <211> 23
   <212> DNA
   <213> Artificial sequence
<220>
   <223> Synthetic oligonucleotide
<400> 31
   ctggctccac ccttgacctg tgg 23
<210> 32
   <211> 23
   <212> DNA
   <213> Artificial sequence
<220>
   <223> Synthetic oligonucleotide
<400> 32
   tgaattatgg taatccccac agg 23
<210> 33
   <211> 20
   <212> DNA
   <213> Artificial sequence
<220>
   <223> Synthetic oligonucleotide
<400> 33
   tgcaaaaacc caaaatattt 20
<210> 34
   <211> 20
   <212> DNA
   <213> Artificial sequence
<220>
   <223> Synthetic oligonucleotide
<400> 34
   aaaatatttt agctcctact 20
<210> 35
   <211> 20
   <212> DNA
   <213> Artificial sequence
<220>
   <223> Synthetic oligonucleotide
<400> 35
   cagagtaaca gtctgagtag 20
<210> 36
   <211> 20
   <212> DNA
   <213> Artificial sequence
<220>
   <223> Synthetic oligonucleotide
<400> 36
   taagggatat ttgttcttac 20
<210> 37
   <211> 20
   <212> DNA
   <213> Artificial sequence
<220>
   <223> Synthetic oligonucleotide
<400> 37
   ctaagggata tttgttctta 20
<210> 38
   <211> 20
   <212> DNA
   <213> Artificial sequence
<220>
   <223> Synthetic oligonucleotide
<400> 38
   tgttcttaca ggcaacaatg 20
<210> 39
   <211> 20
   <212> DNA
   <213> Artificial sequence
<220>
   <223> Synthetic oligonucleotide
<400> 39
   tgtatgcttt tctgttaaag 20
<210> 40
   <211> 20
   <212> DNA
   <213> Artificial sequence
<220>
   <223> Synthetic oligonucleotide
<400> 40
   atgtgtatgc ttttctgtta 20
<210> 41
   <211> 20
   <212> DNA
   <213> Artificial sequence
<220>
   <223> Synthetic oligonucleotide
<400> 41
   gtgtatgctt ttctgttaaa 20
<210> 42
   <211> 20
   <212> DNA
   <213> Artificial sequence
<220>
   <223> Synthetic oligonucleotide
<400> 42
   ttgccttttt ggtatcttac 20
<210> 43
   <211> 20
   <212> DNA
   <213> Artificial sequence
<220>
   <223> Synthetic oligonucleotide
<400> 43
   tttgcctttt tggtatctta 20
<210> 44
   <211> 20
   <212> DNA
   <213> Artificial sequence
<220>
   <223> Synthetic oligonucleotide
<400> 44
   cgctgcccaa tgccatcctg 20
<210> 45
   <211> 20
   <212> DNA
   <213> Artificial sequence
<220>
   <223> Synthetic oligonucleotide
<400> 45
   atttattttt ccttttattc 20
<210> 46
   <211> 20
   <212> DNA
   <213> Artificial sequence
<220>
   <223> Synthetic oligonucleotide
<400> 46
   tttcctttta ttctagttga 20
<210> 47
   <211> 20
   <212> DNA
   <213> Artificial sequence
<220>
   <223> Synthetic oligonucleotide
<400> 47
   tgattctgaa ttctttcaac 20
<210> 48
   <211> 20
   <212> DNA
   <213> Artificial sequence
<220>
   <223> Synthetic oligonucleotide
<400> 48
   atccatatgc ttttacctgc 20
<210> 49
   <211> 20
   <212> DNA
   <213> Artificial sequence
<220>
   <223> Synthetic oligonucleotide
<400> 49
   gatccatatg cttttacctg 20
<210> 50
   <211> 20
   <212> DNA
   <213> Artificial sequence
<220>
   <223> Synthetic oligonucleotide
<400> 50
   cagatctgtc aaatcgcctg 20
<210> 51
   <211> 20
   <212> DNA
   <213> Artificial sequence
<220>
   <223> Synthetic oligonucleotide
<400> 51
   ttattcttct ttctccaggc 20
<210> 52
   <211> 20
   <212> DNA
   <213> Artificial sequence
<220>
   <223> Synthetic oligonucleotide
<400> 52
   aattttattc ttctttctcc 20
<210> 53
   <211> 20
   <212> DNA
   <213> Artificial sequence
<220>
   <223> Synthetic oligonucleotide
<400> 53
   caattttatt cttctttctc 20
<210> 54
   <211> 20
   <212> DNA
   <213> Artificial sequence
<220>
   <223> Synthetic oligonucleotide
<400> 54
   gttttaaaat ttttatatta 20
<210> 55
   <211> 20
   <212> DNA
   <213> Artificial sequence
<220>
   <223> Synthetic oligonucleotide
<400> 55
   ttttatatta cagaatataa 20
<210> 56
   <211> 20
   <212> DNA
   <213> Artificial sequence
<220>
   <223> Synthetic oligonucleotide
<400> 56
   atattacaga atataaaaga 20
<210> 57
   <211> 20
   <212> DNA
   <213> Artificial sequence
<220>
   <223> Synthetic oligonucleotide
<400> 57
   tgtgtatgtg tatgtgtttt 20
<210> 58
   <211> 20
   <212> DNA
   <213> Artificial sequence
<220>
   <223> Synthetic oligonucleotide
<400> 58
   tatgtgtatg tgttttaggc 20
<210> 59
   <211> 20
   <212> DNA
   <213> Artificial sequence
<220>
   <223> Synthetic oligonucleotide
<400> 59
   ctattccagt caaataggtc 20
<210> 60
   <211> 20
   <212> DNA
   <213> Artificial sequence
<220>
   <223> Synthetic oligonucleotide
<400> 60
   gtgtagtgtt aatgtgctta 20
<210> 61
   <211> 20
   <212> DNA
   <213> Artificial sequence
<220>
   <223> Synthetic oligonucleotide
<400> 61
   ggacttctta tctggatagg 20
<210> 62
   <211> 20
   <212> DNA
   <213> Artificial sequence
<220>
   <223> Synthetic oligonucleotide
<400> 62
   taggtggtat caacatctgt 20
<210> 63
   <211> 20
   <212> DNA
   <213> Artificial sequence
<220>
   <223> Synthetic oligonucleotide
<400> 63
   tgaaaattta tttccacatg 20
<210> 64
   <211> 20
   <212> DNA
   <213> Artificial sequence
<220>
   <223> Synthetic oligonucleotide
<400> 64
   gaaaatttat ttccacatgt 20
<210> 65
   <211> 20
   <212> DNA
   <213> Artificial sequence
<220>
   <223> Synthetic oligonucleotide
<400> 65
   ttacattttt gacctacatg 20
<210> 66
   <211> 20
   <212> DNA
   <213> Artificial sequence
<220>
   <223> Synthetic oligonucleotide
<400> 66
   tgaacatttg gtcctttgca 20
<210> 67
   <211> 20
   <212> DNA
   <213> Artificial sequence
<220>
   <223> Synthetic oligonucleotide
<400> 67
   tctgaacatt tggtcctttg 20
<210> 68
   <211> 20
   <212> DNA
   <213> Artificial sequence
<220>
   <223> Synthetic oligonucleotide
<400> 68
   tctcgctcac tcaccctgca 20
<210> 69
   <211> 20
   <212> DNA
   <213> Artificial sequence
<220>
   <223> Synthetic oligonucleotide
<400> 69
   aaagaaaatc acagaaacca 20
<210> 70
   <211> 20
   <212> DNA
   <213> Artificial sequence
<220>
   <223> Synthetic oligonucleotide
<400> 70
   aaaatcacag aaaccaaggt 20
<210> 71
   <211> 20
   <212> DNA
   <213> Artificial sequence
<220>
   <223> Synthetic oligonucleotide
<400> 71
   ggtatctttg atactaacct 20
<210> 72
   <211> 20
   <212> DNA
   <213> Artificial sequence
<220>
   <223> Synthetic oligonucleotide
<400> 72
   tatgtgttac ctacccttgt 20
<210> 73
   <211> 20
   <212> DNA
   <213> Artificial sequence
<220>
   <223> Synthetic oligonucleotide
<400> 73
   aaatgtacaa ggaccgacaa 20
<210> 74
   <211> 20
   <212> DNA
   <213> Artificial sequence
<220>
   <223> Synthetic oligonucleotide
<400> 74
   gtacaaggac cgacaagggt 20
<210> 75
   <211> 20
   <212> DNA
   <213> Artificial sequence
<220>
   <223> Synthetic oligonucleotide
<400> 75
   tgcactattc tcaacaggta 20
<210> 76
   <211> 20
   <212> DNA
   <213> Artificial sequence
<220>
   <223> Synthetic oligonucleotide
<400> 76
   tcaaatgcac tattctcaac 20
<210> 77
   <211> 20
   <212> DNA
   <213> Artificial sequence
<220>
   <223> Synthetic oligonucleotide
<400> 77
   ctttacacac tttacctgtt 20
<210> 78
   <211> 20
   <212> DNA
   <213> Artificial sequence
<220>
   <223> Synthetic oligonucleotide
<400> 78
   atgctctcat ccatagtcat 20
<210> 79
   <211> 20
   <212> DNA
   <213> Artificial sequence
<220>
   <223> Synthetic oligonucleotide
<400> 79
   tctcatccat agtcataggt 20
<210> 80
   <211> 20
   <212> DNA
   <213> Artificial sequence
<220>
   <223> Synthetic oligonucleotide
<400> 80
   catccatagt cataggtaag 20
<210> 81
   <211> 180
   <212> DNA
   <213> Artificial sequence
<220>
   <223> Synthetic oligonucleotide
<400> 81
   tgatatgaat gaaactcatc aaatatgcgt gttagtgtaa atgaacttct atttaatttt 60
   gaggctctgc aaagttcttt aaaggagcag cagaatggct tcaactatct gagtgacact 120
   gtgaaggaga tggccaagaa agcaccttca gaaatatgcc agaaatatct gtcagaattt 180
<210> 82
   <211> 27
   <212> DNA
   <213> Artificial sequence
<220>
   <223> Synthetic primer
<400> 82
   gagaaacttc tgtgatgtga ggacata 27
<210> 83
   <211> 27
   <212> DNA
   <213> Artificial sequence
<220>
   <223> Synthetic primer
<400> 83
   caatatcttt gaaggactct gggtaaa 27
<210> 84
   <211> 25
   <212> DNA
   <213> Artificial sequence
<220>
   <223> Synthetic primer
<400> 84
   cttctattta attttgaggc tctgc 25
<210> 85
   <211> 25
   <212> DNA
   <213> Artificial sequence
<220>
   <223> Synthetic primer
<400> 85
   cctgaaattt tcgaagttta ttcat 25
<210> 86
   <211> 25
   <212> DNA
   <213> Artificial sequence
<220>
   <223> Synthetic primer
<400> 86
   tatgccactt cttcaaagag atgat 25
<210> 87
   <211> 25
   <212> DNA
   <213> Artificial sequence
<220>
   <223> Synthetic primer
<400> 87
   aacaagcaaa caattcaaag gatag 25
<210> 88
   <211> 23
   <212> DNA
   <213> Artificial sequence
<220>
   <223> Synthetic primer
<400> 88
   aagaagatat ggcattgctg gta 23
<210> 89
   <211> 21
   <212> DNA
   <213> Artificial sequence
<220>
   <223> Synthetic primer
<400> 89
   tctggaaaca aaaaggcaat g 21
<210> 90
   <211> 25
   <212> DNA
   <213> Artificial sequence
<220>
   <223> Synthetic primer
<400> 90
   taagagttct gacatgattt ccaca 25
<210> 91
   <211> 25
   <212> DNA
   <213> Artificial sequence
<220>
   <223> Synthetic primer
<400> 91
   tggaacacta ctctctacac tgtgc 25
<210> 92
   <211> 25
   <212> DNA
   <213> Artificial sequence
<220>
   <223> Synthetic primer
<400> 92
   ctatgagttt accaccctaa tgtgc 25
<210> 93
   <211> 24
   <212> DNA
   <213> Artificial sequence
<220>
   <223> Synthetic primer
<400> 93
   cttatgcttg ttcaggcaaa tacc 24
<210> 94
   <211> 25
   <212> DNA
   <213> Artificial sequence
<220>
   <223> Synthetic primer
<400> 94
   ttttgagttg tgttcatttt ctgag 25
<210> 95
   <211> 25
   <212> DNA
   <213> Artificial sequence
<220>
   <223> Synthetic primer
<400> 95
   taggagtaca gctgcttctt cagac 25
<210> 96
   <211> 25
   <212> DNA
   <213> Artificial sequence
<220>
   <223> Synthetic primer
<400> 96
   gaaaaacaaa attactgagg catgt 25
<210> 97
   <211> 25
   <212> DNA
   <213> Artificial sequence
<220>
   <223> Synthetic primer
<400> 97
   cctccaagtt cttatcttgt ttgaa 25
<210> 98
   <211> 25
   <212> DNA
   <213> Artificial sequence
<220>
   <223> Synthetic primer
<400> 98
   agtgattttc tgatgaccca aatta 25
<210> 99
   <211> 25
   <212> DNA
   <213> Artificial sequence
<220>
   <223> Synthetic primer
<400> 99
   tgtttttaat ggctaggtgc taatc 25
<210> 100
   <211> 25
   <212> DNA
   <213> Artificial sequence
<220>
   <223> Synthetic primer
<400> 100
   tttcttggag ctgtagtgtg tactg 25
<210> 101
   <211> 25
   <212> DNA
   <213> Artificial sequence
<220>
   <223> Synthetic primer
<400> 101
   ggaatagagt gagcattgtt ctgat 25
<210> 102
   <211> 25
   <212> DNA
   <213> Artificial sequence
<220>
   <223> Synthetic primer
<400> 102
   tgtcacagtt gcaattctta gtgtt 25
<210> 103
   <211> 25
   <212> DNA
   <213> Artificial sequence
<220>
   <223> Synthetic primer
<400> 103
   cttagaaaaa caaggttcct gacaa 25
<210> 104
   <211> 25
   <212> DNA
   <213> Artificial sequence
<220>
   <223> Synthetic primer
<400> 104
   caataaggac aagtgaaggc taaaa 25
<210> 105
   <211> 25
   <212> DNA
   <213> Artificial sequence
<220>
   <223> Synthetic primer
<400> 105
   aggtctccac acatattcac tcttc 25
<210> 106
   <211> 24
   <212> DNA
   <213> Artificial sequence
<220>
   <223> Synthetic primer
<400> 106
   agatctggga gcttctatca actg 24
<210> 107
   <211> 25
   <212> DNA
   <213> Artificial sequence
<220>
   <223> Synthetic primer
<400> 107
   gggtagaagt gaatcaataa gtgga 25
<210> 108
   <211> 25
   <212> DNA
   <213> Artificial sequence
<220>
   <223> Synthetic primer
<400> 108
   gaacacttct ttgcttctca tcact 25
<210> 109
   <211> 25
   <212> DNA
   <213> Artificial sequence
<220>
   <223> Synthetic primer
<400> 109
   gctgagacta ctgtagccct ttaga 25
<210> 110
   <211> 25
   <212> DNA
   <213> Artificial sequence
<220>
   <223> Synthetic primer
<400> 110
   tagtttttca cattcagtcc agctt 25
<210> 111
   <211> 25
   <212> DNA
   <213> Artificial sequence
<220>
   <223> Synthetic primer
<400> 111
   gctttcaaaa ctacaccaaa cctac 25
<210> 112
   <211> 25
   <212> DNA
   <213> Artificial sequence
<220>
   <223> Synthetic primer
<400> 112
   ctttaaaata caagcctcca gttcc 25
<210> 113
   <211> 25
   <212> DNA
   <213> Artificial sequence
<220>
   <223> Synthetic primer
<400> 113
   tatttgtttc tcaaatttcc agacc 25
<210> 114
   <211> 25
   <212> DNA
   <213> Artificial sequence
<220>
   <223> Synthetic primer
<400> 114
   attttctaga ggtggtctca cacac 25
<210> 115
   <211> 25
   <212> DNA
   <213> Artificial sequence
<220>
   <223> Synthetic primer
<400> 115
   gaaaagtgga tagacagttt cagga 25
<210> 116
   <211> 25
   <212> DNA
   <213> Artificial sequence
<220>
   <223> Synthetic primer
<400> 116
   aacctaaaag aaaggacaag gagaa 25
<210> 117
   <211> 25
   <212> DNA
   <213> Artificial sequence
<220>
   <223> Synthetic primer
<400> 117
   acatgactcg gtaataaacc ttgag 25
<210> 118
   <211> 25
   <212> DNA
   <213> Artificial sequence
<220>
   <223> Synthetic primer
<400> 118
   ttgtaaaagt tccaactccc agtag 25
<210> 119
   <211> 25
   <212> DNA
   <213> Artificial sequence
<220>
   <223> Synthetic primer
<400> 119
   tttaaaatct atttccccag agagg 25
<210> 120
   <211> 24
   <212> DNA
   <213> Artificial sequence
<220>
   <223> Synthetic primer
<400> 120
   tgtccatttt taacctgtgt tctg 24
<210> 121
   <211> 25
   <212> DNA
   <213> Artificial sequence
<220>
   <223> Synthetic primer
<400> 121
   ccctaactca gtttctcttg ttctg 25
<210> 122
   <211> 25
   <212> DNA
   <213> Artificial sequence
<220>
   <223> Synthetic primer
<400> 122
   atctgtgttt tcaatgtgga atctt 25
<210> 123
   <211> 25
   <212> DNA
   <213> Artificial sequence
<220>
   <223> Synthetic primer
<400> 123
   agaaagcgaa taggatttct tgttt 25
<210> 124
   <211> 25
   <212> DNA
   <213> Artificial sequence
<220>
   <223> Synthetic primer
<400> 124
   tcgaatcttc tacaatatgc aatca 25
<210> 125
   <211> 25
   <212> DNA
   <213> Artificial sequence
<220>
   <223> Synthetic primer
<400> 125
   gtgggaaatg tttcaagtat cacat 25
<210> 126
   <211> 25
   <212> DNA
   <213> Artificial sequence
<220>
   <223> Synthetic primer
<400> 126
   gcaaaataca acttctaagc aaacc 25
<210> 127
   <211> 25
   <212> DNA
   <213> Artificial sequence
<220>
   <223> Synthetic primer
<400> 127
   ccagaccaga ggtagagtgt ttcta 25
<210> 128
   <211> 25
   <212> DNA
   <213> Artificial sequence
<220>
   <223> Synthetic primer
<400> 128
   caggagtcag cctcttactt tacaa 25
<210> 129
   <211> 25
   <212> DNA
   <213> Artificial sequence
<220>
   <223> Synthetic primer
<400> 129
   gctagatgac aaagccactt aactc 25
<210> 130
   <211> 25
   <212> DNA
   <213> Artificial sequence
<220>
   <223> Synthetic primer
<400> 130
   gctacagaaa agaggctagg aaagt 25
<210> 131
   <211> 25
   <212> DNA
   <213> Artificial sequence
<220>
   <223> Synthetic primer
<400> 131
   gctttgaaga tgccctagaa atact 25
<210> 132
   <211> 25
   <212> DNA
   <213> Artificial sequence
<220>
   <223> Synthetic primer
<400> 132
   taatacataa ggggacatca cgagt 25
<210> 133
   <211> 25
   <212> DNA
   <213> Artificial sequence
<220>
   <223> Synthetic primer
<400> 133
   gatctttgta gtggtttttc tcctg 25
<210> 134
   <211> 25
   <212> DNA
   <213> Artificial sequence
<220>
   <223> Synthetic primer
<400> 134
   ttaagcggaa agataagctg aagta 25
<210> 135
   <211> 25
   <212> DNA
   <213> Artificial sequence
<220>
   <223> Synthetic primer
<400> 135
   ggaccaatgt tactggaaca catac 25
<210> 136
   <211> 24
   <212> DNA
   <213> Artificial sequence
<220>
   <223> Synthetic primer
<400> 136
   cttctacatt cacctccctg tgtt 24
<210> 137
   <211> 25
   <212> DNA
   <213> Artificial sequence
<220>
   <223> Synthetic primer
<400> 137
   cccagcatct aagaaaggag taata 25
<210> 138
   <211> 25
   <212> DNA
   <213> Artificial sequence
<220>
   <223> Synthetic primer
<400> 138
   aaatttttag tcaaaagtgc ttgga 25
<210> 139
   <211> 25
   <212> DNA
   <213> Artificial sequence
<220>
   <223> Synthetic primer
<400> 139
   caataaacct ttcagacttc attgg 25
<210> 140
   <211> 25
   <212> DNA
   <213> Artificial sequence
<220>
   <223> Synthetic primer
<400> 140
   tatgatttcc agggtaagtc cacta 25
<210> 141
   <211> 25
   <212> DNA
   <213> Artificial sequence
<220>
   <223> Synthetic primer
<400> 141
   gcacttttgc taacatctaa attcc 25
<210> 142
   <211> 25
   <212> DNA
   <213> Artificial sequence
<220>
   <223> Synthetic primer
<400> 142
   aaagtatatc tgagaatgcc actgc 25
<210> 143
   <211> 25
   <212> DNA
   <213> Artificial sequence
<220>
   <223> Synthetic primer
<400> 143
   gtagctgtag gaatgtctgt cctgt 25
<210> 144
   <211> 25
   <212> DNA
   <213> Artificial sequence
<220>
   <223> Synthetic primer
<400> 144
   tgtaataaaa tgagaatttg cacca 25
<210> 145
   <211> 25
   <212> DNA
   <213> Artificial sequence
<220>
   <223> Synthetic primer
<400> 145
   aatgaagcca aggtacatac aaaga 25
<210> 146
   <211> 25
   <212> DNA
   <213> Artificial sequence
<220>
   <223> Synthetic primer
<400> 146
   catgaagata cagaaacatc ccagt 25
<210> 147
   <211> 19
   <212> DNA
   <213> Artificial sequence
<220>
   <223> Synthetic primer
<400> 147
   ggagtggcac cctccttac 19
<210> 148
   <211> 25
   <212> DNA
   <213> Artificial sequence
<220>
   <223> Synthetic primer
<400> 148
   ataccccaag ccatacttgt atcat 25
<210> 149
   <211> 25
   <212> DNA
   <213> Artificial sequence
<220>
   <223> Synthetic primer
<400> 149
   cacttatcca tctaggaaag cagag 25
<210> 150
   <211> 23
   <212> DNA
   <213> Mus musculus
<400> 150
   tctttgaaag agcaacaaaa tgg 23
<210> 151
   <211> 23
   <212> DNA
   <213> Mus musculus
<400> 151
   tttttgaaag agcaacaata agg 23
<210> 152
   <211> 23
   <212> DNA
   <213> Mus musculus
<400> 152
   tttttgaaag atcaacaaaa tag 23
<210> 153
   <211> 23
   <212> DNA
   <213> Mus musculus
<400> 153
   tctgtgaaag agtaacaaaa tgg 23
<210> 154
   <211> 23
   <212> DNA
   <213> Mus musculus
<400> 154
   tcattgaaag agcaacacaa ggg 23
<210> 155
   <211> 23
   <212> DNA
   <213> Mus musculus
<400> 155
   tctgagaaat agcaacaaaa ggg 23
<210> 156
   <211> 23
   <212> DNA
   <213> Mus musculus
<400> 156
   tcttttaaag agcaacaata tgg 23
<210> 157
   <211> 23
   <212> DNA
   <213> Mus musculus
<400> 157
   tctttgaaat aggaacaaaa cag 23
<210> 158
   <211> 23
   <212> DNA
   <213> Mus musculus
<400> 158
   gctgtgaaag agcaacaaac aag 23
<210> 159
   <211> 23
   <212> DNA
   <213> Mus musculus
<400> 159
   tatttaaaaa agcaacaaaa aag 23
<210> 160
   <211> 23
   <212> DNA
   <213> Mus musculus
<400> 160
   tctttgaaag tccaacaaaa gag 23
<210> 161
   <211> 23
   <212> DNA
   <213> Mus musculus
<400> 161
   actttgaaaa agcaacacaa aag 23
<210> 162
   <211> 23
   <212> DNA
   <213> Mus musculus
<400> 162
   tctttgagag aacaacaaac agg 23
<210> 163
   <211> 23
   <212> DNA
   <213> Mus musculus
<400> 163
   tctttgacag agaaacaaac agg 23
<210> 164
   <211> 23
   <212> DNA
   <213> Mus musculus
<400> 164
   attttcaatg agcaacaaaa tgg 23
<210> 165
   <211> 23
   <212> DNA
   <213> Mus musculus
<400> 165
   aatttaaaag agaaacaaaa tag 23
<210> 166
   <211> 23
   <212> DNA
   <213> Mus musculus
<400> 166
   tgtttgaacc agcaacaaat gag 23
<210> 167
   <211> 23
   <212> DNA
   <213> Mus musculus
<400> 167
   tttttgaaag agaagcaaaa tag 23
<210> 168
   <211> 23
   <212> DNA
   <213> Mus musculus
<400> 168
   cctttgagag aacaacaaac agg 23
<210> 169
   <211> 23
   <212> DNA
   <213> Mus musculus
<400> 169
   tttatgaaac agcaacagaa aag 23
<210> 170
   <211> 23
   <212> DNA
   <213> Mus musculus
<400> 170
   tgttagaatg agcaacaata cag 23
<210> 171
   <211> 23
   <212> DNA
   <213> Mus musculus
<400> 171
   tatttaaaat aggaacaaaa aag 23
<210> 172
   <211> 23
   <212> DNA
   <213> Mus musculus
<400> 172
   tcatagaaag agcaaccaat cag 23
<210> 173
   <211> 23
   <212> DNA
   <213> Mus musculus
<400> 173
   tcttggaaag aggaaaaaaa ggg 23
<210> 174
   <211> 23
   <212> DNA
   <213> Mus musculus
<400> 174
   tgtttgtaag ggaaacaaaa ggg 23
<210> 175
   <211> 23
   <212> DNA
   <213> Mus musculus
<400> 175
   tctttcaagc agaaacaaaa cag 23
<210> 176
   <211> 23
   <212> DNA
   <213> Mus musculus
<400> 176
   tctgtgaaac agtaactaaa cgg 23
<210> 177
   <211> 23
   <212> DNA
   <213> Mus musculus
<400> 177
   tctttgaaag agtatctaaa aag 23
<210> 178
   <211> 23
   <212> DNA
   <213> Mus musculus
<400> 178
   tatatgaaag agccacaaga tgg 23
<210> 179
   <211> 23
   <212> DNA
   <213> Mus musculus
<400> 179
   tattagaaag agaaagaaaa gag 23
<210> 180
   <211> 23
   <212> DNA
   <213> Mus musculus
<400> 180
   tcactgaaag agcaaagaaa gag 23
<210> 181
   <211> 23
   <212> DNA
   <213> Mus musculus
<400> 181
   tctctgaagg aacaacaaca aag 23
<210> 182
   <211> 23
   <212> DNA
   <213> Mus musculus
<400> 182
   tctttacaag atcatcaaaa aag 23

## Description

### BACKGROUND

### 1. Field

The present disclosure relates to the fields of molecular biology, medicine and genetics. More particularly, the disclosure relates to the use of genome editing to treat Duchenne muscular dystrophy (DMD).

### 2. Related Art

Duchenne muscular dystrophy (DMD) is caused by mutations in the gene for dystrophin on the X chromosome and affects approximately 1 in 3,500 boys. Dystrophin is a large cytoskeletal structural protein essential for muscle cell membrane integrity. Without it, muscles degenerate, causing weakness and myopathy (Fairclough *et al.,* 2013). Death of DMD patients usually occurs by age 25, typically from breathing complications and cardiomyopathy. Hence, therapy for DMD necessitates sustained rescue of skeletal, respiratory and cardiac muscle structure and function. Although the genetic cause of DMD was identified nearly three decades ago (Worton *et al.,* 1988), and several gene- and cellbased therapies have been developed to deliver functional *Dmd* alleles or dystrophin-like protein to diseased muscle tissue, numerous therapeutic challenges have been encountered and no curative treatment exists (Van Deutekom and Van Ommen, 2003).

RNA-guided nucleases-mediated genome editing, based on Type II CRISPR (Clustered Regularly Interspaced Short Palindromic Repeat)/Cas (CRISPR Associated) systems, offers a new approach to alter the genome (Jinek *et al.,* 2012; Cong *et al.,* 2013 and Mali *et al.,* 2013a). In brief, Cas9, a nuclease guided by single-guide RNA (sgRNA), binds to a targeted genomic locus next to the protospacer adjacent motif (PAM) and generates a double-strand break (DSB). The DSB is then repaired either by non-homologous end-joining (NHEJ), which leads to insertion/deletion (indel) mutations, or by homology-directed repair (HDR), which requires an exogenous template and can generate a precise modification at a target locus (Mali *et al.,* 2013b). Unlike other gene therapy methods, which add a functional, or partially functional, copy of a gene to a patient's cells but retain the original dysfunctional copy of the gene, this system can remove the defect. Genetic correction using engineered nucleases (Urnov *et al.,* 2005; Ousterout *et al.,* 2013; Osborn *et al.,* 2013; Wu *et al.,* 2013 and Schwank *et al.,* 2013) has been demonstrated in tissue culture cells (Schwank *et al.,* 2013) and rodent models of rare diseases (Yin *et al.,* 2014), but not yet in models of relatively common and currently incurable diseases, such as DMD.

### SUMMARY

Thus, in accordance with the present disclosure, there is provided a method of correcting a dystrophin gene defect in a subject comprising contacting a cell in said subject with Cas9 and a DMD guide RNA. The cell may be a muscle cell, a satellite cell, or an iPSC/iCM. The Cas9 and/or DMD guide RNA may be provided to said cell through expression from one or more expression vectors coding therefor, such as a viral vector (e.g., an adeno-associated viral vector) or a non-viral vector. The Cas9 may be provided to said cell as naked plasmid DNA or chemically-modified mRNA. The method may further comprise contacting said cell with a single-stranded DMD oligonucleotide to effect homology directed repair. The method may further comprise designing a dystrophin gene target based on reference to a Duchenne mutation database, such as the Duchenne Skipper Database.

The Cas9, DMD guide RNA and/or single-stranded DMD oligonucleotide, or expression vectors coding therefor, may be provided to said cell in one or more nanoparticles. The Cas9, DMD guide RNA and/or single-stranded DMD oligonucleotide may be delivered directly to a muscle tissue, such as tibialis anterior, quadricep, soleus, diaphragm or heart. The Cas9, DMD guide RNA and/or single-stranded DMD oligonucleotide may be delivered systemically. The correction may be permanent skipping of a mutant exon or more than one exon. The subject may exhibit normal dystrophin-positive myofibers and/or mosaic dystrophin-positive myofibers containing centralized nuclei. The subject may exhibit a decreased serum CK level as compared to a serum CK level prior to contacting. The treated subject may exhibit improved grip strength as compared to a serum CK level prior to contacting.

It is contemplated that any method or composition described herein can be implemented with respect to any other method or composition described herein.

Other objects, features and advantages of the present disclosure will become apparent from the following detailed description.

### BRIEF DESCRIPTION OF THE DRAWINGS

The following drawings form part of the present specification and are included to further demonstrate certain aspects of the present disclosure. The disclosure may be better understood by reference to one or more of these drawings in combination with the detailed description.

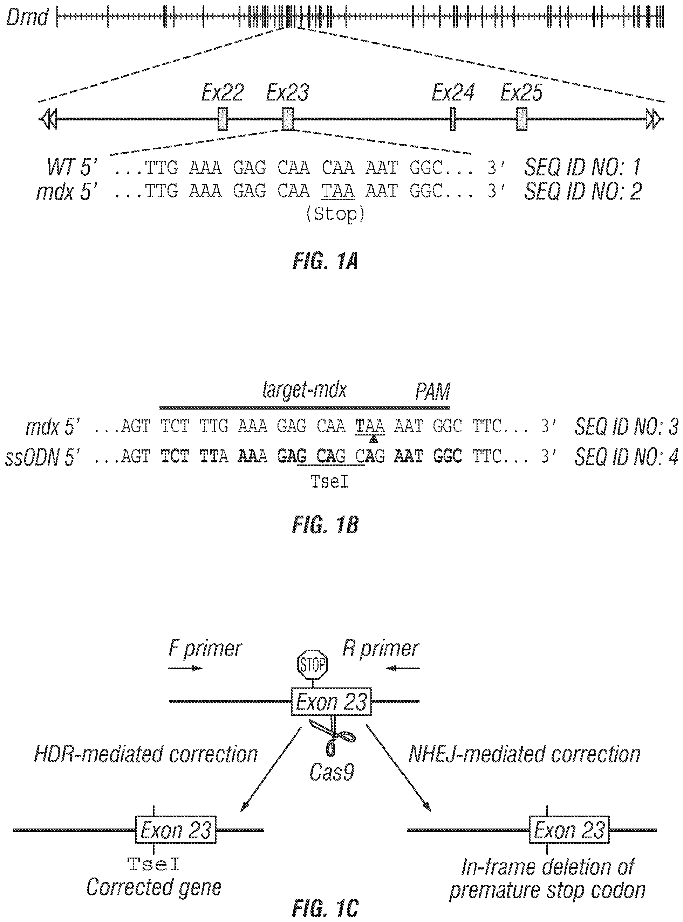

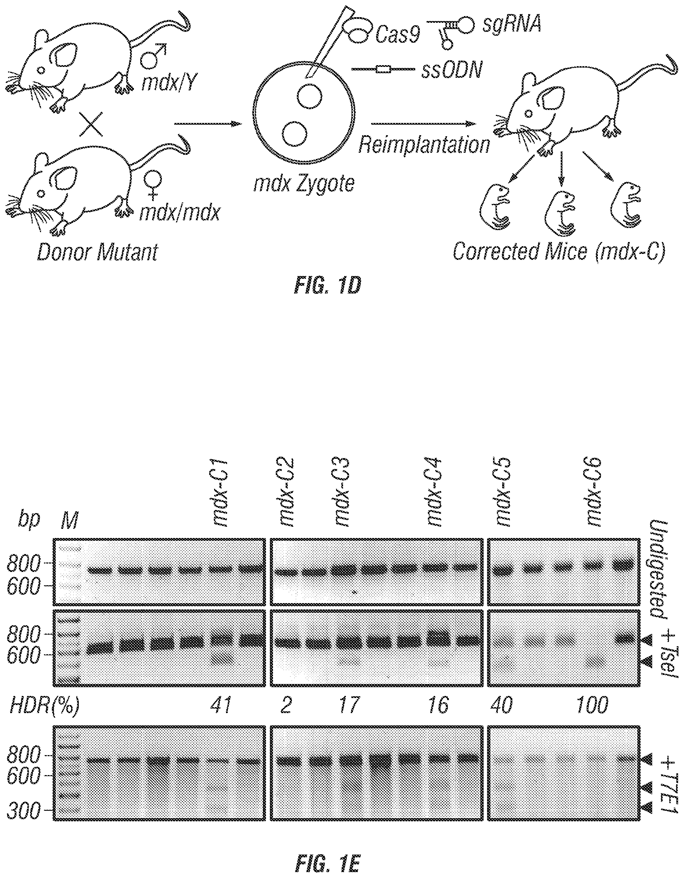

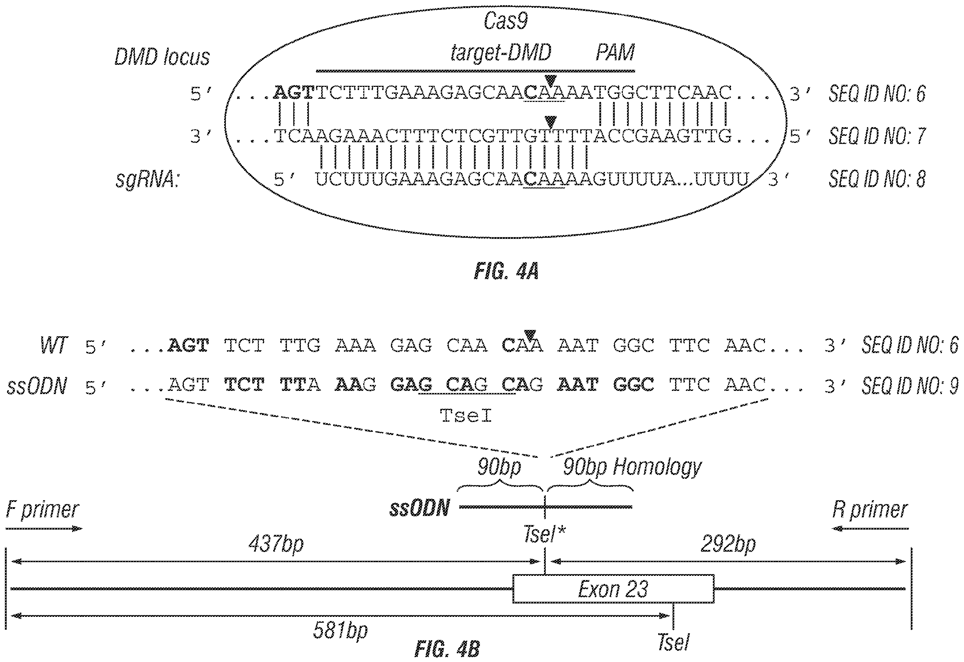

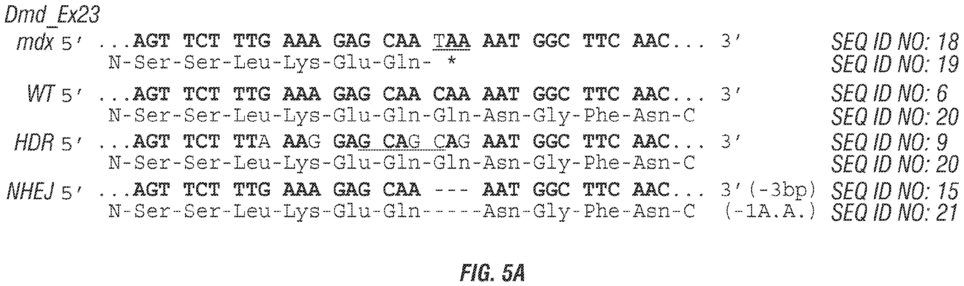

**FIGS. 1A**-E. CRISPR/Cas9-mediated ***Dmd* correction in *mdx* mice. (****FIG. 1A****)** Schematic of the targeted exon of mouse *Dmd* and sequence from wild-type (upper) and *mdx* mice (lower). The premature stop codon with the *mdx* point mutation (C to T) is underlined. **(****FIG. 1B****)** Schematic of the 20-nt sgRNA target sequence of the *mdx* allele (upper) and the PAM. The arrowhead indicates Cas9 cleavage site. ssODN, which contains 90 bp of homology sequence flanking each side of the target site was used as HDR template. ssODN incorporates four silent mutations (grey) and adds a TseI restriction enzyme site (underlined) for genotyping and quantification of HDR-mediated gene editing **(****FIG. 4B****). (****FIG. 1C****)** Schematic for the gene correction by HDR or NHEJ. The corresponding DNA and protein sequences are shown in **FIG. 5A****. (****FIG. 1D****)** Strategy of the gene correction in *mdx* mice via germ line gene therapy. **(FIG. IE)** Genotyping results of *mdx-C* mice with mosaicism of 2-100% corrected *Dmd* gene. Undigested PCR product (upper panel), TseI digestion (middle panel) and T7E1 digestion (lower panel) on a 2% agarose gel. The upper arrowhead in the middle panel marks the DNA band indicating HDR-mediated correction generated by TseI digestion. The lower arrowhead marks the DNA band of the uncorrected *mdx* allele. The relative intensity of the DNA bands (indicated by lower and upper arrowheads) reflects the percentage of HDR in the genomic DNA. The percent of HDR is located under the middle panel. The band intensity was quantified by ImageJ (NIH). The lower and upper arrowheads in the lower panel indicate uncut and cut bands by T7E1. M denotes size marker lane. bp indicates the base pair length of the marker bands.

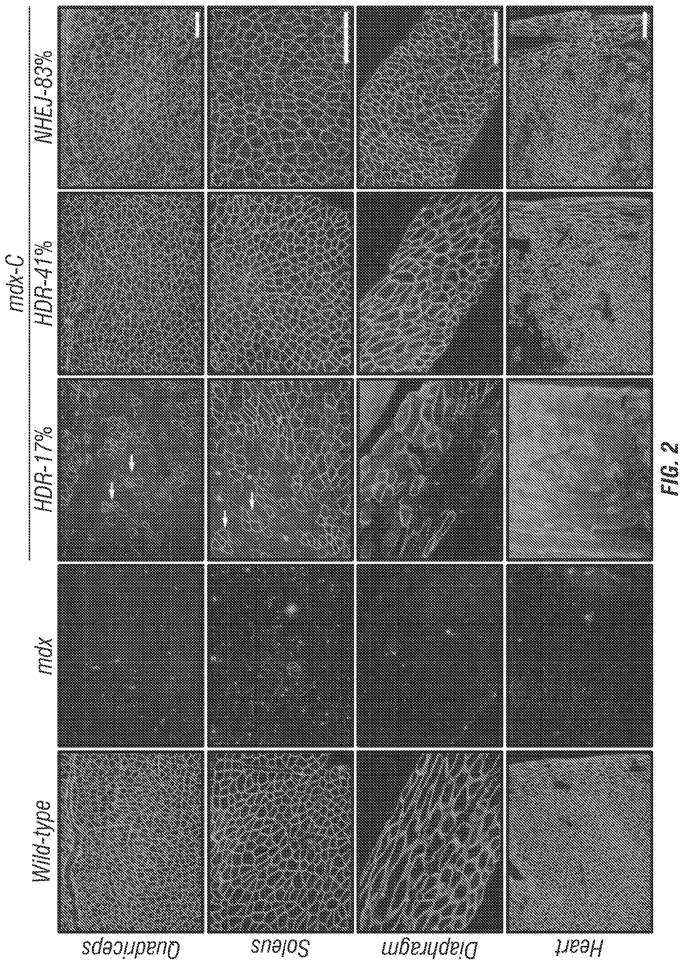

**FIG. 2****. Histological analysis of muscles from wild-type, *mdx* and *mdx-C* mice.** Immunostaining and histological analysis of muscles from 7-9 week old wild-type, *mdx* and *mdx-C* mice (HDR-17%, HDR-41% or NHEJ-83%). Dystrophin immunofluorescence in wild-type mice is present in all muscles, including quadriceps, soleus, diaphragm and heart and is absent in *mdx* mice, except for a single revertant fiber in skeletal muscle. Skeletal muscle from the HDR-17% mouse has a unique pattern of clusters of dystrophin-positive fibers adjacent to clusters of dystrophin-negative fibers, while HDR-41% or NHEJ-83% *mdx-*C skeletal muscle is composed of dystrophin-positive myofibers only. White arrows indicate the adjacent clusters of dystrophin-positive fibers. Scale bar, 100 microns.

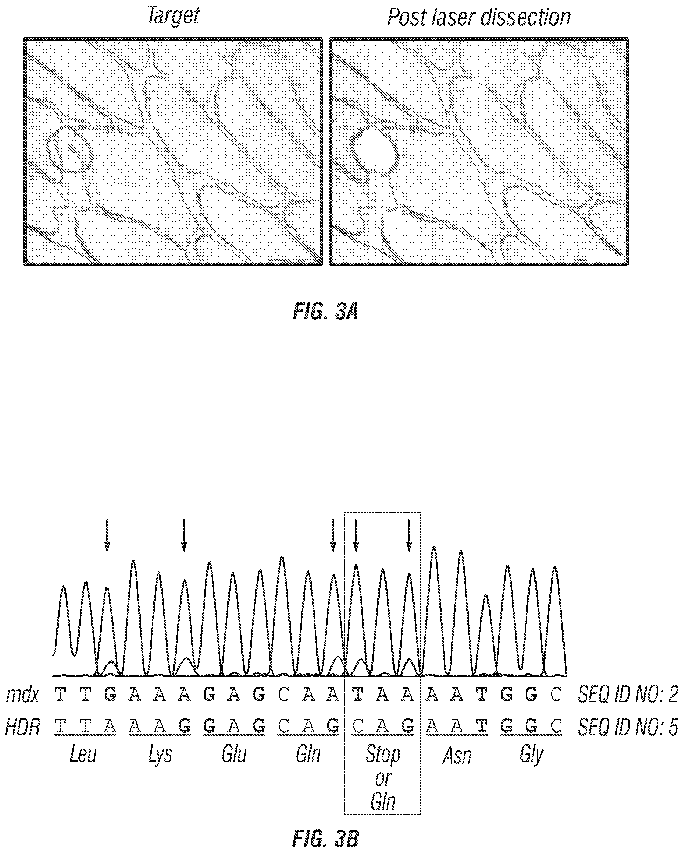

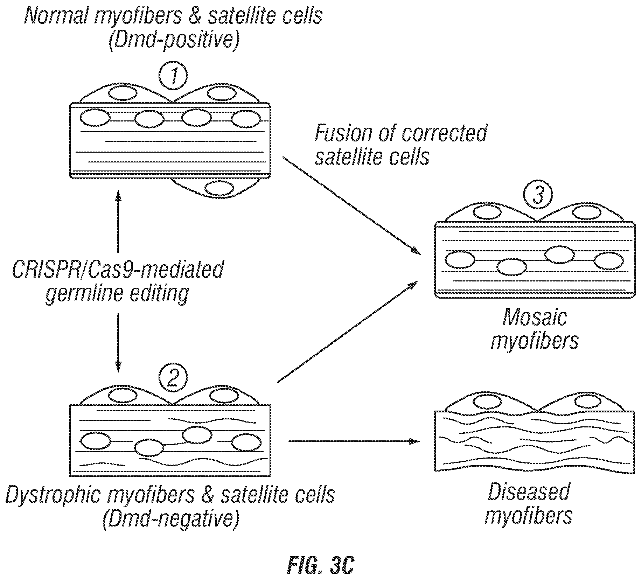

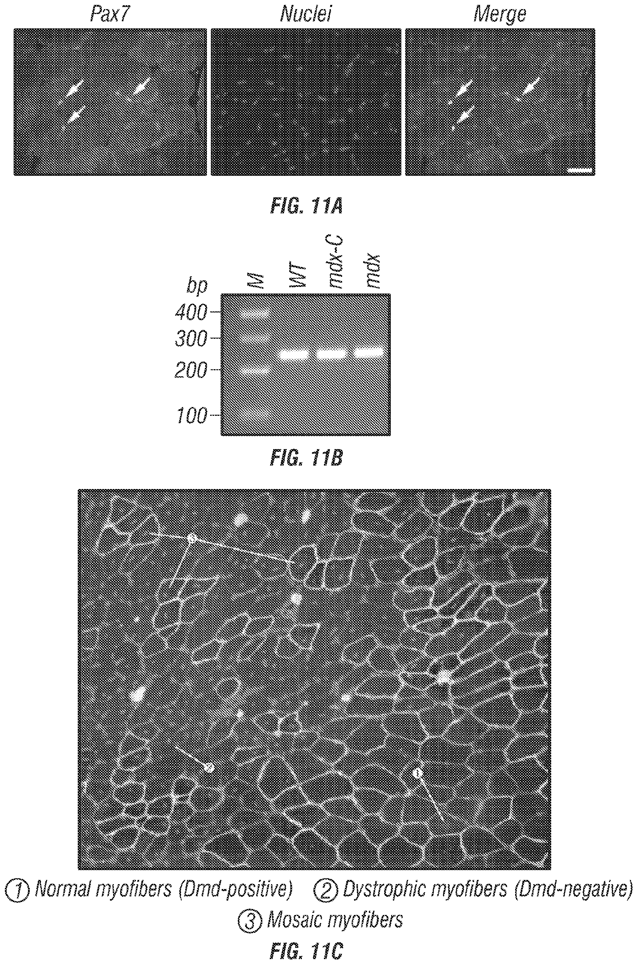

**FIGS. 3A**-C Analysis of satellite cells from ***mdx-C* mice and a model for rescue of muscular dystrophy by CRISPR/Cas9-mediated genomic correction. (****FIG. 3A****) Frozen** sections of *mdx-C* gastrocnemius were mounted onto polyethylene membrane frame slides and immunohistochemically stained for Pax-7, a marker for satellite cells. Cross-section of muscle before (left) and after (right) laser dissection shows the precise isolation of satellite cells (in circle). Scale bar, 25 microns. **(****FIG. 3B****)** PCR products corresponding to *Dmd* exon 23 were generated from genomic DNA isolated from satellite cells of *mdx-C* mice. PCR products were sequenced and show that CRISPR/Cas9-mediated genomic editing corrected a subset of satellite cells *in vivo.* The fourth arrow (1-r) indicates the corrected allele mediated by HDR. The other arrows indicate the silent mutation sites. The corresponding amino acid residues are shown under the DNA sequence. Grey box indicates the corrected site. **(****FIG. 3C****)** A model for rescue of muscular dystrophy by CRISPR/Cas9-mediated genomic correction. There are three types of myofibers in *mdx-C* mice: 1) normal dystrophin-positive myofibers (gray membrane) and satellite cells originating from corrected progenitors (grey nuclei); 2) dystrophic dystrophin-negative myofibers (light grey membrane) and satellite cells originating from *mdx* progenitors (light grey nuclei); 3) mosaic dystrophin-positive myofibers with centralized nuclei (grey and light grey nuclei) generated by fusion of corrected and *mdx* progenitors or by fusion of corrected satellite cells with pre-existing dystrophic fibers. Immunostaining of the three types of myofibers in *mdx-C* mice is shown in **FIG. 11C****.**

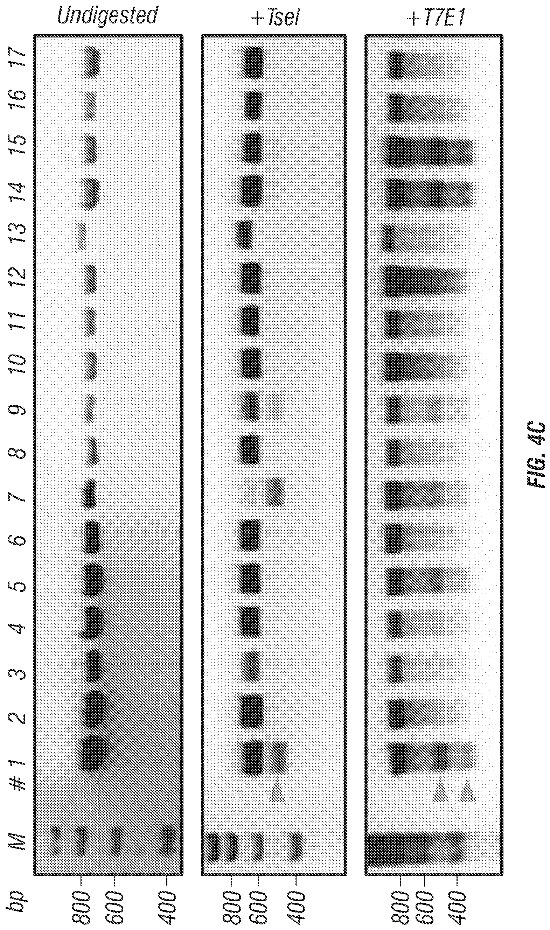

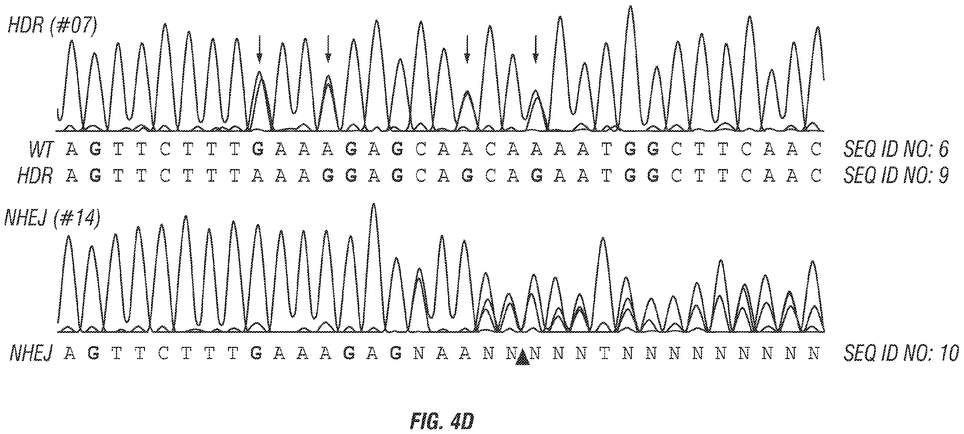

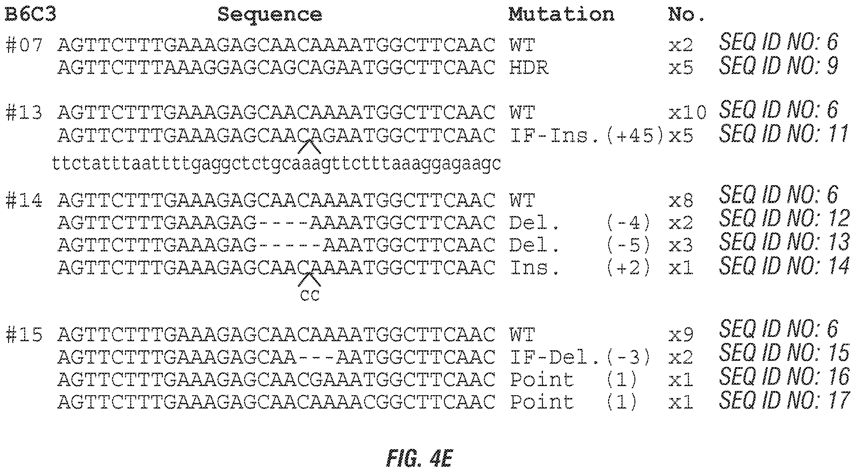

**FIGS. 4A**-E. HDR- and NHEJ-mediated gene editing of ***Dmd* in wild-type mice. (****FIG. 4A****)** Schematic of the 20-nt sgRNA target sequence *of Dmd* and the PAM (grey). The arrowhead indicates Cas9 cleavage site. **(****FIG. 4B****)** Strategy of PCR-based genotyping. ssODN, which contains 90 bp of homology sequence flanking each side of the target site was used as HDR template. ssODN incorporates four silent mutations (grey) that eliminate recutting by the sgRNA/Cas9 complex and adds a TseI restriction enzyme site (underlined) for genotyping and quantification of HDR-mediated gene editing. Black arrows indicate the positions of the PCR primers corresponding to the *Dmd* gene editing site. Digestion of the PCR product (729 bp) with TseI reveals the occurrence of HDR (437 bp). **(****FIG. 4C****)** (Upper panel) PCR-based genotyping using DNA isolated from tail biopsies of 17 mouse pups from one litter **(Table S2)** with primers listed in **Table S1.** (Middle panel) The PCR products were cut with TseI for restriction fragment length polymorphism (RFLP) analysis to screen for HDR. (Lower panel) T7 endonuclease I (T7E1), which is specific to heteroduplex DNA caused by CRISPR/Cas9-mediated genome editing, was used to screen for mutations. DNA products were loaded on a 2% agarose gel. The arrowhead indicates cleavage bands of TseI or T7E1. M denotes size marker lane. "bp" indicates the base pair length of the marker bands. **(****FIG. 4D****)** Sequencing results of PCR product of (upper panel) mouse #07 (from **FIG. 4C****)** showing HDR and of (lower panel) mouse #14 (from **FIG. 4C****)** showing NHEJ-mediated editing of the *Dmd* gene. The arrows indicate the location of the point mutations introduced by HDR. Thearrowhead points to the mixed sequencing peaks on chromatograms near the targeted site indicating heterozygous NHEJ-mediated gene editing. **(****FIG. 4E****)** Sequence of *Dmd* alleles from four F₀ mice (#07, #13, #14 and #15 from **FIG. 4C****)** from microinjection of Cas9, sgRNA and ssODN into B6C3F1 mouse zygotes. PCR products from genomic tail DNA of each mouse were subcloned into pCRII-TOPO vector and individual clones were picked and sequenced. Point mutations and silent mutations are indicated with grey letters. The lower case letters are the inserted sequences at the site indicated by thearrowhead. Deleted sequences are replaced by black dashes. The genotype of each genomic DNA clone is listed next to the sequence and in-frame insertions or deletions are termed IF-Ins. and IF-Del.. The number of inserted nucleotides is indicated by (+) and the deletion is indicated with (-). The number (No.) of clones with identical sequence is indicated by (×).

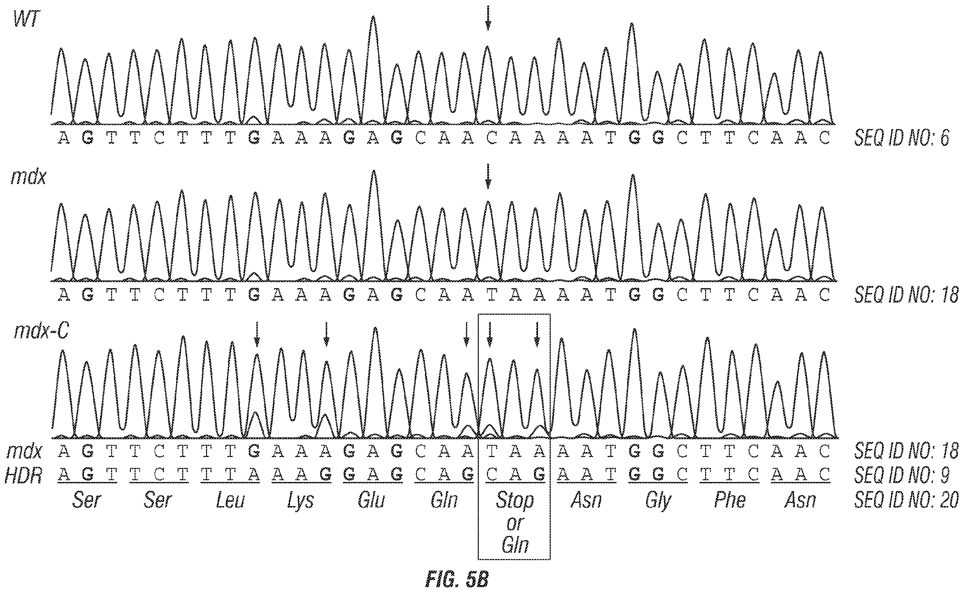

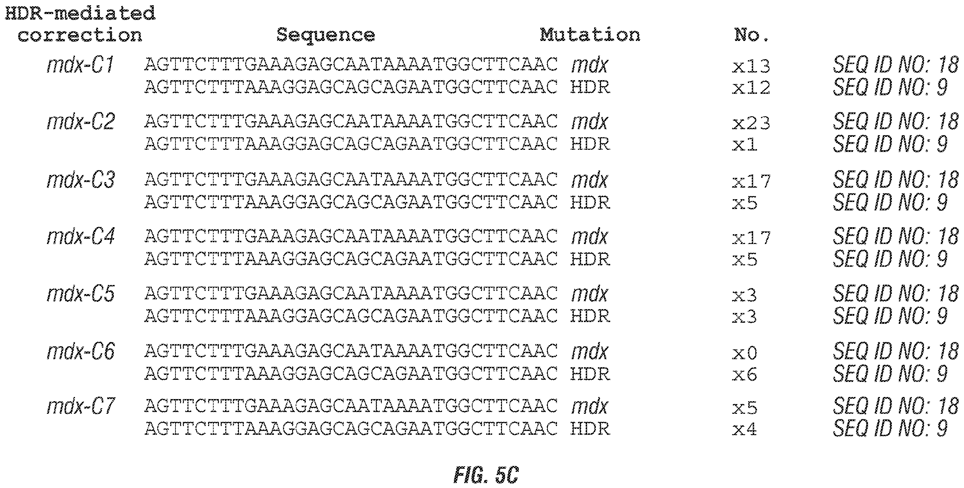

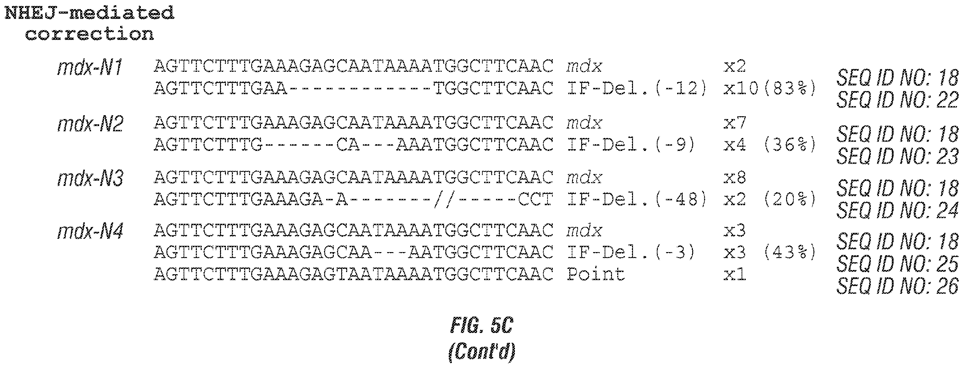

**FIGS. 5A**-C. HDR- and NHEJ-mediated gene correction in ***mdx* mice. (****FIG. 5A****)** Schematic illustrating CRISPR/Cas9-mediated gene correction via HDR or NHEJ. The corresponding amino acid residues are shown under the DNA sequence. **(****FIG. 5B****)** Direct sequencing results of WT, *mdx* and corrected *mdx-C* mice. an arrow indicates the WT allele (upper). An arrow indicates the *mdx* allele (middle). The fourth arrow (l-r)indicated the corrected allele mediated by HDR. The other arrows indicate the silent mutation sites (lower). The corresponding amino acid residues are shown under the DNA sequence. Grey box indicates the corrected site. **(****FIG. 5C****)** Sequence of *Dmd* alleles present in F₀ *mdx-C* mice **(FIG. IE)** from microinjection of Cas9, sgRNA and ssODN into *mdx* mouse (C57BL/10ScSn-*Dmd^{mdx}*/J) zygotes. PCR products from genomic tail DNA of each mouse were subcloned into pCRII-TOPO vector and individual clones were picked and sequenced. The *mdx* point mutation (C to T) and silent mutations are indicated with grey letters. The number (No.) of clones with identical sequence is indicated by (×). The variability observed in the ratio of HDR or NHEJ sequence to *mdx* sequence for each *mdx-C* mouse reflects the degree of mosaicism.

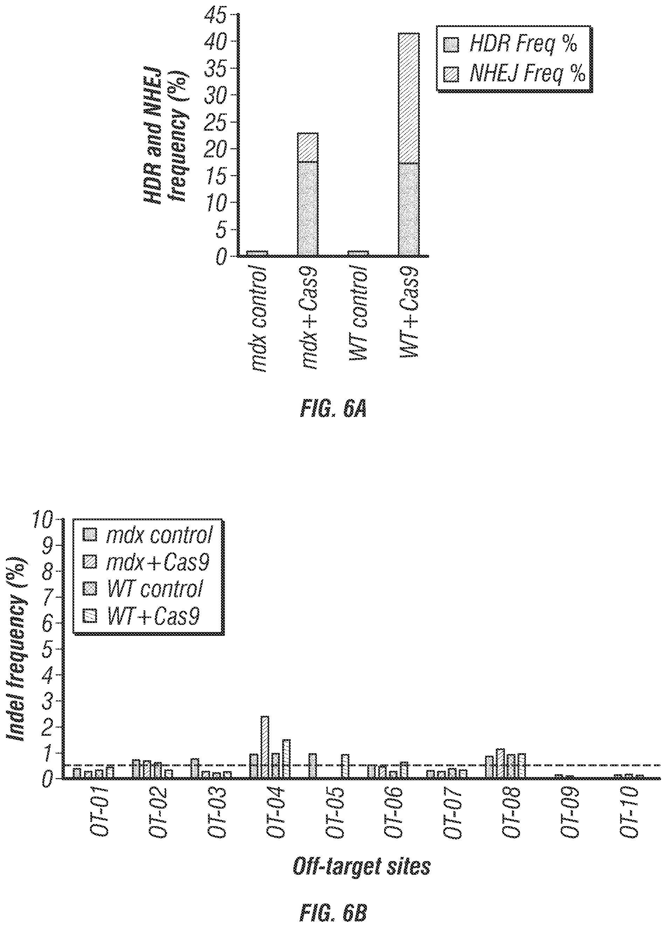

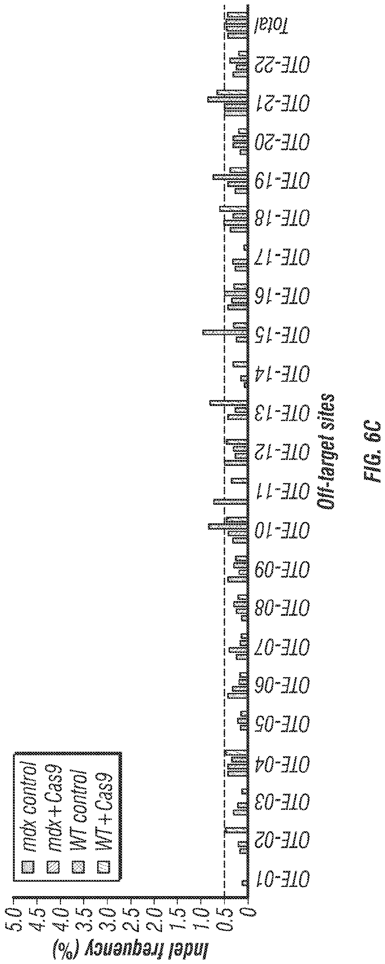

**FIG. 6A**-C. Deep sequencing analysis of target site (***Dmd*) and 32 theoretical offtarget sites. (****FIG. 6A****)** Frequency of HDR- (bottom of bar) and NHEJ-mediated (top of bar) gene correction at target site (*Dmd*) from deep sequencing of DNA from four groups of mice: *mdx, mdx*+Cas9*,* WT and WT+Cas9. **(****FIG. 6B****)** Frequency of NHEJ-mediated indels at genome-wide "top ten" theoretical off-target sites (OT-01 to OT-10) **(Table S3)** from deep sequencing results of DNA from the four groups of mice (top to bottom of key is left to right). **(****FIG. 6C****)** Frequency of NHEJ-mediated indels at twenty-two theoretical off-target sites within exons (OTE-01 to OTE-22) **(Table S3)** from deep sequencing of DNA from the four groups of mice (top to bottom of key is left to right).

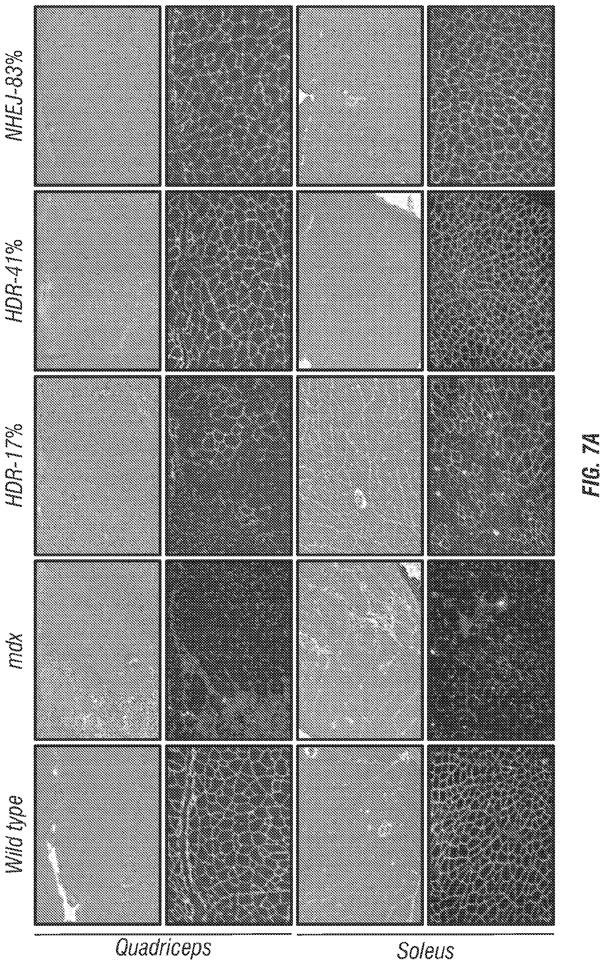

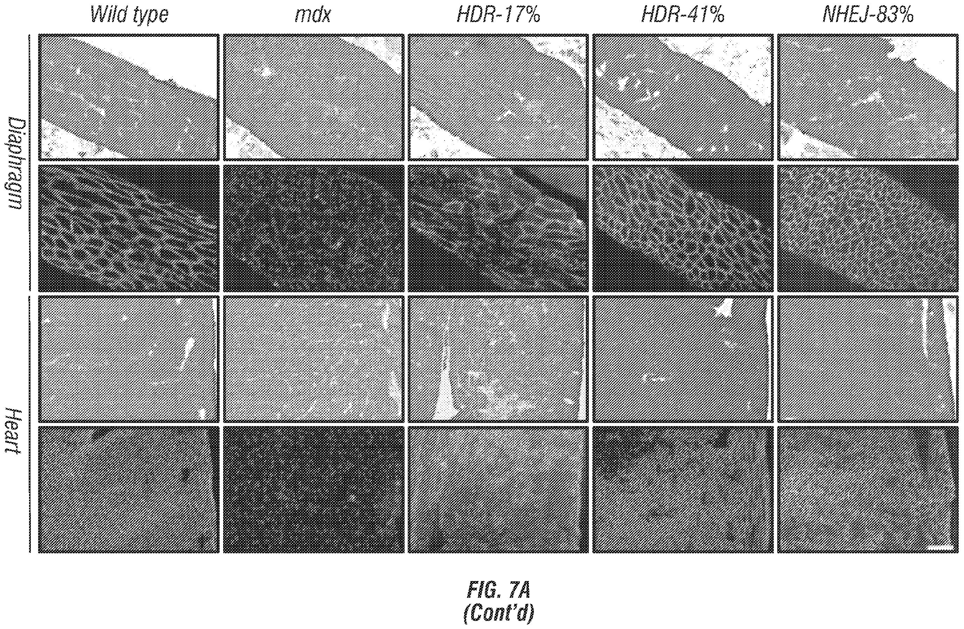

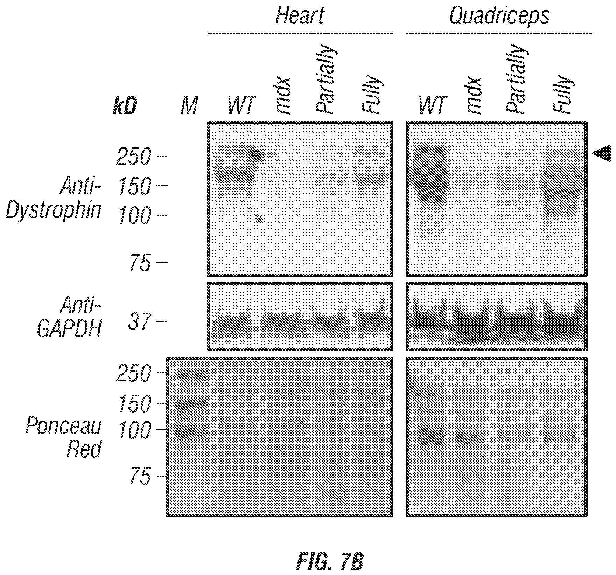

**FIGS. 7A**-B. Histological and Western blot analysis of muscle from wild-type, ***mdx,* and *mdx-C* mice. (****FIG. 7A****)** Hematoxylin and eosin (H&E) and immunostaining of muscles from 7-9 week old wild-type, *mdx, and mdx-C* mice (HDR-17%, HDR-41% and NHEJ-83% corrected allele; as seen in **FIG. 2****).** Immunofluorescence detects dystrophin. Nuclei are labeled by propidium iodide (red). Scale bar, 100 microns. **(****FIG. 7B****)** Western blot analysis of heart and skeletal muscle (quadriceps) samples from wild-type, *mdx,* and partially corrected (HDR-17%) and fully corrected (HDR-41%) *mdx-C* mice. Red arrowhead (>250kD) indicates the immunoreactive bands of dystrophin. Lower bands (<250kD), which were also absent in *mdx* samples, likely represent proteolytic breakdown of full-length dystrophin protein, natural variants or protein synthesis intermediates. The same pattern of bands was observed in samples from wild-type and *mdx-C* mice. GAPDH is a loading control. PVDF membrane was stained for total protein by 2% Ponceau Red. M denotes size marker lane. kD indicates the protein length of the marker bands.

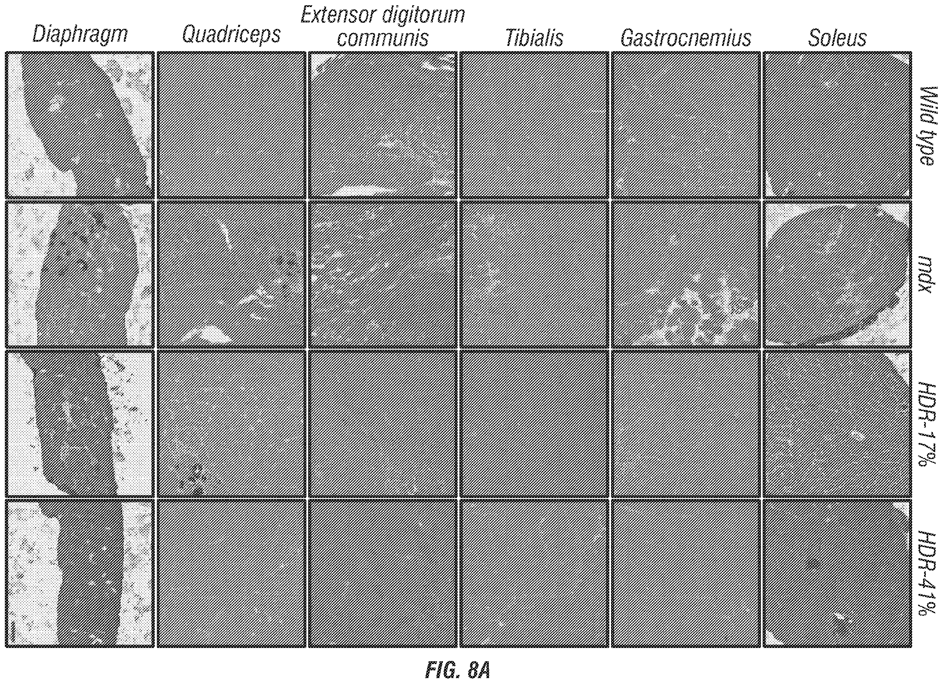

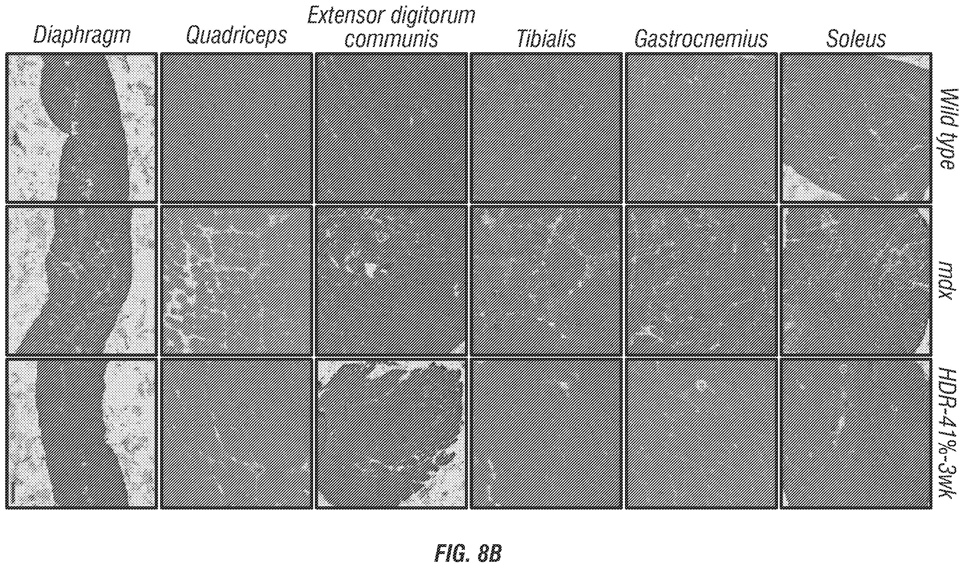

**FIG. 8A**-B. Histology of muscles showing decrease in fibrosis and necrosis by **CRISPR/Cas9-mediated genomic editing of *Dmd* allele.** Hematoxylin and eosin (H&E) stained transverse cryosections of whole soleus, gastrocnemius, tibialis-anterior, extensor-digitorum-communis, quadriceps, and diaphragm from **(****FIG. 8A****)** 7-9 week old wild-type, *mdx,* HDR-17% and HDR-41% and **(****FIG. 8B****)** 3-week old wild-type, *mdx,* HDR-40%-3wk. Scale bar, 125 microns.

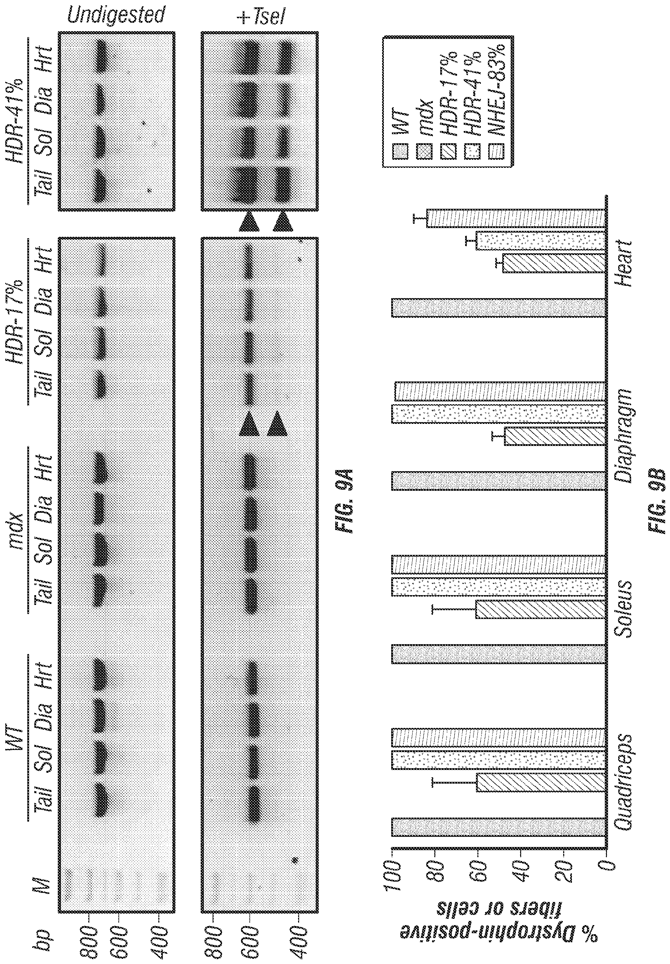

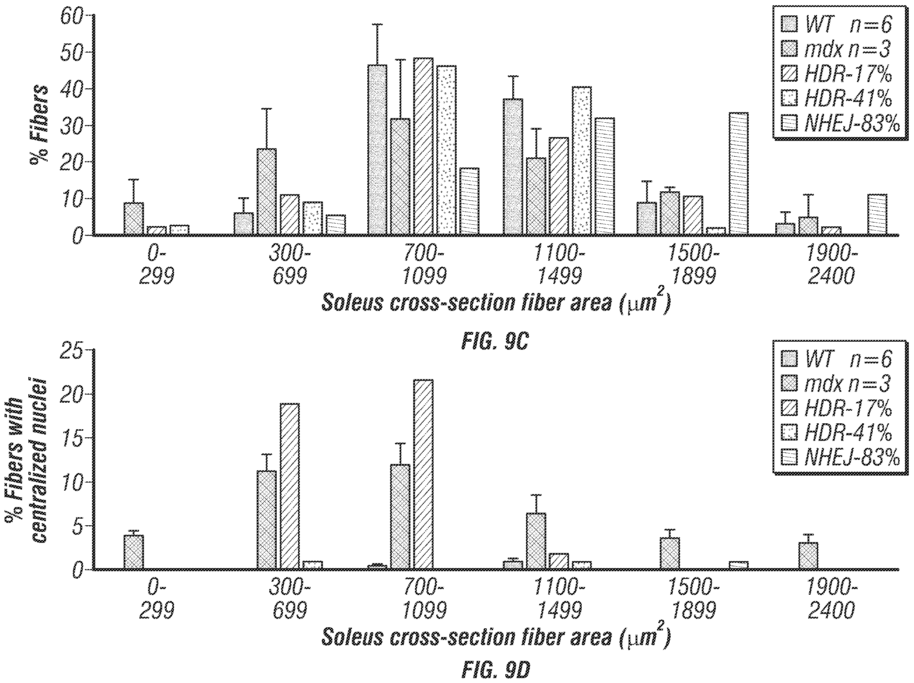

**FIGS. 9A**-D. RFLP analysis and myofiber measurements of muscle from wild-**type, *mdx,* and corrected *mdx-C* mice. (****FIG. 9A****)** RFLP analysis to quantify the degree of mosaicism of genomic DNA isolated from tail, soleus (Sol), diaphragm (Dia) and heart (Hrt) of wild-type, *mdx,* HDR-17% and HDR-41% mice. PCR was performed using genomic DNA using primers (Dmd729F and Dmd729R) (upper panel) and digested with TseI (lower panel). DNA products were loaded on a 2% agarose gel. The lower arrowhead marks the DNA band indicating HDR-mediated correction, generated by TseI digestion. The upper arrowhead marks the DNA band of the uncorrected *mdx* allele. M denotes size marker lane. bp indicates the base pair length of the marker bands. **(****FIG. 9B****)** Quantification of dystrophin-positive cells in quadriceps, soleus, diaphragm and heart. n= 6 for WT; n=3 for *mdx.* Error bars show standard deviation based on data from multiple muscle sections (top to bottom of key is left to right). **(****FIG. 9C****)** Measurement of the distribution of the cross-sectional areas of myofibers from the soleus of wild-type mice showed uniformly sized fibers with 90% of the fibers ranging from 700-1499 µm². In contrast, myofibers from *mdx* mice were heterogeneous in size, ranging between 300-1899 µm². The size distribution of the myofibers from HDR-41% muscle was strikingly similar to that of wild-type mice. n= 6 for WT; n=3 for *mdx.* Error bars show standard deviation based on data from multiple muscle sections (top to bottom of key is left to right). **(****FIG. 9D****)** Distribution of soleus myofibers with centralized nucleus. The percentage of regenerated myofibers of muscle from HDR-17% ranged from 700-1099 µm², which was higher than the percentage of fibers from the *mdx* muscle (top to bottom of key is left to right).

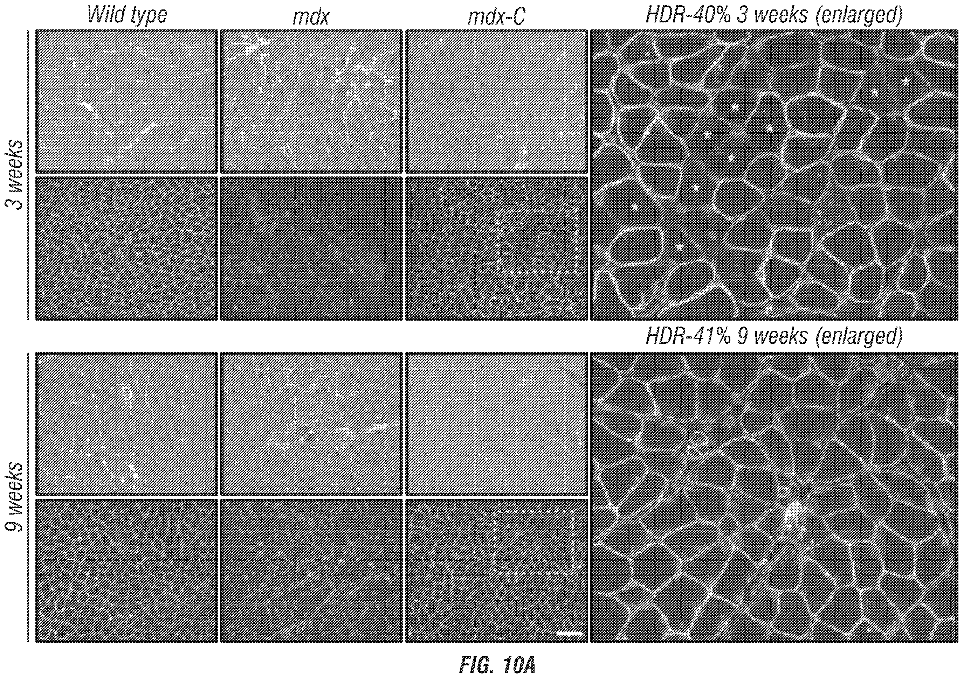

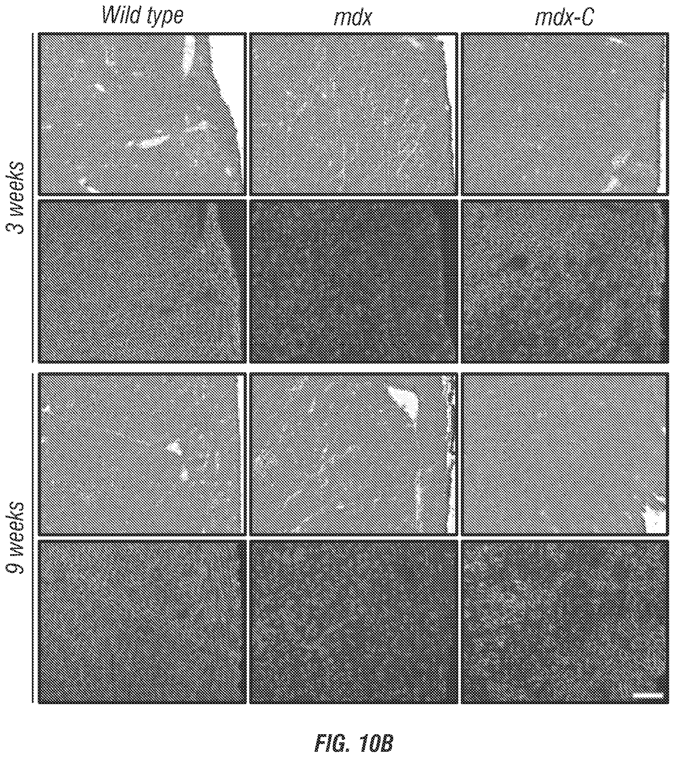

**FIGS. 10A**-B. Progressive recovery of skeletal muscle not heart following **CRISPR/Cas9-mediated genomic editing of *Dmd* allele.** Hematoxylin and eosin (H&E) and dystrophin immunostaining of **(****FIG. 10A****)** soleus or **(****FIG. 10B****)** heart from 3-week old and 9-week old wild-type, *mdx, and mdx-C* mice (3-week-old is HDR-40%-3wk; 9-week old is HDR-41%). Immunofluorescence detects dystrophin. Nuclei are labeled by propidium iodide (red). Magnification of boxed area shows 3-week old (HDR-40%-3wk) and 9-week old (HDR-41%) muscle. At 3-weeks of age many, but not all, of the myofibers express dystrophin showing partial recovery. White star indicates dystrophin-negative myofibers. By 9-weeks of age, all myofibers in the corrected muscle show dystrophin expression. Although dystrophin expression has been restored in hearts of *mdx-C* mice, no progressive improvement with age is seen from 3-weeks to 9-weeks of age. Scale bar, 100 microns.
**FIGS. 11A**-C. Analysis of satellite cells and three types of myofibers in ***mdx-C* mice. (****FIG. 11A****)** Cross-section of gastrocnemius from *mdx-C* mouse immunostained for satellite cell-specific marker, Pax7 (left,) and nuclei (middle, propidium iodide). A merged image (right) shows the 'yellow' satellite cells located at the edges of the muscle fibers and distinguishes them from "red" myofiber nuclei. White arrows indicate Pax-7 positive satellite cells. Scale bar, 40 microns. **(****FIG. 11B****)** The 232 bp PCR products corresponding to exon 23 of the *Dmd* gene from laser dissected satellite cells of wild-type, *mdx-C* and *mdx* mice were analyzed on a 2% agarose gel. M denotes size marker lane. bp indicates the base pair length of the marker bands. **(****FIG. 11C****)** Immunostaining of *mdx-C* soleus with anti-dystrophin and propidium iodide highlighting three types of myofibers in the partially corrected *mdx* muscle: 1) normal dystrophin-positive myofibers that originated from CRISPR/Cas9-mediated genome-corrected muscle progenitors; 2) dystrophic dystrophin-negative myofibers that originated from *mdx* mutant progenitors; 3) mosaic dystrophin-positive myofibers with centralized nuclei that formed from fusion of corrected satellite cells with pre-existing dystrophic muscle.

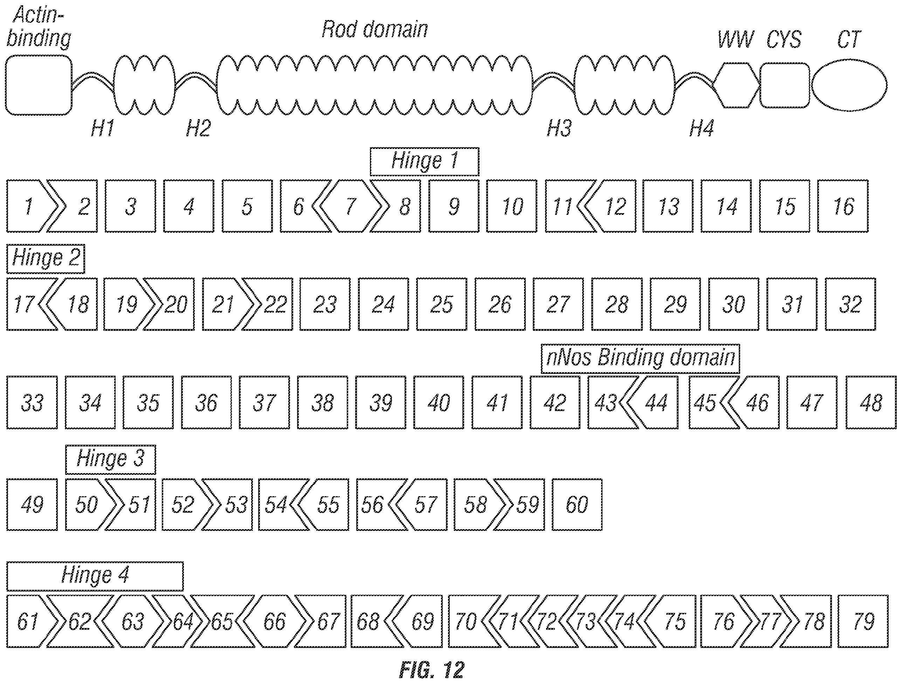

**FIG. 12****. Strategy for exon skipping.** Domains of dystrophin and structure of the exons are showed. Shapes of intron-exon junctions indicate complementarity that maintains the open reading frame upon splicing.

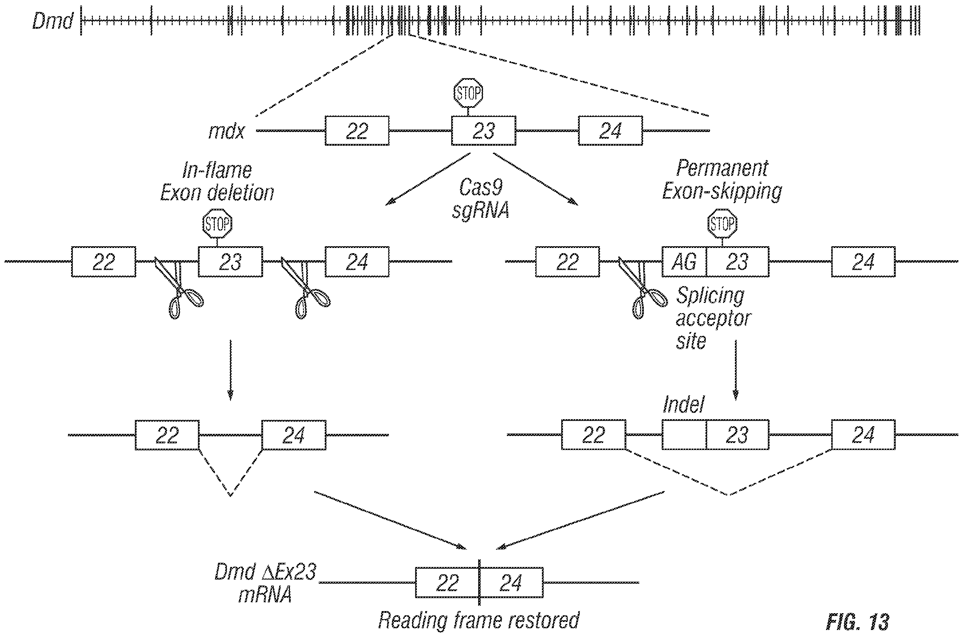

**FIG. 13****. Two types of Myo-editing-mediated exon-skipping.** Strategies for bypassing exon 23 by NHEJ are shown.

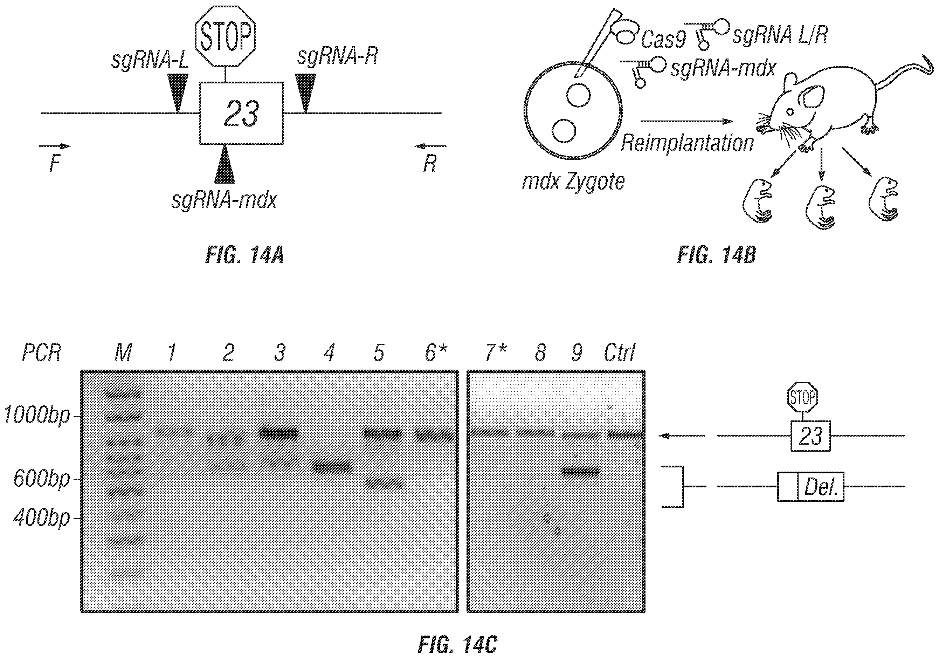

**FIG. 14****. Strategy of Myo-editing-mediated exon-skipping in germline of *mdx* mice. (****FIG. 14A****)** Guide RNAs target the 5' and 3' of exon 23 were indicated by black and blue arrowheads. **(****FIG. 14B****)** Cas9 mRNA and guide RNAs were co-injected into *mdx* eggs. **(****FIG. 14C****)** PCR products corresponding to *Dmd* exon 23 from pups were analyzed on the agarose gel. The upper band indicate the full-length PCR products, the lower bands indicate the PCR product with ~200 bp deletion (exon 23). 7 out of 9 pups skipped exon23. M denotes size marker lane. Lanes 2-5 & 9, and lanes 6 & 7, respectively, indicate positive mdx pups with large and small indel mutations.

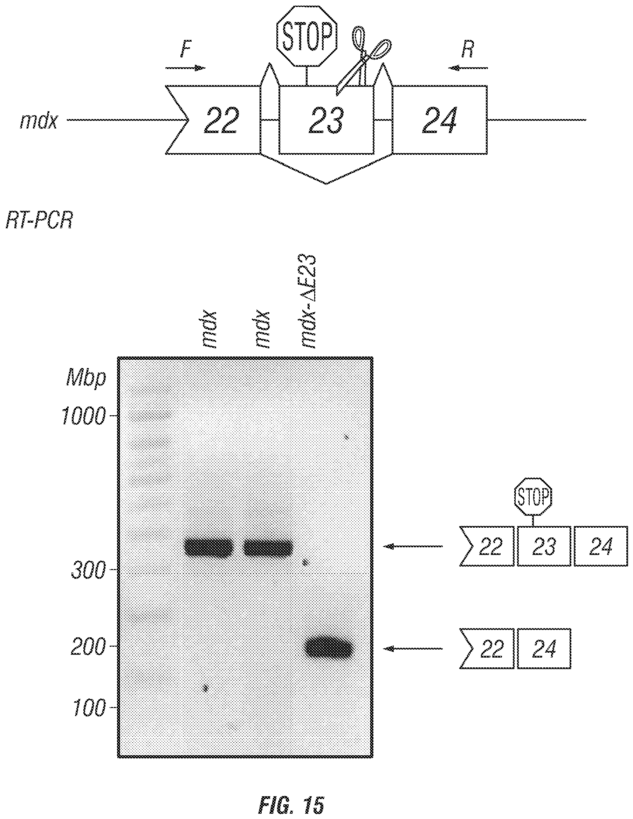

**FIG. 15****. Skipping of exon 23 following Myo-editing.** RT-PCR of RNA from *mdx* and edited *mdx* mice was performed with the indicated sets of primers (F and R). Destruction of the exon 23 splice site allows splicing from exon 22 to 24 (lower band) and restoration of the dystrophin open reading frame.

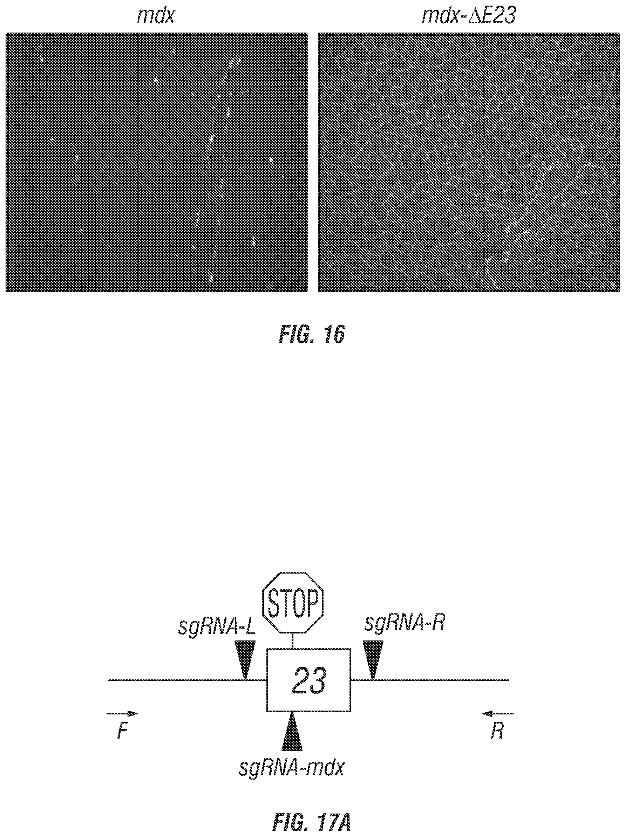

**FIG. 16****. Rescue of dystrophin expression in *mdx* mice by skipping of exon 23.** Dystrophin staining (green) of muscle from *mdx* mice and *mdx* mice following NHEJ mediated skipping of exon 23 is shown. Dystrophin expression is fully restored by skipping exon 23.

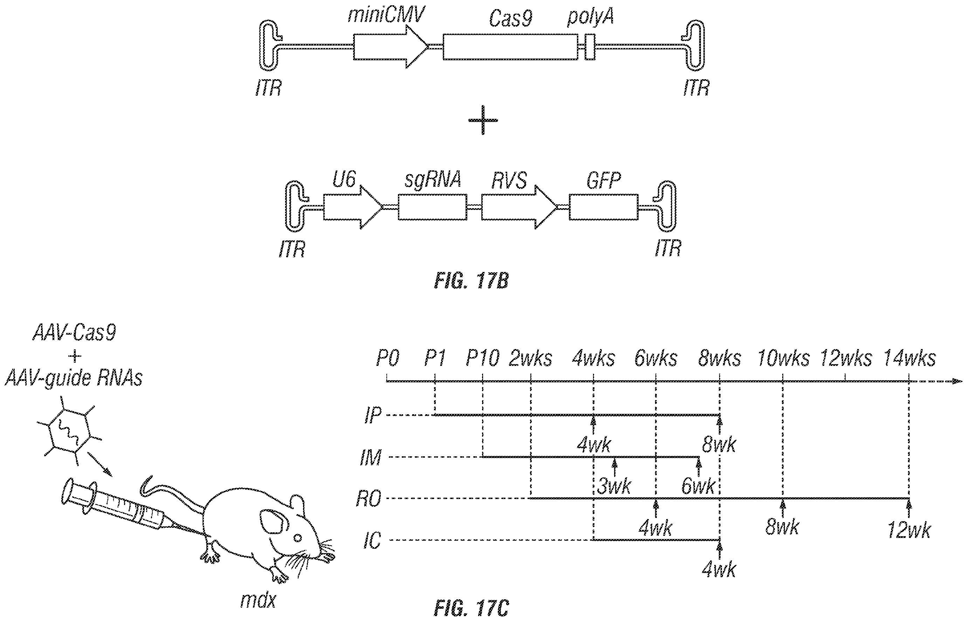

**FIGS. 17A**-C. Schematic for ***in vivo* rescue of muscular dystrophy in *mdx* mice by AAV-mediated Myo-editing. (****FIG. 17A****)** Guide RNAs target the 5' and 3' of exon 23 were indicated by upper arrowheads. **(****FIG. 17B****)** Strategies for Cas9, guide RNAs and GFP expression from AAV viral vectors. **(****FIG. 17C****)** Schematic for different modes of AAV9 delivery: intra-pertitoneal injection (IP), intra-muscular injection (IM), retro-orbital injection (RO), and intra-cardiac injection (IC). Black arrows indicate the post-injection time points for tissue collection.

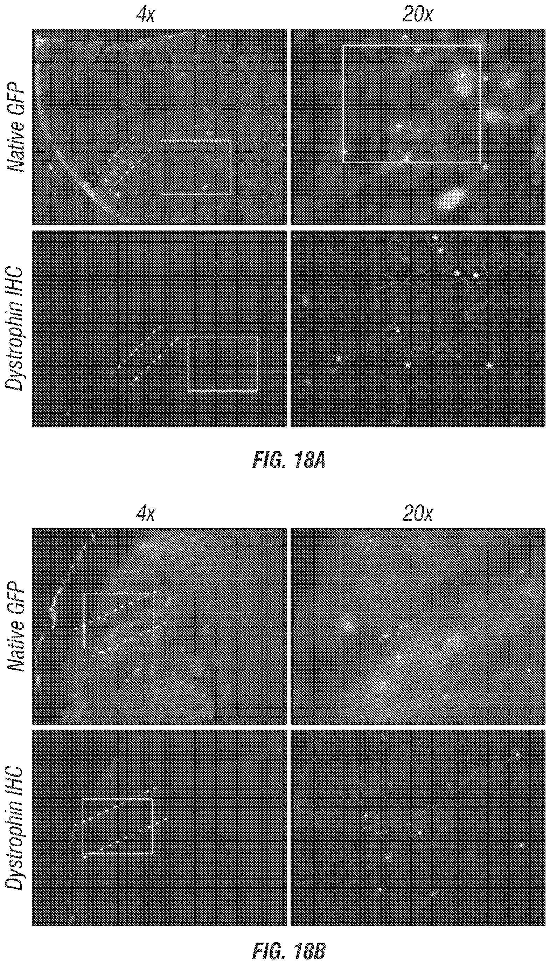

**FIGS. 18A**-B. Rescue of dystrophin expression in ***mdx* mice by Myo-editing with AAV9-Cas9 delivery by direct intramuscular injection (IM-AAV) or intra-cardiac injection (IC-AAV). (****FIG. 18A****)** Native green fluorescent protein (GFP) and dystrophin immunostaining from serial sections of *mdx* mouse tibialis anterior muscle is illustrated 3-week post-IM-AAV of AAV9-Cas9 + AAV9-gsRNA-GFP (IM-AAV at postnatal day 10; P10). A transduction frequency or rescue of 7.7%±3.1% of myofibers is estimated in treated *mdx* mouse tibialias anterior muscle 3-weeks post-IM-AAV (n=3, dystrophin positive myofibers as a function of total myofibers). **(****FIG. 18B****)** Native GFP and dystrophin immunostaining from serial sections of mdx mouse heart showing evidence of cardiomyocyte rescue 4-weeks post-IC-AAV (IC-AAV at 28-days of age). Dotted lines indicate injecting needle track, boxes indicate fields of higher magnification, and asterisks indicate serial section myofiber alignment.

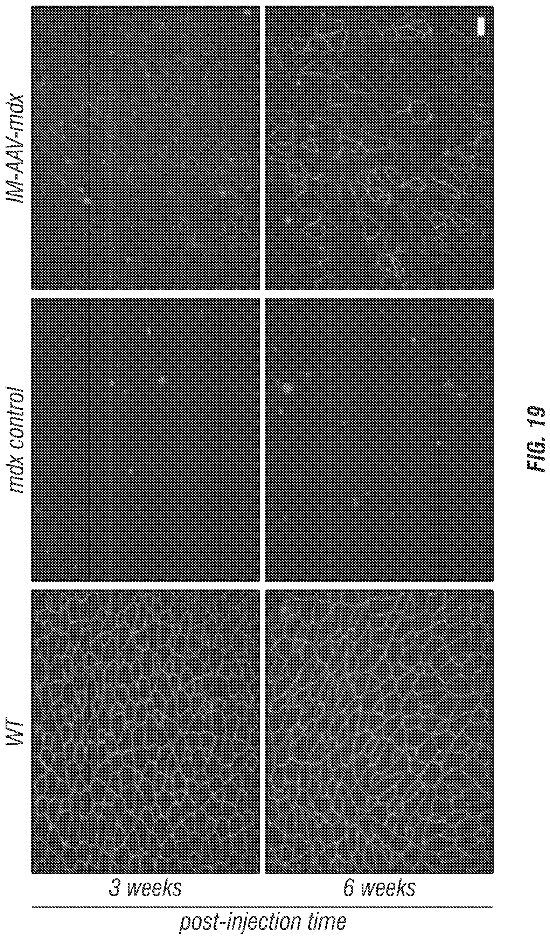

**FIG. 19****. Progressive rescue of dystrophin expression in *mdx* mice by Myo-editing with IM-AAV.** Dystrophin immunostaining of tibialis anterior muscle is illustrated for wild-type mice (WT), *mdx* mice, and IM-AAV treated *mdx* mice at 3 and 6-weeks post-injection. Transduction frequency (rescue) increases to an estimated 25.5%±2.9% of myofibers by sixweek post-IM-AAV (n=3). Scale bar, 40 microns.

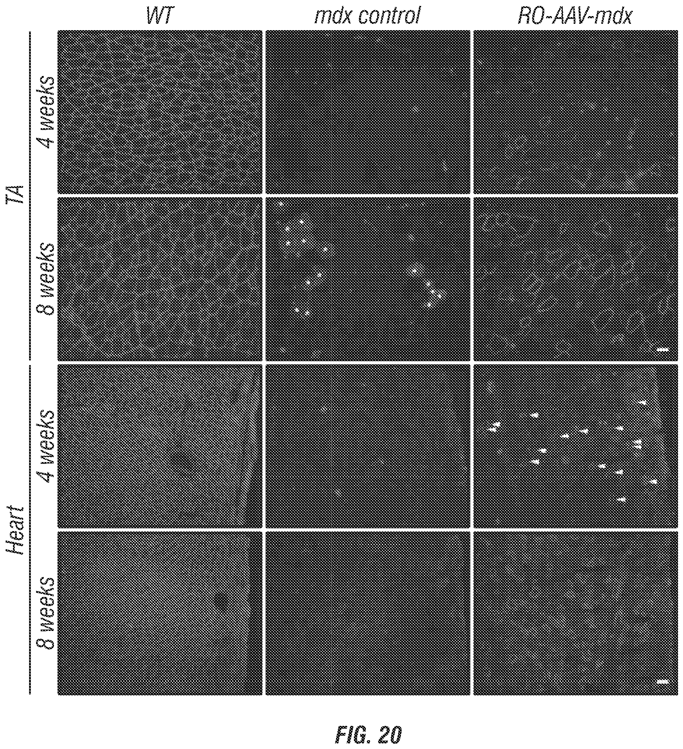

**FIG. 20****. Progressive rescue of dystrophin expression in *mdx* mice by Myo-editing with AAV9-Cas9 systemic delivery by retro-orbital injection (RO-AAV).** Dystrophin immunostaining of tibialis anterior muscle and heart is illustrated for wild-type mice (WT), *mdx* mice, and RO-AAV treated *mdx* mice at 4 and 8-weeks post-injection (RO-AAV at P10). A transduction frequency (rescue) of 1.9%±0.51% of myofibers is estimated in treated *mdx* mouse tibialis anterior muscle and 1.3%±0.05% of cardiomyocytes in treated mdx mouse heart at 4-weeks post-RO-AAV. Rescue increases to an estimated 6.1±3.2% of myofibers in tibialis anterior muscle, and rescue of as many as 8.7% of cardiomyocytes (5.0%±2.1 %), by 8-weeks post-RO-AAV (n=3 for all groups). Myofiber necrosis of tibialis anterior muscle of unedited mdx control mice exhibit cytoplasm-filling autofluorescence are highlighted with white asterisks. Arrowheads indicate dystrophin positive cardiomyocytes in 4-weeks post-RO-AAV treated *mdx* mouse heart. Scale bar, 40 microns.

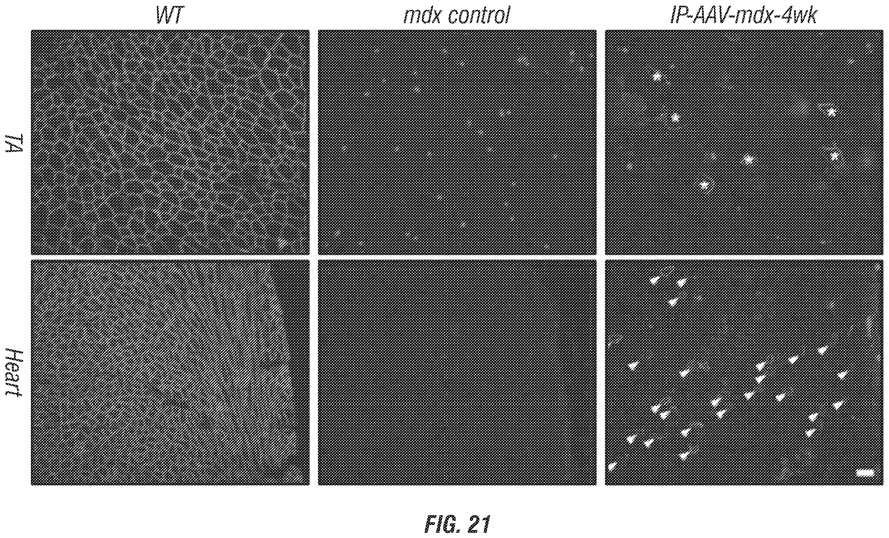

**FIG. 21****. Rescue of dystrophin expression in *mdx* mice by Myo-editing with AAV9-Cas9 systemic delivery by intraperitoneal injection (IP-AAV).** Dystrophin immunostaining of tibialis anterior muscle and heart is illustrated for wild-type mice (WT), *mdx* mice, and IP-AAV treated *mdx* mice at 4-weeks post-injection (IP-AAV at P1). A transduction frequency (rescue) of 3.0% of myofibers is estimated in treated *mdx* mouse tibialias anterior muscle (n=1), and 2.4% of cardiomyocytes in treated mdx mouse heart (n=1), at 28-days post-IP-AAV. Asterisks and arrowheads indicate dystrophin positive myofibers and cardiomyocytes, respectively, in IP-AAV treated *mdx* mice. Scale bar, 40 microns.

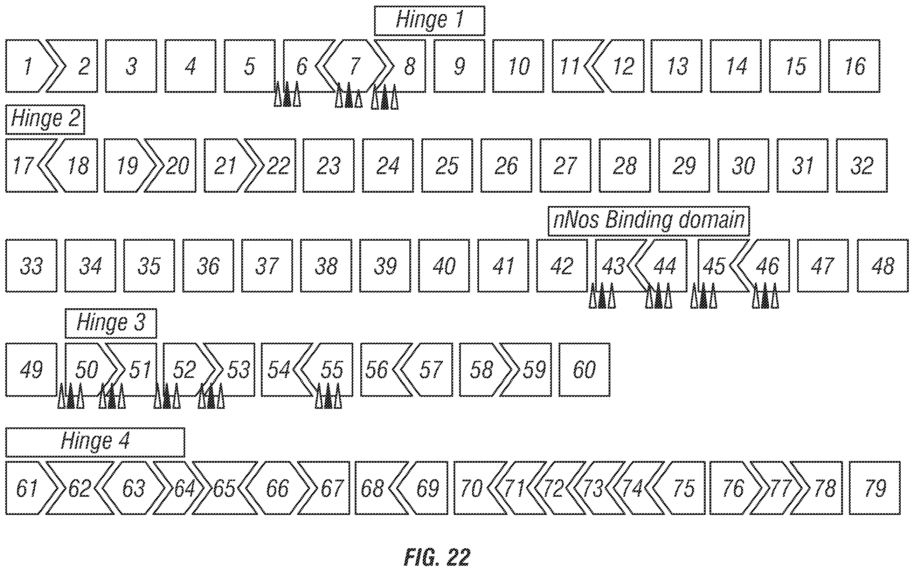

**FIG. 22****. A pool of sgRNAs target the hot spot mutation regions in *DMD.*** The arrowheads indicate the target sites.

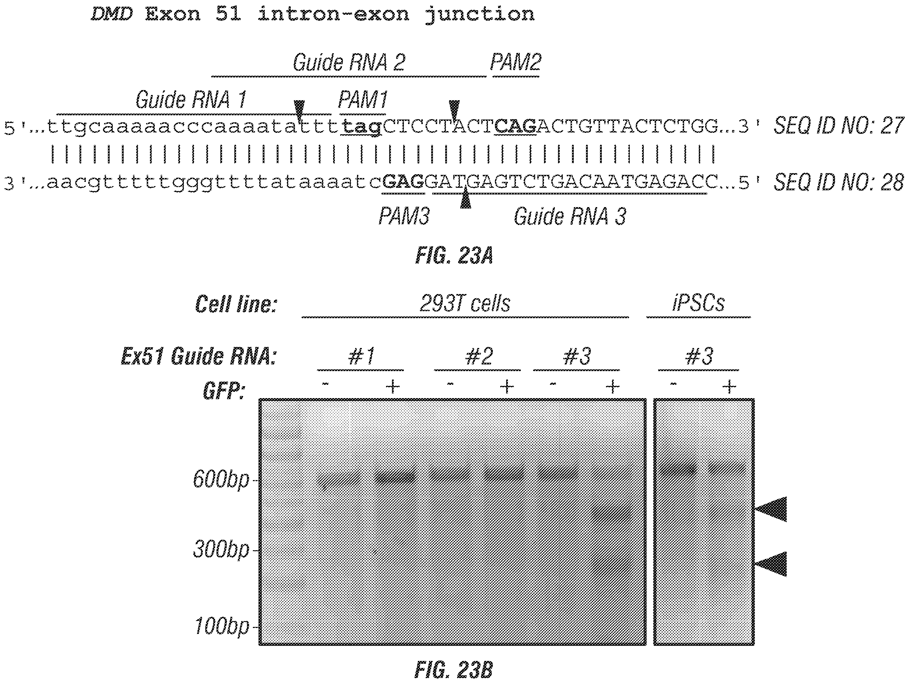

FIGS. 23A-B. Myo-editing target exon 51 splice acceptor site in human cells. **(****FIG. 23A****)** Using the guide RNA library, three guide RNAs that target 5' of exon 51 were selected. **(****FIG. 23B****)** Myo-editing efficiency was demonstrated via T7E1 assay. Guide RNA #3 (red) showed high activity in 293T cells, while guide RNA #1 and 2 had no detectable activity. The same results of guide RNA #3 was observed in normal human iPSCs.

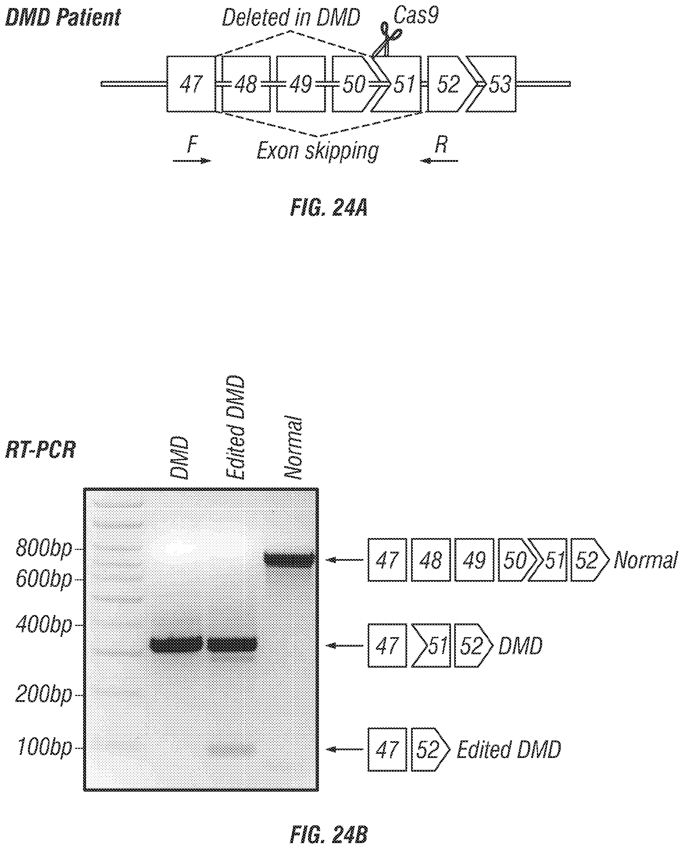

**FIGS. 24A**-B RT-PCR of cardiomyocytes differentiated from normal, DMD **(Riken HPS0164) and edited-iPSCs. (****FIG. 24A****)** A deletion (exons 48-50) in DMD patient creates a frame-shift mutation in exon 51. **(****FIG. 24B****)** RT-PCR of RNA from cardiomyocytes in which the exon 51 splice acceptor sequence was destroyed by Myo-editing was performed with the indicated sets of primers (F and R). Destruction of the exon 51 splice acceptor in DMD-iPSCs allows splicing from exon 47 to 52 and restoration of the dystrophin open reading frame (the lowest band).

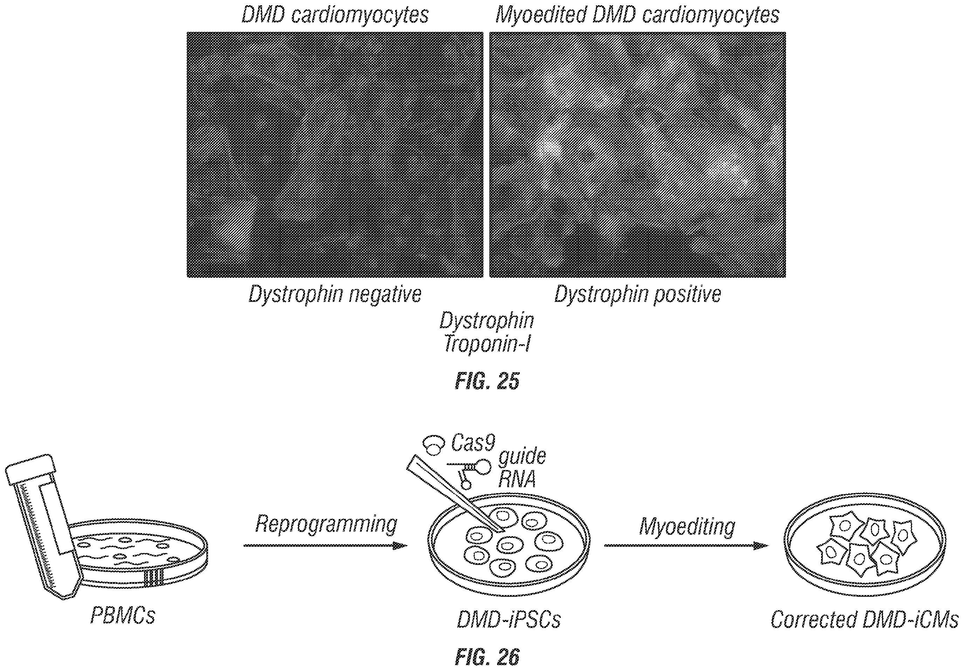

**FIG. 25****. Successful rescue of dystrophin expression by CRISPR/Cas9 Myo-editing in DMD iPSC-derived cardiomyocytes.** Immunocytochemistry of dystrophin expression (green staining) shows DMD iPSC (Riken HPS0164) derived cardiomyocytes normally lack dystrophin and successful Myo-editing in DMD iPSC cardiomyocytes has dystrophin expression. Immunofluorescence (red) detects cardiac marker Troponin-I. Nuclei are labeled by Hoechst dye (blue).
**FIG. 26****. Schematic of the Myo-editing in DMD-iPSCs.**

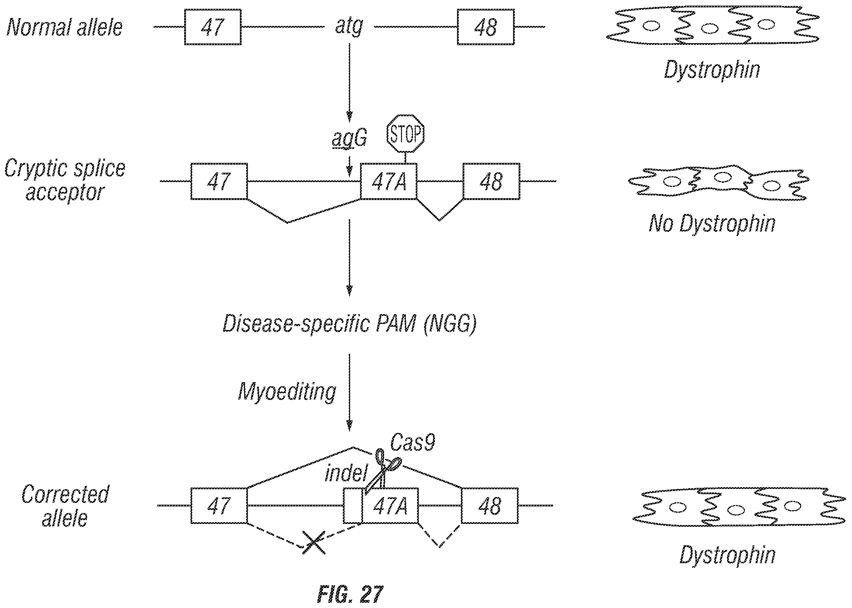

**FIG. 27****. Myo-editing strategy for pseudoexon 47A of patient DC0160.** *DMD* exons are represented as gray boxes. Pseudoexon with stop code is marked by a stop sign. Black box indicates Myo-editing-mediated indel. Grey membrane indicates normal dystrophin-positive cardiomyocytes.

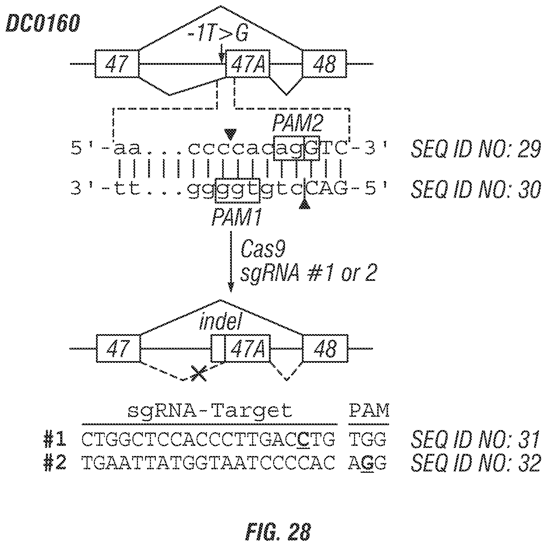

**FIG. 28****. Sequence of guide RNAs for pseudoexon 47A of patient DC0160.** *DMD* exons are represented as black boxes, pseduoexons are represented as light grey boxes (47A). Grey box indicates indel.

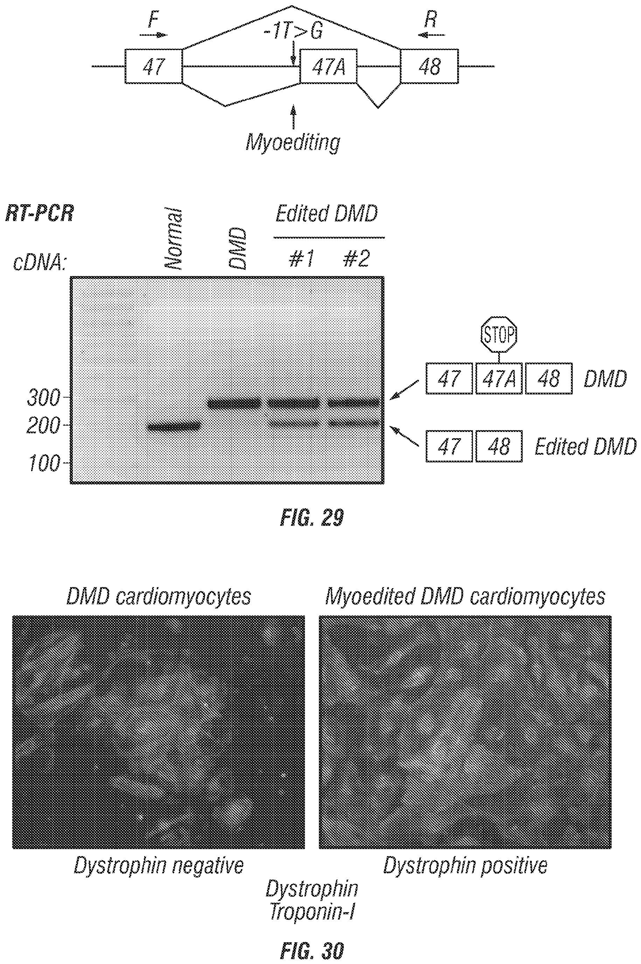

**FIG. 29****. RT-PCR of human cardiomyocytes differentiated from normal, DMD and edited-iPSCs.**
**FIG. 30****. Rescue of dystrophin expression by Myo-editing of DMD (DC0160)-iPSCs-derived human cardiomyocytes.** Immunocytochemistry of dystrophin expression shows DMD iPSC (DC0160) cardiomyocytes lacking dystrophin expression. Following successful Myo-editing, the edited-DMD iPSC cardiomyocytes express dystrophin. Immunofluorescence detects cardiac marker Troponin-I. Nuclei are labeled by Hoechst dye.

### DETAILED DESCRIPTION

Duchenne muscular dystrophy, like many other diseases of genetic origin, present challenging therapeutic scenarios. Recently, the development of "gene editing" has increased the ability to correct genetic effects in cells. The following disclosure describes the use of the CRIPSR/Cas9 system to edit the genomes of cells carrying defects in the dystrophin gene using either non-homologous end-joining (NHEJ), resulting in insertion/deletion (indel) mutations, or by homology-directed repair (HDR), that generates a precise modification at a target locus. These and other aspects of the disclosure are set out in detail below.

### I. Duchenne Muscular Dystrophy

### A. Background

Duchenne muscular dystrophy (DMD) is a recessive X-linked form of muscular dystrophy, affecting around 1 in 3,500 boys, which results in muscle degeneration and premature death. The disorder is caused by a mutation in the gene dystrophin, located on the human X chromosome, which codes for the protein dystrophin. Dystrophin is an important component within muscle tissue that provides structural stability to the dystroglycan complex (DGC) of the cell membrane. While both sexes can carry the mutation, females are rarely affected with the skeletal muscle form of the disease..

### B. Symptoms

Symptoms usually appear in boys between the ages of 2 and 3 and may be visible in early infancy. Even though symptoms do not appear until early infancy, laboratory testing can identify children who carry the active mutation at birth. Progressive proximal muscle weakness of the legs and pelvis associated with loss of muscle mass is observed first. Eventually this weakness spreads to the arms, neck, and other areas. Early signs may include pseudohypertrophy (enlargement of calf and deltoid muscles), low endurance, and difficulties in standing unaided or inability to ascend staircases. As the condition progresses, muscle tissue experiences wasting and is eventually replaced by fat and fibrotic tissue (fibrosis). By age 10, braces may be required to aid in walking but most patients are wheelchair dependent by age 12. Later symptoms may include abnormal bone development that lead to skeletal deformities, including curvature of the spine. Due to progressive deterioration of muscle, loss of movement occurs, eventually leading to paralysis. Intellectual impairment may or may not be present but if present, does not progressively worsen as the child ages. The average life expectancy for males afflicted with DMD is around 25.

The main symptom of Duchenne muscular dystrophy, a progressive neuromuscular disorder, is muscle weakness associated with muscle wasting with the voluntary muscles being first affected, especially those of the hips, pelvic area, thighs, shoulders, and calves. Muscle weakness also occurs later, in the arms, neck, and other areas. Calves are often enlarged. Symptoms usually appear before age 6 and may appear in early infancy. Other physical symptoms are:
- Awkward manner of walking, stepping, or running - (patients tend to walk on their forefeet, because of an increased calf muscle tone. Also, toe walking is a compensatory adaptation to knee extensor weakness.)
- Frequent falls
- Fatigue
- Difficulty with motor skills (running, hopping, jumping)
- Lumbar hyperlordosis, possibly leading to shortening of the hip-flexor muscles. This has an effect on overall posture and a manner of walking, stepping, or running.
- Muscle contractures of Achilles tendon and hamstrings impair functionality because the muscle fibers shorten and fibrose in connective tissue
- Progressive difficulty walking
- Muscle fiber deformities
- Pseudohypertrophy (enlarging) of tongue and calf muscles. The muscle tissue is eventually replaced by fat and connective tissue, hence the term pseudohypertrophy.
- Higher risk of neurobehavioral disorders (e.g., ADHD), learning disorders (dyslexia), and non-progressive weaknesses in specific cognitive skills (in particular short-term verbal memory), which are believed to be the result of absent or dysfunctional dystrophin in the brain.
- Eventual loss of ability to walk (usually by the age of 12)
- Skeletal deformities (including scoliosis in some cases)
- Trouble getting up from lying or sitting position

The condition can often be observed clinically from the moment the patient takes his first steps, and the ability to walk usually completely disintegrates between the time the boy is 9 to 12 years of age. Most men affected with DMD become essentially "paralyzed from the neck down" by the age of 21. Muscle wasting begins in the legs and pelvis, then progresses to the muscles of the shoulders and neck, followed by loss of arm muscles and respiratory muscles. Calf muscle enlargement (pseudohypertrophy) is quite obvious. Cardiomyopathy particularly (dilated cardiomyopathy) is common, but the development of congestive heart failure or arrhythmia (irregular heartbeat) is only occasional.

A positive Gowers' sign reflects the more severe impairment of the lower extremities muscles. The child helps himself to get up with upper extremities: first by rising to stand on his arms and knees, and then "walking" his hands up his legs to stand upright. Affected children usually tire more easily and have less overall strength than their peers. Creatine kinase (CPK-MM) levels in the bloodstream are extremely high. An electromyography (EMG) shows that weakness is caused by destruction of muscle tissue rather than by damage to nerves. Genetic testing can reveal genetic errors in the Xp21 gene. A muscle biopsy (immunohistochemistry or immunoblotting) or genetic test (blood test) confirms the absence of dystrophin, although improvements in genetic testing often make this unnecessary.
- Abnormal heart muscle (cardiomyopathy)
- Congestive heart failure or irregular heart rhythm (arrhythmia)
- Deformities of the chest and back (scoliosis)
- Enlarged muscles of the calves, buttocks, and shoulders (around age 4 or 5). These muscles are eventually replaced by fat and connective tissue (pseudohypertrophy).
- Loss of muscle mass (atrophy)
- Muscle contractures in the heels, legs
- Muscle deformities
- Respiratory disorders, including pneumonia and swallowing with food or fluid passing into the lungs (in late stages of the disease)

### C. Causes

Duchenne muscular dystrophy (DMD) is caused by a mutation of the dystrophin gene at locus Xp21, located on the short arm of the X chromosome. Dystrophin is responsible for connecting the cytoskeleton of each muscle fiber to the underlying basal lamina (extracellular matrix), through a protein complex containing many subunits. The absence of dystrophin permits excess calcium to penetrate the sarcolemma (the cell membrane). Alterations in calcium and signalling pathways cause water to enter into the mitochondria, which then burst.

In skeletal muscle dystrophy, mitochondrial dysfunction gives rise to an amplification of stress-induced cytosolic calcium signals and an amplification of stress-induced reactiveoxygen species (ROS) production. In a complex cascading process that involves several pathways and is not clearly understood, increased oxidative stress within the cell damages the sarcolemma and eventually results in the death of the cell. Muscle fibers undergo necrosis and are ultimately replaced with adipose and connective tissue.

DMD is inherited in an X-linked recessive pattern. Females will typically be carriers for the disease while males will be affected. Typically, a female carrier will be unaware they carry a mutation until they have an affected son. The son of a carrier mother has a 50% chance of inheriting the defective gene from his mother. The daughter of a carrier mother has a 50% chance of being a carrier and a 50% chance of having two normal copies of the gene. In all cases, an unaffected father will either pass a normal Y to his son or a normal X to his daughter. Female carriers of an X-linked recessive condition, such as DMD, can show symptoms depending on their pattern of X-inactivation.

Duchenne muscular dystrophy has an incidence of 1 in 3,500 male infants. Mutations within the dystrophin gene can either be inherited or occur spontaneously during germline transmission.

### D. Diagnosis

Genetic counseling is advised for people with a family history of the disorder. Duchenne muscular dystrophy can be detected with about 95% accuracy by genetic studies performed during pregnancy.

**DNA test.** The muscle-specific isoform of the dystrophin gene is composed of 79 exons, and DNA testing and analysis can usually identify the specific type of mutation of the exon or exons that are affected. DNA testing confirms the diagnosis in most cases.

**Muscle biopsy.** If DNA testing fails to find the mutation, a muscle biopsy test may be performed. A small sample of muscle tissue is extracted (usually with a scalpel instead of a needle) and a dye is applied that reveals the presence of dystrophin. Complete absence of the protein indicates the condition.

Over the past several years DNA tests have been developed that detect more of the many mutations that cause the condition, and muscle biopsy is not required as often to confirm the presence of Duchenne's.

**Prenatal tests.** DMD is carried by an X-linked recessive gene. Males have only one X chromosome, so one copy of the mutated gene will cause DMD. Fathers cannot pass X-linked traits on to their sons, so the mutation is transmitted by the mother.

If the mother is a carrier, and therefore one of her two X chromosomes has a DMD mutation, there is a 50% chance that a female child will inherit that mutation as one of her two X chromosomes, and be a carrier. There is a 50% chance that a male child will inherit that mutation as his one X chromosome, and therefore have DMD.

Prenatal tests can tell whether their unborn child has the most common mutations. There are many mutations responsible for DMD, and some have not been identified, so genetic testing only works when family members with DMD have a mutation that has been identified.

Prior to invasive testing, determination of the fetal sex is important; while males are sometimes affected by this X-linked disease, female DMD is extremely rare. This can be achieved by ultrasound scan at 16 weeks or more recently by free fetal DNA testing. Chorion villus sampling (CVS) can be done at 11-14 weeks, and has a 1% risk of miscarriage. Amniocentesis can be done after 15 weeks, and has a 0.5% risk of miscarriage. Fetal blood sampling can be done at about 18 weeks. Another option in the case of unclear genetic test results is fetal muscle biopsy.

### E. Treatment

There is no current cure for DMD, and an ongoing medical need has been recognized by regulatory authorities. Phase 1-2a trials with exon skipping treatment for certain mutations have halted decline and produced small clinical improvements in walking. Treatment is generally aimed at controlling the onset of symptoms to maximize the quality of life, and include the following:
- Corticosteroids such as prednisolone and deflazacort increase energy and strength and defer severity of some symptoms.
- Randomised control trials have shown that beta-2-agonists increase muscle strength but do not modify disease progression. Follow-up time for most RCTs on beta2-agonists is only around 12 months and hence results cannot be extrapolated beyond that time frame.
- Mild, non-jarring physical activity such as swimming is encouraged. Inactivity (such as bed rest) can worsen the muscle disease.
- Physical therapy is helpful to maintain muscle strength, flexibility, and function.
- Orthopedic appliances (such as braces and wheelchairs) may improve mobility and the ability for self-care. Form-fitting removable leg braces that hold the ankle in place during sleep can defer the onset of contractures.
- Appropriate respiratory support as the disease progresses is important.

Comprehensive multi-disciplinary care standards/guidelines for DMD have been developed by the Centers for Disease Control and Prevention (CDC), and were published in two parts in The Lancet Neurology in 2010. To download the two articles in PDF format, go to the TREAT-NMD website.

### 1. Physical Therapy

Physical therapists are concerned with enabling patients to reach their maximum physical potential. Their aim is to:
- minimize the development of contractures and deformity by developing a programme of stretches and exercises where appropriate
- anticipate and minimize other secondary complications of a physical nature by recommending bracing and durable medical equipment
- monitor respiratory function and advise on techniques to assist with breathing exercises and methods of clearing secretions

### 2. Respiration Assistance

Modern "volume ventilators/respirators," which deliver an adjustable volume (amount) of air to the person with each breath, are valuable in the treatment of people with muscular dystrophy related respiratory problems. The ventilator may require an invasive endotracheal or tracheotomy tube through which air is directly delivered, but, for some people non-invasive delivery through a face mask or mouthpiece is sufficient. Positive airway pressure machines, particularly bi-level ones, are sometimes used in this latter way. The respiratory equipment may easily fit on a ventilator tray on the bottom or back of a power wheelchair with an external battery for portability.

Ventilator treatment may start in the mid to late teens when the respiratory muscles can begin to collapse. If the vital capacity has dropped below 40% of normal, a volume ventilator/respirator may be used during sleeping hours, a time when the person is most likely to be under ventilating ("hypoventilating"). Hypoventilation during sleep is determined by a thorough history of sleep disorder with an oximetry study and a capillary blood gas (See Pulmonary Function Testing). A cough assist device can help with excess mucus in lungs by hyperinflation of the lungs with positive air pressure, then negative pressure to get the mucus up. If the vital capacity continues to decline to less than 30 percent of normal, a volume ventilator/respirator may also be needed during the day for more assistance. The person gradually will increase the amount of time using the ventilator/respirator during the day as needed.

### F. Prognosis

Duchenne muscular dystrophy is a progressive disease which eventually affects all voluntary muscles and involves the heart and breathing muscles in later stages. The life expectancy is currently estimated to be around 25, but this varies from patient to patient. Recent advancements in medicine are extending the lives of those afflicted. The *Muscular Dystrophy Campaign,* which is a leading UK charity focusing on all muscle disease, states that "with high standards of medical care young men with Duchenne muscular dystrophy are often living well into their 30s."

In rare cases, persons with DMD have been seen to survive into the forties or early fifties, with the use of proper positioning in wheelchairs and beds, ventilator support (via tracheostomy or mouthpiece), airway clearance, and heart medications, if required. Early planning of the required supports for later-life care has shown greater longevity in people living with DMD.

Curiously, in the mdx mouse model of Duchenne muscular dystrophy, the lack of dystrophin is associated with increased calcium levels and skeletal muscle myonecrosis. The intrinsic laryngeal muscles (ILM) are protected and do not undergo myonecrosis. ILM have a calcium regulation system profile suggestive of a better ability to handle calcium changes in comparison to outher muscles, and this may provide a mechanistic insight for their unique pathophysiological properties. The ILM may facilitate the development of novel strategies for the prevention and treatment of muscle wasting in a variety of clinical scenarios.

### II. CRISPR/CAS System

### A. CRISPR/CAS

CRISPRs (clustered regularly interspaced short palindromic repeats) are DNA loci containing short repetitions of base sequences. Each repetition is followed by short segments of "spacer DNA" from previous exposures to a virus. CRISPRs are found in approximately 40% of sequenced eubacteria genomes and 90% of sequenced archaea. CRISPRs are often associated with cas genes that code for proteins related to CRISPRs. The CRISPR/Cas system is a prokaryotic immune system that confers resistance to foreign genetic elements such as plasmids and phages and provides a form of acquired immunity. CRISPR spacers recognize and silence these exogenous genetic elements like RNAi in eukaryotic organisms.

Repeats were first described in 1987 for the bacterium *Escherichia coli.* In 2000, similar clustered repeats were identified in additional bacteria and archaea and were termed Short Regularly Spaced Repeats (SRSR). SRSR were renamed CRISPR in 2002. A set of genes, some encoding putative nuclease or helicase proteins, were found to be associated with CRISPR repeats (the *cas,* or *CRISPR-associated* genes).

In 2005, three independent researchers showed that CRISPR spacers showed homology to several phage DNA and extrachromosomal DNA such as plasmids. This was an indication that the CRISPR/cas system could have a role in adaptive immunity in bacteria. Koonin and colleagues proposed that spacers serve as a template for RNA molecules, analogously to eukaryotic cells that use a system called RNA interference.

In 2007 Barrangou, Horvath (food industry scientists at Danisco) and others showed that they could alter the resistance of *Streptococcus thermophilus* to phage attack with spacer DNA. Doudna and Charpentier had independently been exploring CRISPR-associated proteins to learn how bacteria deploy spacers in their immune defenses. They jointly studied a simpler CRISPR system that relies on a protein called Cas9. They found that bacteria respond to an invading phage by transcribing spacers and palindromic DNA into a long RNA molecule that the cell then uses tracrRNA and Cas9 to cut it into pieces called crRNAs.

CRISPR was first shown to work as a genome engineering/editing tool in human cell culture by 2012 It has since been used in a wide range of organisms including bakers yeast (S. *cerevisiae*)*,* zebra fish, nematodes (C. *elegans*)*,* plants, mice, and several other organisms. Additionally CRISPR has been modified to make programmable transcription factors that allow scientists to target and activate or silence specific genes. Libraries of tens of thousands of guide RNAs are now available.

The first evidence that CRISPR can reverse disease symptoms in living animals was demonstrated in March 2014, when MIT researchers cured mice of a rare liver disorder. Since 2012, the CRISPR/Cas system has been used for gene editing (silencing, enhancing or changing specific genes) that even works in eukaryotes like mice and primates. By inserting a plasmid containing cas genes and specifically designed CRISPRs, an organism's genome can be cut at any desired location.

CRISPR repeats range in size from 24 to 48 base pairs. They usually show some dyad symmetry, implying the formation of a secondary structure such as a hairpin, but are not truly palindromic. Repeats are separated by spacers of similar length. Some CRISPR spacer sequences exactly match sequences from plasmids and phages, although some spacers match the prokaryote's genome (self-targeting spacers). New spacers can be added rapidly in response to phage infection.

CRISPR-associated (*cas*) genes are often associated with CRISPR repeat-spacer arrays. As of 2013, more than forty different Cas protein families had been described. Of these protein families, Cas1 appears to be ubiquitous among different CRISPR/Cas systems. Particular combinations of *cas* genes and repeat structures have been used to define 8 CRISPR subtypes (Ecoli, Ypest, Nmeni, Dvulg, Tneap, Hmari, Apern, and Mtube), some of which are associated with an additional gene module encoding repeat-associated mysterious proteins (RAMPs). More than one CRISPR subtype may occur in a single genome. The sporadic distribution of the CRISPR/Cas subtypes suggests that the system is subject to horizontal gene transfer during microbial evolution.

Exogenous DNA is apparently processed by proteins encoded by Cas genes into small elements (~30 base pairs in length), which are then somehow inserted into the CRISPR locus near the leader sequence. RNAs from the CRISPR loci are constitutively expressed and are processed by Cas proteins to small RNAs composed of individual, exogenously-derived sequence elements with a flanking repeat sequence. The RNAs guide other Cas proteins to silence exogenous genetic elements at the RNA or DNA level. Evidence suggests functional diversity among CRISPR subtypes. The Cse (Cas subtype *Ecoli)* proteins (called CasA-E in *E. coli*) form a functional complex, Cascade, that processes CRISPR RNA transcripts into spacer-repeat units that Cascade retains. In other prokaryotes, Cas6 processes the CRISPR transcripts. Interestingly, CRISPR-based phage inactivation in *E. coli* requires Cascade and Cas3, but not Cas1 and Cas2. The Cmr (Cas RAMP module) proteins found in *Pyrococcus furiosus* and other prokaryotes form a functional complex with small CRISPR RNAs that recognizes and cleaves complementary target RNAs. RNA-guided CRISPR enzymes are classified as type V restriction enzymes.

See also U.S. Patent Publication 2014/0068797.

### B. Cas9

Cas9 is a nuclease, an enzyme specialized for cutting DNA, with two active cutting sites, one for each strand of the double helix. The team demonstrated that they could disable one or both sites while preserving Cas9's ability to home located its target DNA. Jinek *et al.* (2012) combined tracrRNA and spacer RNA into a "single-guide RNA" molecule that, mixed with Cas9, could find and cut the correct DNA targets. Jinek *et al.* (2012) proposed that such synthetic guide RNAs might be able to be used for gene editing.

Cas9 proteins are highly enriched in pathogenic and commensal bacteria. CRISPR/Cas-mediated gene regulation may contribute to the regulation of endogenous bacterial genes, particularly during bacterial interaction with eukaryotic hosts. For example, Cas protein Cas9 of *Francisella novicida* uses a unique, small, CRISPR/Cas-associated RNA (scaRNA) to repress an endogenous transcript encoding a bacterial lipoprotein that is critical for *F. novicida* to dampen host response and promote virulence. Wang *et al.* showed that coinjection of Cas9 mRNA and sgRNAs into the germline (zygotes) generated nice with mutations. Delivery of Cas9 DNA sequences also is contemplated.

### C. gRNA

As an RNA guided protein, Cas9 requires a short RNA to direct the recognition of DNA targets (Mali *et al.,* 2013a). Though Cas9 preferentially interrogates DNA sequences containing a PAM sequence NGG it can bind here without a protospacer target. However, the Cas9-gRNA complex requires a close match to the gRNA to create a double strand break (Cho *et al.,* 2013; Hsu *et al.,* 2013). CRISPR sequences in bacteria are expressed in multiple RNAs and then processed to create guide strands for RNA (Bikard *et al.,* 2013). Because Eukaryotic systems lack some of the proteins required to process CRISPR RNAs the synthetic construct gRNA was created to combine the essential pieces of RNA for Cas9 targeting into a single RNA expressed with the RNA polymerase type III promoter U6 (Mali *et al.,* 2013a, b). Synthetic gRNAs are slightly over 100bp at the minimum length and contain a portion which is targets the 20 protospacer nucleotides immediately preceding the PAM sequence NGG; gRNAs do not contain a PAM sequence.

### III. Nucleic Acid Delivery

As discussed above, in certain embodiments, expression cassettes are employed to express a transcription factor product, either for subsequent purification and delivery to a cell/subject, or for use directly in a genetic-based delivery approach. Expression requires that appropriate signals be provided in the vectors, and include various regulatory elements such as enhancers/promoters from both viral and mammalian sources that drive expression of the genes of interest in cells. Elements designed to optimize messenger RNA stability and translatability in host cells also are defined. The conditions for the use of a number of dominant drug selection markers for establishing permanent, stable cell clones expressing the products are also provided, as is an element that links expression of the drug selection markers to expression of the polypeptide.

### A. Regulatory Elements

Throughout this application, the term "expression cassette" is meant to include any type of genetic construct containing a nucleic acid coding for a gene product in which part or all of the nucleic acid encoding sequence is capable of being transcribed and translated, *i.e.,* is under the control of a promoter. A "promoter" refers to a DNA sequence recognized by the synthetic machinery of the cell, or introduced synthetic machinery, required to initiate the specific transcription of a gene. The phrase "under transcriptional control" means that the promoter is in the correct location and orientation in relation to the nucleic acid to control RNA polymerase initiation and expression of the gene. An "expression vector" is meant to include expression cassettes comprised in a genetic construct that is capable of replication, and thus including one or more of origins of replication, transcription termination signals, poly-A regions, selectable markers, and multipurpose cloning sites.

The term promoter will be used here to refer to a group of transcriptional control modules that are clustered around the initiation site for RNA polymerase II. Much of the thinking about how promoters are organized derives from analyses of several viral promoters, including those for the HSV thymidine kinase *(tk)* and SV40 early transcription units. These studies, augmented by more recent work, have shown that promoters are composed of discrete functional modules, each consisting of approximately 7-20 bp of DNA, and containing one or more recognition sites for transcriptional activator or repressor proteins.

At least one module in each promoter functions to position the start site for RNA synthesis. The best known example of this is the TATA box, but in some promoters lacking a TATA box, such as the promoter for the mammalian terminal deoxynucleotidyl transferase gene and the promoter for the SV40 late genes, a discrete element overlying the start site itself helps to fix the place of initiation.

Additional promoter elements regulate the frequency of transcriptional initiation. Typically, these are located in the region 30-110 bp upstream of the start site, although a number of promoters have recently been shown to contain functional elements downstream of the start site as well. The spacing between promoter elements frequently is flexible, so that promoter function is preserved when elements are inverted or moved relative to one another. In the tk promoter, the spacing between promoter elements can be increased to 50 bp apart before activity begins to decline. Depending on the promoter, it appears that individual elements can function either co-operatively or independently to activate transcription.

In certain embodiments, viral promotes such as the human cytomegalovirus (CMV) immediate early gene promoter, the SV40 early promoter, the Rous sarcoma virus long terminal repeat, rat insulin promoter and glyceraldehyde-3-phosphate dehydrogenase can be used to obtain high-level expression of the coding sequence of interest. The use of other viral or mammalian cellular or bacterial phage promoters which are well-known in the art to achieve expression of a coding sequence of interest is contemplated as well, provided that the levels of expression are sufficient for a given purpose. By employing a promoter with well-known properties, the level and pattern of expression of the protein of interest following transfection or transformation can be optimized. Further, selection of a promoter that is regulated in response to specific physiologic signals can permit inducible expression of the gene product.

Enhancers are genetic elements that increase transcription from a promoter located at a distant position on the same molecule of DNA. Enhancers are organized much like promoters. That is, they are composed of many individual elements, each of which binds to one or more transcriptional proteins. The basic distinction between enhancers and promoters is operational. An enhancer region as a whole must be able to stimulate transcription at a distance; this need not be true of a promoter region or its component elements. On the other hand, a promoter must have one or more elements that direct initiation of RNA synthesis at a particular site and in a particular orientation, whereas enhancers lack these specificities. Promoters and enhancers are often overlapping and contiguous, often seeming to have a very similar modular organization.

Below is a list of promoters/enhancers and inducible promoters/enhancers that could be used in combination with the nucleic acid encoding a gene of interest in an expression construct. Additionally, any promoter/enhancer combination (as per the Eukaryotic Promoter Data Base EPDB) could also be used to drive expression of the gene. Eukaryotic cells can support cytoplasmic transcription from certain bacterial promoters if the appropriate bacterial polymerase is provided, either as part of the delivery complex or as an additional genetic expression construct.

| **TABLE A** | |
|---|---|
| **Promoter and/or Enhancer** | |
| Promoter/Enhancer | References |
| Immunoglobulin Heavy Chain | Banerji *et al.,* 1983; Gilles *et al.,* 1983; Grosschedl *et al.,* 1985; Atchinson *et al.,* 1986, 1987; Imler *et al.,* 1987; Weinberger *et al.,* 1984; Kiledjian *et al.,* 1988; Porton *et al.*; 1990 |
| Immunoglobulin Light Chain | Queen and Baltimore, 1983; Picard *et al.,* 1984 |
| T-Cell Receptor | Luria *et al.,* 1987; Winoto *et al.,* 1989; Redondo *et al.*; 1990 |
| HLA DQ a and/or DQ β | Sullivan *et al.,* 1987 |
| β-Interferon | Goodbourn *et al.,* 1986; Fujita *et al.,* 1987; Goodbourn and Maniatis *et al.,* 1988 |
| Interleukin-2 | Greene *et al.,* 1989 |
| Interleukin-2 Receptor | Greene *et al.,* 1989; Lin *et al.,* 1990 |
| MHC Class II 5 | Koch *et al.,* 1989 |
| MHC Class II HLA-DRa | Sherman *et al.,* 1989 |
| β-Actin | Kawamoto *et al.,* 1988; Ng *et al.*; 1989 |
| Muscle Creatine Kinase (MCK) | Jaynes *et al.,* 1988; Horlick *et al.,* 1989; Johnson *et al.,* 1989 |
| Prealbumin (Transthyretin) | Costa *et al.,* 1988 |
| Elastase I | Ornitz *et al.,* 1987 |
| Metallothionein (MTII) | Karin *et al.,* 1987; Culotta *et al.,* 1989 |
| Collagenase | Pinkert *et al.,* 1987; Angel *et al.,* 1987a |
| Albumin | Pinkert *et al.,* 1987; Tronche *et al.,* 1989, 1990 |
| α-Fetoprotein | Godbout *et al.,* 1988; Campere and Tilghman *et al.,* 1989 |
| t-Globin | Bodine and Ley *et al.,* 1987; Perez-Stable *et al.,* 1990 |
| β-Globin | Trudel *et al.,* 1987 |
| c-fos | Cohen *et al.,* 1987 |
| *c-HA-ras* | Triesman, 1986; Deschamps *et al.,* 1985 |
| Insulin | Edlund *et al.,* 1985 |
| Neural Cell Adhesion Molecule (NCAM) | Hirsh *et al.,* 1990 |
| α₁-Antitrypain | Latimer *et al.,* 1990 |
| H2B (TH2B) Histone | Hwang *et al.,* 1990 |
| Mouse and/or Type I Collagen | Ripe *et al.,* 1989 |
| Glucose-Regulated Proteins (GRP94 and GRP78) | Chang *et al.,* 1989 |
| Rat Growth Hormone | Larsen *et al.,* 1986 |
| Human Serum Amyloid A (SAA) | Edbrooke *et al.,* 1989 |
| Troponin I (TN I) | Yutzey *et al.,* 1989 |
| Platelet-Derived Growth Factor (PDGF) | Pech *et al.,* 1989 |
| Duchenne Muscular Dystrophy | Klamut *et al.,* 1990 |
| SV40 | Banerji *et al.,* 1981; Moreau *et al.,* 1981; Sleigh *et al.,* 1985; Firak *et al.,* 1986; Herr and Clarke *et al.,* 1986; Imbra and Karin *et al.,* 1986; Kadesch and Berg, 1986; Wang *et al.,* 1986; Ondek *et al.,* 1987; Kuhl *et al.,* 1987; Schaffner *et al.,* 1988 |
| Polyoma | Swartzendruber *et al.,* 1975; Vasseur *et al.,* 1980; Katinka *et al.,* 1980, 1981; Tyndell *et al.,* 1981; Dandolo *et al.,* 1983; de Villiers *et al.,* 1984; Hen *et al.,* 1986; Satake *et al.,* 1988; Campbell and Villarreal, 1988 |
| Retroviruses | Kriegler *et al.,* 1982, 1983; Levinson *et al.,* 1982; Kriegler *et al.,* 1983, 1984a, b, 1988; Bosze *et al.,* 1986; Miksicek *et al.,* 1986; Celander and Haseltine, 1987; Thiesen *et al.,* 1988; Celander *et al.,* 1988; Choi *et al.,* 1988; Reisman *et al.,* 1989 |
| Papilloma Virus | Campo *et al.,* 1983; Lusky *et al.,* 1983; Spandidos and/or Wilkie, 1983; Spalholz *et al.,* 1985; Lusky *et al.,* 1986; Cripe *et al.,* 1987; Gloss *et al.,* 1987; Hirochika *et al.,* 1987; Stephens *et al.,* 1987 |
| Hepatitis B Virus | Bulla and Siddiqui *et al.,* 1986; Jameel and Siddiqui, 1986; Shaul and Ben-Levy, 1987; Spandau *et al.,* 1988; Vannice *et al.,* 1988 |
| Human Immunodeficiency Virus | Muesing *et al.,* 1987; Hauber and Cullen *et al.,* 1988; Jakobovits *et al.,* 1988; Feng *et al.,* 1988; Takebe *et al.,* 1988; Rosen *et al.,* 1988; Berkhout *et al.,* 1989; Laspia *et al.,* 1989; Sharp *et al.,* 1989; Braddock *et al.,* 1989 |
| Cytomegalovirus (CMV) | Weber *et al.,* 1984; Boshart *et al.,* 1985; Foecking *et al.,* 1986 |
| Gibbon Ape Leukemia Virus | Holbrook *et al.,* 1987; Quinn *et al.,* 1989 |

| **TABLE B** | | |
|---|---|---|
| **Inducible Elements** | | |
| Element | Inducer | References |
| MT II | Phorbol Ester (TFA) Heavy metals | Palmiter *et al.,* 1982; Haslinger *et al.,* 1985; Searle *et al.,* 1985; Stuart *et al.,* 1985; Imagawa *et al.,* 1987, Karin *et al.,* 1987; Angel *et al.,* 1987b; McNeall *et al.,* 1989 |
| MMTV (mouse mammary tumor virus) | Glucocorticoids | Huang *et al.,* 1981; Lee *et al.,* 1981; Majors and Varmas *et al.,* 1983; Chandler *et al.,* 1983; Ponta *et al.,* 1985; Sakai *et al.,* 1988 |
| β-Interferon | poly(rI)x poly(rc) | Tavernier *et al.,* 1983 |
| Adenovirus 5 E2 | ElA | Imperiale *et al.,* 1984 |
| Collagenase | Phorbol Ester (TPA) | Angel *et al.,* 1987a |
| Stromelysin | Phorbol Ester (TPA) | Angel *et al.,* 1987b |
| SV40 | Phorbol Ester (TPA) | Angel *et al.,* 1987b |
| Murine MX Gene | Interferon, Newcastle Disease Virus | Hug *et al.,* 1988 |
| GRP78 Gene | A23187 | Resendez *et al.,* 1988 |
| α-2-Macroglobulin | IL-6 | Kunz *et al.,* 1989 |
| Vimentin | Serum | Rittling *et al.,* 1989 |
| MHC Class I Gene H-2κb | Interferon | Blanar *et al.,* 1989 |
| HSP70 | ElA, SV40 Large T Antigen | Taylor *et al.,* 1989, 1990a, 1990b |
| Proliferin | Phorbol Ester-TPA | Mordacq and Linzer, 1989 |
| Tumor Necrosis Factor | PMA | Hensel *et al.,* 1989 |
| Thyroid Stimulating Hormone α Gene | Thyroid Hormone | Chatterjee *et al.,* 1989 |

Of particular interest are muscle specific promoters. These include the myosin light chain-2 promoter (Franz *et al.,* 1994; Kelly *et al.,* 1995), the α-actin promoter (Moss *et al.,* 1996), the troponin 1 promoter (Bhavsar *et al.,* 1996); the Na⁺/Ca²⁺ exchanger promoter (Barnes *et al.,* 1997), the dystrophin promoter (Kimura *et al.,* 1997), the α7 integrin promoter (Ziober and Kramer, 1996), the brain natriuretic peptide promoter (LaPointe *et al.,* 1996) and the αB-crystallin/small heat shock protein promoter (Gopal-Srivastava, 1995), α-myosin heavy chain promoter (Yamauchi-Takihara *et al.,* 1989) and the ANF promoter (LaPointe *et al.,* 1988).

Where a cDNA insert is employed, one will typically desire to include a polyadenylation signal to effect proper polyadenylation of the gene transcript. The nature of the polyadenylation signal is not believed to be crucial to the successful practice of the invention, and any such sequence may be employed such as human growth hormone and SV40 polyadenylation signals. Also contemplated as an element of the expression cassette is a terminator. These elements can serve to enhance message levels and to minimize read through from the cassette into other sequences.

### B. 2A Peptide

The inventor utilizes the 2A-like self-cleaving domain from the insect virus Thosea asigna (TaV 2A peptide) (Chang *et al.,* 2009). These 2A-like domains have been shown to function across Eukaryotes and cause cleavage of amino acids to occur co-translationally within the 2A-like peptide domain. Therefore, inclusion of TaV 2A peptide allows the expression of multiple proteins from a single mRNA transcript. Importantly, the domain of TaV when tested in eukaryotic systems have shown greater than 99% cleavage activity (Donnelly *et al.,* 2001).

### C. Delivery of Expression Vectors

There are a number of ways in which expression vectors may introduced into cells. In certain embodiments of the invention, the expression construct comprises a virus or engineered construct derived from a viral genome. The ability of certain viruses to enter cells via receptor-mediated endocytosis, to integrate into host cell genome and express viral genes stably and efficiently have made them attractive candidates for the transfer of foreign genes into mammalian cells (Ridgeway, 1988; Nicolas and Rubenstein, 1988; Baichwal and Sugden, 1986; Temin, 1986). The first viruses used as gene vectors were DNA viruses including the papovaviruses (simian virus 40, bovine papilloma virus, and polyoma) (Ridgeway, 1988; Baichwal and Sugden, 1986) and adenoviruses (Ridgeway, 1988; Baichwal and Sugden, 1986). These have a relatively low capacity for foreign DNA sequences and have a restricted host spectrum. Furthermore, their oncogenic potential and cytopathic effects in permissive cells raise safety concerns. They can accommodate only up to 8 kB of foreign genetic material but can be readily introduced in a variety of cell lines and laboratory animals (Nicolas and Rubenstein, 1988; Temin, 1986).

One of the preferred methods for *in vivo* delivery involves the use of an adenovirus expression vector. "Adenovirus expression vector" is meant to include those constructs containing adenovirus sequences sufficient to (a) support packaging of the construct and (b) to express an antisense polynucleotide that has been cloned therein. In this context, expression does not require that the gene product be synthesized.

The expression vector comprises a genetically engineered form of adenovirus. Knowledge of the genetic organization of adenovirus, a 36 kB, linear, double-stranded DNA virus, allows substitution of large pieces of adenoviral DNA with foreign sequences up to 7 kB (Grunhaus and Horwitz, 1992). In contrast to retrovirus, the adenoviral infection of host cells does not result in chromosomal integration because adenoviral DNA can replicate in an episomal manner without potential genotoxicity. Also, adenoviruses are structurally stable, and no genome rearrangement has been detected after extensive amplification. Adenovirus can infect virtually all epithelial cells regardless of their cell cycle stage. So far, adenoviral infection appears to be linked only to mild disease such as acute respiratory disease in humans.

Adenovirus is particularly suitable for use as a gene transfer vector because of its midsized genome, ease of manipulation, high titer, wide target cell range and high infectivity. Both ends of the viral genome contain 100-200 base pair inverted repeats (ITRs), which are *cis* elements necessary for viral DNA replication and packaging. The early (E) and late (L) regions of the genome contain different transcription units that are divided by the onset of viral DNA replication. The E1 region (E1A and E1B) encodes proteins responsible for the regulation of transcription of the viral genome and a few cellular genes. The expression of the E2 region (E2A and E2B) results in the synthesis of the proteins for viral DNA replication. These proteins are involved in DNA replication, late gene expression and host cell shut-off (Renan, 1990). The products of the late genes, including the majority of the viral capsid proteins, are expressed only after significant processing of a single primary transcript issued by the major late promoter (MLP). The MLP, (located at 16.8 m.u.) is particularly efficient during the late phase of infection, and all the mRNA's issued from this promoter possess a 5'-tripartite leader (TPL) sequence which makes them preferred mRNA's for translation.

In one system, recombinant adenovirus is generated from homologous recombination between shuttle vector and provirus vector. Due to the possible recombination between two proviral vectors, wild-type adenovirus may be generated from this process. Therefore, it is critical to isolate a single clone of virus from an individual plaque and examine its genomic structure.

Generation and propagation of the current adenovirus vectors, which are replication deficient, depend on a unique helper cell line, designated 293, which was transformed from human embryonic kidney cells by Ad5 DNA fragments and constitutively expresses E1 proteins (Graham *et al.,* 1977). Since the E3 region is dispensable from the adenovirus genome (Jones and Shenk, 1978), the current adenovirus vectors, with the help of 293 cells, carry foreign DNA in either the E1, the D3 or both regions (Graham and Prevec, 1991). In nature, adenovirus can package approximately 105% of the wild-type genome (Ghosh-Choudhury *et al.,* 1987), providing capacity for about 2 extra kb of DNA. Combined with the approximately 5.5 kb of DNA that is replaceable in the E1 and E3 regions, the maximum capacity of the current adenovirus vector is under 7.5 kb, or about 15% of the total length of the vector. More than 80% of the adenovirus viral genome remains in the vector backbone and is the source of vector-borne cytotoxicity. Also, the replication deficiency of the E1-deleted virus is incomplete.

Helper cell lines may be derived from human cells such as human embryonic kidney cells, muscle cells, hematopoietic cells or other human embryonic mesenchymal or epithelial cells. Alternatively, the helper cells may be derived from the cells of other mammalian species that are permissive for human adenovirus. Such cells include, *e.g.,* Vero cells or other monkey embryonic mesenchymal or epithelial cells. As stated above, the preferred helper cell line is 293.

Racher *et al.* (1995) disclosed improved methods for culturing 293 cells and propagating adenovirus. In one format, natural cell aggregates are grown by inoculating individual cells into 1 liter siliconized spinner flasks (Techne, Cambridge, UK) containing 100-200 ml of medium. Following stirring at 40 rpm, the cell viability is estimated with trypan blue. In another format, Fibra-Cel microcarriers (Bibby Sterlin, Stone, UK) (5 g/l) is employed as follows. A cell inoculum, resuspended in 5 ml of medium, is added to the carrier (50 ml) in a 250 ml Erlenmeyer flask and left stationary, with occasional agitation, for 1 to 4 h. The medium is then replaced with 50 ml of fresh medium and shaking initiated. For virus production, cells are allowed to grow to about 80% confluence, after which time the medium is replaced (to 25% of the final volume) and adenovirus added at an MOI of 0.05. Cultures are left stationary overnight, following which the volume is increased to 100% and shaking commenced for another 72 h.

Other than the requirement that the adenovirus vector be replication defective, or at least conditionally defective, the nature of the adenovirus vector is not believed to be crucial to the successful practice of the invention. The adenovirus may be of any of the 42 different known serotypes or subgroups A-F. Adenovirus type 5 of subgroup C is the preferred starting material in order to obtain the conditional replication-defective adenovirus vector for use in the present invention. This is because Adenovirus type 5 is a human adenovirus about which a great deal of biochemical and genetic information is known, and it has historically been used for most constructions employing adenovirus as a vector.

As stated above, the typical vector according to the present invention is replication defective and will not have an adenovirus E1 region. Thus, it will be most convenient to introduce the polynucleotide encoding the gene of interest at the position from which the E1-coding sequences have been removed. However, the position of insertion of the construct within the adenovirus sequences is not critical to the invention. The polynucleotide encoding the gene of interest may also be inserted in lieu of the deleted E3 region in E3 replacement vectors, as described by Karlsson *et al.* (1986), or in the E4 region where a helper cell line or helper virus complements the E4 defect.

Adenovirus is easy to grow and manipulate and exhibits broad host range *in vitro* and *in vivo.* This group of viruses can be obtained in high titers, *e.g.,* 10⁹-10¹² plaque-forming units per ml, and they are highly infective. The life cycle of adenovirus does not require integration into the host cell genome. The foreign genes delivered by adenovirus vectors are episomal and, therefore, have low genotoxicity to host cells. No side effects have been reported in studies of vaccination with wild-type adenovirus (Couch *et al.,* 1963; Top *et al.,* 1971), demonstrating their safety and therapeutic potential as *in vivo* gene transfer vectors.

Adenovirus vectors have been used in eukaryotic gene expression (Levrero *et al.,* 1991; Gomez-Foix *et al.,* 1992) and vaccine development (Grunhaus and Horwitz, 1992; Graham and Prevec, 1991). Animal studies suggested that recombinant adenovirus could be used for gene therapy (Stratford-Perricaudet and Perricaudet, 1991; Stratford-Perricaudet *et al.,* 1990; Rich *et al.,* 1993). Studies in administering recombinant adenovirus to different tissues include trachea instillation (Rosenfeld *et al.,* 1991; Rosenfeld *et al.,* 1992), muscle injection (Ragot *et al.,* 1993), peripheral intravenous injections (Herz and Gerard, 1993) and stereotactic inoculation into the brain (Le Gal La Salle *et al.,* 1993).

The retroviruses are a group of single-stranded RNA viruses characterized by an ability to convert their RNA to double-stranded DNA in infected cells by a process of reverse-transcription (Coffin, 1990). The resulting DNA then stably integrates into cellular chromosomes as a provirus and directs synthesis of viral proteins. The integration results in the retention of the viral gene sequences in the recipient cell and its descendants. The retroviral genome contains three genes, gag, pol, and env that code for capsid proteins, polymerase enzyme, and envelope components, respectively. A sequence found upstream from the gag gene contains a signal for packaging of the genome into virions. Two long terminal repeat (LTR) sequences are present at the 5' and 3' ends of the viral genome. These contain strong promoter and enhancer sequences and are also required for integration in the host cell genome (Coffin, 1990).

In order to construct a retroviral vector, a nucleic acid encoding a gene of interest is inserted into the viral genome in the place of certain viral sequences to produce a virus that is replication-defective. In order to produce virions, a packaging cell line containing the gag, pol, and env genes but without the LTR and packaging components is constructed (Mann *et al.,* 1983). When a recombinant plasmid containing a cDNA, together with the retroviral LTR and packaging sequences is introduced into this cell line (by calcium phosphate precipitation for example), the packaging sequence allows the RNA transcript of the recombinant plasmid to be packaged into viral particles, which are then secreted into the culture media (Nicolas and Rubenstein, 1988; Temin, 1986; Mann *et al.,* 1983). The media containing the recombinant retroviruses is then collected, optionally concentrated, and used for gene transfer. Retroviral vectors are able to infect a broad variety of cell types. However, integration and stable expression require the division of host cells (Paskind *et al.,* 1975).

A novel approach designed to allow specific targeting of retrovirus vectors was recently developed based on the chemical modification of a retrovirus by the chemical addition of lactose residues to the viral envelope. This modification could permit the specific infection of hepatocytes via sialoglycoprotein receptors.

A different approach to targeting of recombinant retroviruses was designed in which biotinylated antibodies against a retroviral envelope protein and against a specific cell receptor were used. The antibodies were coupled via the biotin components by using streptavidin (Roux *et al.,* 1989). Using antibodies against major histocompatibility complex class I and class II antigens, they demonstrated the infection of a variety of human cells that bore those surface antigens with an ecotropic virus *in vitro* (Roux *et al.,* 1989).

There are certain limitations to the use of retrovirus vectors in all aspects of the present invention. For example, retrovirus vectors usually integrate into random sites in the cell genome. This can lead to insertional mutagenesis through the interruption of host genes or through the insertion of viral regulatory sequences that can interfere with the function of flanking genes (Varmus *et al.,* 1981). Another concern with the use of defective retrovirus vectors is the potential appearance of wild-type replication-competent virus in the packaging cells. This can result from recombination events in which the intact- sequence from the recombinant virus inserts upstream from the gag, pol, env sequence integrated in the host cell genome. However, new packaging cell lines are now available that should greatly decrease the likelihood of recombination (Markowitz *et al.,* 1988; Hersdorffer *et al.,* 1990).

Other viral vectors may be employed as expression constructs in the present disclosure.

Vectors derived from viruses such as vaccinia virus (Ridgeway, 1988; Baichwal and Sugden, 1986; Coupar *et al.,* 1988) adeno-associated virus (AAV) (Ridgeway, 1988; Baichwal and Sugden, 1986; Hermonat and Muzycska, 1984) and herpesviruses may be employed. They offer several attractive features for various mammalian cells (Friedmann, 1989; Ridgeway, 1988; Baichwal and Sugden, 1986; Coupar *et al.,* 1988; Horwich *et al.,* 1990).

In order to effect expression of sense or antisense gene constructs, the expression construct must be delivered into a cell. This delivery may be accomplished *in vitro,* as in laboratory procedures for transforming cells lines, or *in vivo* or *ex vivo,* as in the treatment of certain disease states. One mechanism for delivery is via viral infection where the expression construct is encapsidated in an infectious viral particle.

Several non-viral methods for the transfer of expression constructs into cultured mammalian cells also are contemplated by the present disclosure.

These include calcium phosphate precipitation (Graham and Van Der Eb, 1973; Chen and Okayama, 1987; Rippe *et al.,* 1990) DEAE-dextran (Gopal, 1985), electroporation (Tur-Kaspa *et al.,* 1986; Potter *et al.,* 1984), direct microinjection (Harland and Weintraub, 1985), DNA-loaded liposomes (Nicolau and Sene, 1982; Fraley *et al.,* 1979) and lipofectamine-DNA complexes, cell sonication (Fechheimer *et al.,* 1987), gene bombardment using high velocity microprojectiles (Yang *et al.,* 1990), and receptor-mediated transfection (Wu and Wu, 1987; Wu and Wu, 1988). Some of these techniques may be successfully adapted for *in vivo* or *ex vivo* use.

Once the expression construct has been delivered into the cell the nucleic acid encoding the gene of interest may be positioned and expressed at different sites. In certain embodiments, the nucleic acid encoding the gene may be stably integrated into the genome of the cell. This integration may be in the cognate location and orientation via homologous recombination (gene replacement) or it may be integrated in a random, non-specific location (gene augmentation). In yet further embodiments, the nucleic acid may be stably maintained in the cell as a separate, episomal segment of DNA. Such nucleic acid segments or "episomes" encode sequences sufficient to permit maintenance and replication independent of or in synchronization with the host cell cycle. How the expression construct is delivered to a cell and where in the cell the nucleic acid remains is dependent on the type of expression construct employed.

In yet another embodiment, the expression construct may simply consist of naked recombinant DNA or plasmids. Transfer of the construct may be performed by any of the methods mentioned above which physically or chemically permeabilize the cell membrane. This is particularly applicable for transfer *in vitro* but it may be applied to *in vivo* use as well. Dubensky *et al.* (1984) successfully injected polyomavirus DNA in the form of calcium phosphate precipitates into liver and spleen of adult and newborn mice demonstrating active viral replication and acute infection. Benvenisty and Neshif (1986) also demonstrated that direct intraperitoneal injection of calcium phosphate-precipitated plasmids results in expression of the transfected genes. It is envisioned that DNA encoding a gene of interest may also be transferred in a similar manner *in vivo* and express the gene product.

In still another embodiment for transferring a naked DNA expression construct into cells may involve particle bombardment. This method depends on the ability to accelerate DNA-coated microprojectiles to a high velocity allowing them to pierce cell membranes and enter cells without killing them (Klein *et al.,* 1987). Several devices for accelerating small particles have been developed. One such device relies on a high voltage discharge to generate an electrical current, which in turn provides the motive force (Yang *et al.,* 1990). The microprojectiles used have consisted of biologically inert substances such as tungsten or gold beads.

Selected organs including the liver, skin, and muscle tissue of rats and mice have been bombarded *in vivo* (Yang *et al.,* 1990; Zelenin *et al.,* 1991). This may require surgical exposure of the tissue or cells, to eliminate any intervening tissue between the gun and the target organ, *i.e., ex vivo* treatment. Again, DNA encoding a particular gene may be delivered via this method and still be incorporated by the present disclosure.

In a further aspect, the expression construct may be entrapped in a liposome. Liposomes are vesicular structures characterized by a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers (Ghosh and Bachhawat, 1991). Also contemplated are lipofectamine-DNA complexes.

Liposome-mediated nucleic acid delivery and expression of foreign DNA *in vitro* has been very successful. Wong *et al.,* (1980) demonstrated the feasibility of liposome-mediated delivery and expression of foreign DNA in cultured chick embryo, HeLa and hepatoma cells. Nicolau *et al.,* (1987) accomplished successful liposome-mediated gene transfer in rats after intravenous injection. A reagent known as Lipofectamine 2000^{™} is widely used and commercially available.

In certain embodiments of the invention, the liposome may be complexed with a hemagglutinating virus (HVJ). This has been shown to facilitate fusion with the cell membrane and promote cell entry of liposome-encapsulated DNA (Kaneda *et al.,* 1989). In other embodiments, the liposome may be complexed or employed in conjunction with nuclear non-histone chromosomal proteins (HMG-1) (Kato *et al.,* 1991). In yet further embodiments, the liposome may be complexed or employed in conjunction with both HVJ and HMG-1. In that such expression constructs have been successfully employed in transfer and expression of nucleic acid *in vitro* and *in vivo,* then they are applicable for the present invention. Where a bacterial promoter is employed in the DNA construct, it also will be desirable to include within the liposome an appropriate bacterial polymerase.

Other expression constructs which can be employed to deliver a nucleic acid encoding a particular gene into cells are receptor-mediated delivery vehicles. These take advantage of the selective uptake of macromolecules by receptor-mediated endocytosis in almost all eukaryotic cells. Because of the cell type-specific distribution of various receptors, the delivery can be highly specific (Wu and Wu, 1993).

Receptor-mediated gene targeting vehicles generally consist of two components: a cell receptor-specific ligand and a DNA-binding agent. Several ligands have been used for receptor-mediated gene transfer. The most extensively characterized ligands are asialoorosomucoid (ASOR) (Wu and Wu, 1987) and transferrin (Wagner *et al.,* 1990). A synthetic neoglycoprotein, which recognizes the same receptor as ASOR, has been used as a gene delivery vehicle (Ferkol *et al.,* 1993; Perales *et al.,* 1994) and epidermal growth factor (EGF) has also been used to deliver genes to squamous carcinoma cells (Myers, EP 0273085).

### IV. Pharmaceutical Compositions and Delivery Methods

Where clinical applications are contemplated, pharmaceutical compositions will be prepared in a form appropriate for the intended application. Generally, this will entail preparing compositions that are essentially free of pyrogens, as well as other impurities that could be harmful to humans or animals.

One will generally desire to employ appropriate salts and buffers to render drugs, proteins or delivery vectors stable and allow for uptake by target cells. Aqueous compositions comprise an effective amount of the drug, vector or proteins, dissolved or dispersed in a pharmaceutically acceptable carrier or aqueous medium. The phrase "pharmaceutically or pharmacologically acceptable" refer to molecular entities and compositions that do not produce adverse, allergic, or other untoward reactions when administered to an animal or a human. As used herein, "pharmaceutically acceptable carrier" includes solvents, buffers, solutions, dispersion media, coatings, antibacterial and antifungal agents, isotonic and absorption delaying agents and the like acceptable for use in formulating pharmaceuticals, such as pharmaceuticals suitable for administration to humans. The use of such media and agents for pharmaceutically active substances is well known in the art. Except insofar as any conventional media or agent is incompatible with the active ingredients of the present invention, its use in therapeutic compositions is contemplated. Supplementary active ingredients also can be incorporated into the compositions, provided they do not inactivate the vectors or cells of the compositions.

The active compositions of the present invention may include classic pharmaceutical preparations. Administration of these compositions according to the present invention may be via any common route so long as the target tissue is available via that route, but generally including systemic administration. This includes oral, nasal, or buccal. Alternatively, administration may be by intradermal, subcutaneous, intramuscular, intraperitoneal or intravenous injection, or by direct injection into muscle tissue. Such compositions would normally be administered as pharmaceutically acceptable compositions, as described *supra.*

The active compounds may also be administered parenterally or intraperitoneally. By way of illustration, solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations generally contain a preservative to prevent the growth of microorganisms.

The pharmaceutical forms suitable for injectable use include, for example, sterile aqueous solutions or dispersions and sterile powders for the extemporaneous preparation of sterile injectable solutions or dispersions. Generally, these preparations are sterile and fluid to the extent that easy injectability exists. Preparations should be stable under the conditions of manufacture and storage and should be preserved against the contaminating action of microorganisms, such as bacteria and fungi. Appropriate solvents or dispersion media may contain, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial an antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions may be prepared by incorporating the active compounds in an appropriate amount into a solvent along with any other ingredients (for example as enumerated above) as desired, followed by filtered sterilization. Generally, dispersions are prepared by incorporating the various sterilized active ingredients into a sterile vehicle which contains the basic dispersion medium and the desired other ingredients, *e.g*., as enumerated above. In the case of sterile powders for the preparation of sterile injectable solutions, the preferred methods of preparation include vacuum-drying and freeze-drying techniques which yield a powder of the active ingredient(s) plus any additional desired ingredient from a previously sterile-filtered solution thereof.

The compositions of the present invention generally may be formulated in a neutral or salt form. Pharmaceutically-acceptable salts include, for example, acid addition salts (formed with the free amino groups of the protein) derived from inorganic acids (*e.g*., hydrochloric or phosphoric acids, or from organic acids (*e.g*., acetic, oxalic, tartaric, mandelic, and the like. Salts formed with the free carboxyl groups of the protein can also be derived from inorganic bases (*e.g.*, sodium, potassium, ammonium, calcium, or ferric hydroxides) or from organic bases (*e.g*., isopropylamine, trimethylamine, histidine, procaine and the like.

Upon formulation, solutions are preferably administered in a manner compatible with the dosage formulation and in such amount as is therapeutically effective. The formulations may easily be administered in a variety of dosage forms such as injectable solutions, drug release capsules and the like. For parenteral administration in an aqueous solution, for example, the solution generally is suitably buffered and the liquid diluent first rendered isotonic for example with sufficient saline or glucose. Such aqueous solutions may be used, for example, for intravenous, intramuscular, subcutaneous and intraperitoneal administration. Preferably, sterile aqueous media are employed as is known to those of skill in the art, particularly in light of the present disclosure. By way of illustration, a single dose may be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject. Moreover, for human administration, preparations should meet sterility, pyrogenicity, general safety and purity standards as required by FDA Office of Biologics standards.

### V. Examples

The following examples are included to demonstrate preferred embodiments of the disclosure. It should be appreciated by those of skill in the art that the techniques disclosed in the examples which follow represent techniques discovered by the inventor to function well in the practice of the disclosure, and thus can be considered to constitute preferred modes for its practice.

### EXAMPLE 1 - Materials and Methods

**Plasmids.** The hCas9 plasmid (Addgene plasmid 41815) containing the human codon optimized Cas9 gene and the gRNA Cloning Vector plasmid (Addgene plasmid 41824) containing the backbone of sgRNA were purchased from Addgene. Cloning of sgRNA was done according to the Church Lab CRISPR plasmid instructions (world-wide-web at addgene.org/crispr/church/).

***In vitro* transcription of Cas9 mRNA and sgRNA.** T3 promoter sequence was added to the hCas9 coding region by PCR. T3-hCas9 PCR product was gel purified and subcloned into pCRII-TOPO vector (Invitrogen) according to the manufacturer's instructions. Linearized T3-hCas9 plasmid was used as the template for *in vitro* transcription using the mMESSAGE mMACHINE T3 Transcription Kit (Life Technologies). T7 promoter sequence was added to the sgRNA template by PCR. The gel purified PCR products were used as template for in vitro transcription using the MEGAshortscript T7 Kit (Life Technologies). hCas9 RNA and sgRNA were purified by MEGAclear kit (Life Technologies) and eluted with nuclease-free water (Ambion). The concentration of RNA was measured by a NanoDrop instrument (Thermo Scientific).

**Single-stranded oligodeoxynucleotide (ssODN).** ssODN was used as HDR template and purchased from Integrated DNA Technologies as Ultramer DNA Oligonucleotides. ssODN was mixed with Cas9 mRNA and sgRNA directly without purification. The sequence of ssODN is listed in **Table S1.**

**CRISPR/Cas9-mediated genomic editing by one-cell embryo injection.** All animal procedures were approved by the Institutional Animal Care and Use Committee at the University of Texas Southwestern Medical Center. B6C3F1 (C57BL/6NCr female X C3H/HeN MTV male), C57BL/6NCr, and C57BL/10ScSn-*Dmd^{mdx}*/J were three mouse strains used as oocyte donors. Superovulated female B6C3F1 mice (6 weeks old) were mated to B6C3F1 stud males. Superovulated female C57BL/6NCr females (12-18 grams) were mated to C57BL/6NCr males and superovulated female homozygote C57BL/10ScSn-*Dmd^{mdx}*/J (12-18 grams) were mated to hemizygote C57BL/10ScSn-*Dmd^{mdx}*/J stud males. Zygotes were harvested and kept in M16 medium (Brinster's medium for ovum culture with 100U/ml penicillin and 50 mg/ml streptomycin) at 37 °C for 1 hour. Zygotes were transferred to M2 medium (M16 medium and 20 mM HEPES) and injected with hCas9 mRNA, sgRNA and ssODN. Cas9/sgRNA was injected into the pronucleus only (termed Nuc) or pronucleus and cytoplasm (termed Nuc+Cyt). Different doses of Cas9 mRNA, sgRNA and ssODNs were injected into zygotes by Nuc or Nuc+Cyt (as detailed in **Table S2).** Injected zygotes were cultured in M16 medium for 1 hour at 37 °C and then transferred into the oviducts of pseudopregnant ICR female mice.

**Isolation of genomic DNA.** Tail biopsies were added to 100 µl of 25 mM NaOH/0.2 mM EDTA solution and placed at 95 °C for 15 min and then cooled to room temperature. Following the addition of 100 µl of 40 mM Tris-HCl (pH 5.5), the tubes were centrifuged at 15,000 x g for 5 minutes. DNA samples were kept at 4 °C for several weeks or at -20°C for long-term storage. Genomic DNA was isolated from muscle using TRIzol (Life Technologies) according to the manufacturer's instructions.

**Amplifying the target genomic region by PCR.** PCR assays contained 2 µl of GoTaq (Promega), 20 µl of 5X Green GoTaq Reaction Buffer, 8 µl of 25 mM MgCl₂, 2 µl of 10 µM primer (DMD729F and DMD729R) **(Table S1),** 2 µl of 10 mM dNTP, 4 µl of genomic DNA, and ddH₂O to 100 µl. PCR conditions were: 94 °C for 2 min; 32X (94 °C for 15 sec, 59 °C for 30 sec, 72 °C for 1 min); 72 °C for 7 min; followed by 4 °C. PCR products were analyzed by 2% agarose gel electrophoresis and purified from the gel using the QIAquick PCR Purification Kit (Qiagen) for direct sequencing. These PCR products were subcloned into pCRII-TOPO vector (Invitrogen) according to the manufacturer's instructions. Individual clones were picked and the DNA was sequenced.

**RFLP analysis of PCR products.** Digestion reactions consisting of 20 µl of genomic PCR product, 3 µl of 10X NEB buffer CS, and 1 µl of TseI (New England BioLabs) were incubated for 1 hour at 65 °C and analyzed by 2% agarose gel electrophoresis. Digested PCR product from wild-type DNA is 581bp, while HDR-mediated genomic editing DNA from F₀ mice shows an additional product at approximately 437bp.

**T7E1 analysis of PCR products.** Mismatched duplex DNA was obtained by denaturation/renaturation of 25 µl of the genomic PCR samples using the following conditions:
95 °C for 10 min, 95 °C to 85 °C (-2.0 °C/s), 85 °C for 1 min, 85 °C to 75 °C (-0.3
°C/s), 75 °C for 1 min, 75 °C to 65 °C (-0.3 °C/s), 65 °C for 1 min, 65 °C to 55 °C (-0.3
°C/s), 55 °C for 1 min, 55 °C to 45 °C (-0.3 °C/s), 45 °C for 1 min, 45 °C to 35 °C (-0.3
°C/s), 35 °C for 1 min, 35 °C to 25 °C (-0.3 °C/s), 25 °C for 1 min, hold at 4 °C

Following denaturation/renaturation, the following was added to the samples: 3 µl of 10X NEB buffer 2, 0.3 µl of T7E1 (New England BioLabs), and ddH₂O to 30 µl. Digestion reactions were incubated for 1 hour at 37°C. Undigested PCR samples and T7E1 digested PCR products were analyzed by 2% agarose gel electrophoresis. Undigested PCR product is 729bp, while genomic DNA from F₀ mice with mismatched DNA showed two additional digestion products at approximately 440bp and 290bp.

**Grip strength test.** Muscle strength was assessed by a grip strength behavior task performed by the Neuro-Models Core Facility at UT Southwestern Medical Center. The mouse was removed from the cage, weighed and lifted by the tail causing the forelimbs to grasp the pull-bar assembly connected to the grip strength meter (Columbus Instruments). The mouse was drawn along a straight line leading away from the sensor until the grip is broken and the peak amount of force in grams was recorded. This was repeated 5 times.

**Serum creatine kinase (CK) measurement.** Blood was collected from the submandibular vein and serum CK level was measured by VITROS Chemistry Products CK Slides to quantitatively measure CK activity using VITROS 250 Chemistry System.

**Histological analysis of muscles.** Skeletal muscle from wild-type, *mdx,* and corrected *mdx-C* mice were individually dissected and cryoembedded in a 1:2 volume mixture of Gum Tragacanth powder (Sigma-Aldrich) to Tissue Freezing Medium (TFM) (Triangle Bioscience). Hearts were cryoembedded in TFM. All embeds were snap frozen in isopentane heat extractant supercooled to -155 °C. Resulting blocks were stored overnight at -80 °C prior to sectioning. Eight-micron transverse sections of skeletal muscle, and frontal sections of heart were prepared on a Leica CM3050 cryostat and air-dried prior to same day staining. H&E staining was performed according to established staining protocols and dystrophin immunohistochemistry was performed using MANDYS8 monoclonal antisera (Sigma-Aldrich) with modifications to manufacturer's instructions. In brief, cryostat sections were thawed and rehydrated/delipidated in 1% triton/phosphate-buffered-saline, pH 7.4 (PBS). Following delipidation, sections were washed free of Triton, incubated with mouse IgG blocking reagent (M.O.M. Kit, Vector Laboratories), washed, and sequentially equilibrated with MOM protein concentrate/PBS, and MANDYS8 diluted 1:1800 in MOM protein concentrate/PBS. Following overnight primary antibody incubation at 4 °C, sections were washed, incubated with MOM biotinylated anti-mouse IgG, washed, and detection completed with incubation of Vector fluorescein-avidin DCS. Nuclei were counterstained with propidium iodide (Molecular Probes) prior to cover slipping with Vectashield.

**Imaging and analysis.** Specimens were reviewed with a Zeiss Axioplan 2iE upright photomicroscope equipped with epifluiorescence illumination, CRI color wheel, and Zeiss Axiocam monochromatic CCD camera. OpenLab 4.0 acquisition and control software (Perkin-Elmer) was used to capture 4x, 10x and 20x objective magnification fields, and further used to apply indexed pseudocoloring and merge image overlays. Images were peak levels-adjusted with Adobe Photoshop CS2 and saved for image analysis. ImageJ 1.47 was used to apply stereo logic morphometric randomization grid overlays and the software's counting functions used to mark and score approximately 500 aggregate myofibers (from a minimum of three interval-sections) for dystrophin positive and negative immunostaining from each muscle group. H&E stained sections of soleus muscle for each genotype were further analyzed with ImageJ 1.47 for size and characteristic. In brief, sarcolemmal boundaries of 115+ stereo logically randomized myofibers were manually delineated, their cross sectional area calculated, and central-nuclear phenotype recorded.

**Western blot analysis.** Muscles were dissected and rapidly frozen in liquid nitrogen. Protein extraction and western blot analysis were performed as described (Nicholson *et al.,* 1989 and Kodippili *et al.,* 2014) with modification. Samples were homogenized with a homogenizer (POLYTRON System PT 1200 E) for 2 X 20 seconds in 400 uL sample buffer containing 10%SDS, 62.5 mM Tris, 1mM EDTA and protease inhibitor (Roche). Protein concentration was measured using the BCA Protein Assay Kit (Pierce). Fifty micrograms of protein from each muscle sample was loaded onto a gradient SDS-PAGE (Bio-Rad). The gel was run at 100V for 2.5 hours. Separated proteins were transferred to a PVDF membrane at 35V overnight in a cold room (4 °C). The PVDF membrane was stained for total protein using 2% Ponceau Red and then blocked for one hour with 5% w/v nonfat dry milk, 1× TBS, 0.1% Tween-20 (TBST) at 25 °C with gentle shaking. The blocked membrane was incubated with a mouse anti-dystrophin monoclonal antibody (MANDYS8, Sigma-Aldrich, 1: 1,000 dilution in 5% milk/TBST) overnight at 4 °C and then washed in TBST. The blot was then incubated with horseradish peroxidase conjugated goat anti-mouse IgG secondary antibody (Bio-Rad, 1:10,000 dilution) for one hour at 25 °C. After washing with TBST, the blot was exposed to Western Blotting Luminol Reagent (Santa Cruz Biotech) for 1 min to detect signal. Protein loading was monitored by anti-GAPDH antibody (Millipore, 1:10,000 dilution).

**Deep sequencing of off-target sites.** Off-target loci were amplified by PCR using primers listed in **Table S1** for (A) *mdx* (B) *mdx*+Cas9 (C) WT and (D) WT+Cas9. PCR products were purified by MinElute PCR purification kit (QIAGEN), adjusted to the same concentration (10 ng/µL), and equal volumes (5 µL) were combined for each group. Library preparation was performed according to the manufacturer's instructions (KAPA Library Preparation Kits with standard PCR library amplification module, Kapa Biosystems). Sequencing was performed on the Hiseq 2500 from Illumina and was run using Rapid Mode 150PE chemistry. Sequencing reads were mapped using BWA (bio-bwa.sourceforge.net/). Reads with mapping quality greater than 30 were retained for variant discovery. The mean read depth across all regions and all samples was 2570-fold. The variants were called using SAMtools (samtools.sourceforge.net/) plus custom scripts. In each region, insertion and deletion of 3 base pairs or longer were counted in a 50-bp window centered on the Cas9 potential cleavage sites.

**Laser microdissection of satellite cells.** Frozen sections from cryoembedments of gastrocnemius were mounted onto polyethylene membrane frame slides (Leica Microsystems PET-Foil 11505151) for same-day set-up of Pax-7 immunohistochemistry. Monoclonal Pax-7 antibody (Developmental Studies Hybridoma Bank) was used as described (Murphy *et al.,* 2011) with modifications to antigen retrieval for working with PET-foil membrane frame slides (Gjerdrum *et al.,* 2001). In brief, gastrocnemius cryosections were air-dried, fixed with 4% paraformaldehyde, Triton-X100 delipidated and incubated in antigen-retrieval buffer (sodium citrate buffer pH 6.0) at 65 °C for 20 hours. Following antigen retrieval, sections were quenched free of endogenous peroxidases with 0.6% hydrogen peroxide, and incubated with mouse IgG blocking reagent (M.O.M. Kit, Vector Laboratories), washed with PBS, incubated with MOM protein concentrate/PBS, and followed by overnight incubation with Pax-7 antibody (2 µg/ml) in MOM protein concentrate/PBS at 4°C. Sections were washed with PBS and incubated with MOM biotinylated anti-mouse IgG, streptavidin-peroxidase (Vector Laboratories), and color developed with diaminobenzidine chromagen (DAB, Dako). Nuclei were counterstained with nuclease-free Mayer's hematoxylin. Pax-7 positive satellite cells were microscopically identified and isolated by laser microdissection at 63x objective magnification using a Leica AS-LMD. Sixty to seventy Pax-7 positive satellite cells were isolated for each genotype and pooled into 10µl of capture buffer (DirectPCR Lysis Reagent, Viagen Biotech Inc.) and stored at -20 °C. The target genomic region was amplified by PCR using primers DMD232_f and DMD232_r **(Table S1),** as described above.

### EXAMPLE 2 - Results

The objective of this study was to correct the genetic defect in the *Dmd* gene *of mdx* mice by CRISPR/Cas9-mediated genome editing *in vivo.* The *mdx* mouse (C57BL/10ScSn-*Dmd^{mdx}l*J) contains a nonsense mutation in exon 23 of the *Dmd* gene (*14, 15*) **(****FIG. 1A****).** The inventors injected Cas9, sgRNA and HDR template into mouse zygotes to correct the diseasecausing gene mutation in the germ line (*16, 17*)*,* a strategy that has the potential to correct the mutation in all cells of the body, including myogenic progenitors. Safety and efficacy of CRISPR/Cas9-based gene therapy was also evaluated.

Initially, the inventors tested the feasibility and optimized the conditions of CRISPR/Cas9-mediated *Dmd* gene editing in wild-type mice (C57BL6/C3H and C57BL/6). The inventors designed a sgRNA to target *Dmd* exon 23 **(****FIG. 4A****)** and a single-stranded oligodeoxynucleotide (ssODN) as a template for HDR-mediated gene repair **(****FIG. 4B** and **Table S1).** The wild-type zygotes were co-injected with Cas9 mRNA, sgRNA-DMD and ssODN and then implanted into pseudopregnant female mice. Polymerase chain reaction products corresponding to *Dmd* exon 23 from progeny mice were sequenced **(****FIG. 4C-E).** Efficiency of CRISPR/Cas9-mediated *Dmd* gene editing is shown in **Table S2.**

The inventors next applied the optimized CRISPR/Cas9-mediated genomic editing method to *mdx* mice **(****FIG. 1B****).** The CRISPR/Cas9-mediated genomic editing system will correct the point mutation in *mdx* mice during embryonic development via HDR or NHEJ **(****FIGS. 1C-D and** **FIG. 5A****).** "Corrected" *mdx* progeny (termed *mdx-C)* were identified by RFLP analysis and the mismatch-specific T7 endonuclease I (T7E1) assay **(****FIG. 1E****, Table S2).** The inventors analyzed a total of eleven different *mdx-C* mice. PCR products of *Dmd* exon 23 from seven *mdx-C* mice with HDR-mediated gene correction (termed *mdx*-C1 to C7) and four *mdx-C* mice containing NHEJ-mediated in-frame deletions of the stop codon (termed *mdx*-N1 to N4) were sequenced. Sequencing results revealed that CRISPR/Cas9-mediated germline editing produced genetically mosaic *mdx-C* mice displaying from 2 to 100% correction of the *Dmd* gene **(****FIG. 1E** and **FIG. 5B-C).** A wide range of mosaicism occurs if CRISPR/Cas9-mediated repair occurs after the zygote stage, resulting in genomic editing in a subset of embryonic cells (Yen *et al.,* 2014). All mouse progeny developed to adults without signs of tumor growth or other abnormal phenotypes.

The inventors tested four different mouse groups for possible off-target effects of CRISPR/Cas9-mediated genome editing: (a) *mdx* mice without treatment (termed *mdx),* (b) CRISPR/Cas9-edited *mdx* mice (termed *mdx*+Cas9)*,* (c) wild-type control mice (C57BL6/C3H) without treatment (termed WT) and (d) CRISPR/Cas9-edited wild-type mice (termed WT+Cas9) **(****FIG. 6A****).** Sequences of the target site (*Dmd* exon 23) and a total of 32 potential off-target (OT) sites in the mouse genome were predicted by CRISPR design tool (crispr.mit.edu/) and are listed in **Table S3.** Ten of the 32 sites, termed OT-01 through OT-10 represent the genome-wide top-ten hits. Twenty-two of the 32 sites, termed OTE-01 through OTE-22 are located within exons.

Deep sequencing of PCR products corresponding to *Dmd* exon 23 revealed high ratios of HDR and NHEJ-mediated genetic modification in groups B and D but not in control groups A and C **(****FIG. 6A** and **Table S4).** There was no difference in the frequency of indel mutations in the 32 potential off-target regions among the different groups **(****FIGS. 6B-C,** **Table S5).** These results are also consistent with recent genome-wide studies showing that DNA cleavage by Cas9 is not promiscuous (Wu *et al.,* 2014; Kuscu *et al.,* 2014 and Duan *et al.,* 2014). Thus, off-target effects may be less of a concern *in vivo* than previously observed *in vitro (*Pattanayak *et al.,* 2013 and Fu *et al.,* 2013).

To analyze the effect of CRISPR/Cas9-mediated genomic editing on the development of muscular dystrophy, the inventors performed histological analyses of four different muscle types (quadriceps, soleus (hindlimb muscle), diaphragm (respiratory muscle) and heart muscle) from wild-type mice, *mdx* mice, and three chosen *mdx-C* mice with different percentages of *Dmd* gene correction at 7-9 weeks old age. *mdx* muscle showed histopathologic hallmarks of muscular dystrophy, including variation in fiber diameter, centralized nuclei, degenerating fibers, necrotic fibers, and mineralized fibers, as well as interstitial fibrosis **(****FIG. 2****,** **FIGS. 7A** and **8A****).** Immunohistochemistry showed no dystrophin expression in skeletal muscle or heart of *mdx* mice, while wild-type mice showed dystrophin expression in the subsarcolemmal region of the fibers and the heart **(****FIG. 2****).** Although *mdx* mice carry a stop mutation in the *Dmd* gene, the inventors observed 0.2-0.6% revertant fibers, consistent with a previous report (24). *mdx-*C mice with 41% of the *mdx* alleles corrected by HDR (termed HDR-41%) or with 83% correction by in-frame NHEJ (termed NHEJ-83%) showed complete absence of the dystrophic muscle phenotype and restoration of dystrophin expression in the subsarcolemmal region of all myofibers **(****FIG. 2****).** Strikingly, correction of only 17% of the mutant *Dmd* alleles (termed HDR-17%) was sufficient to allow dystrophin expression in a majority of myofibers at a level of intensity comparable to that of wild-type mice, and the muscle exhibited fewer histopathologic hallmarks of muscular dystrophy than *mdx* muscle **(****FIG. 7A****).** The substantially higher percentage (47-60%) of dystrophin-positive fibers associated with only 17% gene correction **(****FIG. 9A-B)** suggests a selective advantage of the corrected skeletal muscle cells. Western blot analysis showed restored dystrophin protein in skeletal muscle (quadriceps) and heart of *mdx-C* mice to levels consistent with percentages of dystrophin-positive fibers **(****FIG. 7B** and **FIG. 9B****).**

To compare the efficiency of rescue over time, the inventors chose *mdx-C* mice with comparable mosaicism of rescue of approximately 40%. As shown in **FIG. 10A****,** a 3-week *mdx-C* mouse with ~40% HDR-mediated gene correction (termed HDR-40%-3wks) showed occasional dystrophin-negative myofibers amongst a majority of dystrophin-positive fibers. In contrast, no dystrophin-negative fibers were seen in a mouse with comparable gene correction at 9 weeks of age, suggesting progressive rescue with age in skeletal muscle. In *mdx-C* mice with comparable mosaicism, the inventors did not observe a significant difference in dystrophin expression in the heart between 3 and 9 weeks of age **(****FIG. 10B****),** suggesting that age-dependent improvement may be restricted to skeletal muscle.

The widespread and progressive rescue of dystrophin expression in skeletal muscle might reflect the multi-nucleated structure of myofibers, such that a subset of nuclei with corrected *Dmd* genes can compensate for nuclei with *Dmd* mutations. Fusion of corrected satellite cells (the stem cell population of skeletal muscle) with dystrophic fibers might also progressively contribute to the regeneration of dystrophic muscle (Yin *et al.,* 2013). To investigate this possibility, the inventors identified satellite cells in muscle sections of *mdx-C* mice by Immunostaining with Pax-7, a specific-marker for satellite cells **(****FIG. 11A****).** Using laser microdissection, the inventors dissected Pax-7 positive satellite cells and isolated genomic DNA for PCR analysis **(****FIG. 3A** and **FIG. 11B****).** Sequencing results of PCR products corresponding to *Dmd* exon 23 from these isolated satellite cells showed the corrected *Dmd* gene **(****FIG. 3B****).** These results indicate that CRISPR/Cas9 genomic editing corrected the mutation in satellite cells allowing these muscle stem cells to rescue the dystrophic muscle **(****FIG. 3C** and **FIG. 11C****).**

Serum creatine kinase (CK), a diagnostic marker for muscular dystrophy that reflects muscle leakage, was measured in wild-type, *mdx* and *mdx-C* mice. Consistent with the histological results, serum CK levels of the *mdx-C* mice were substantially decreased compared to *mdx* mice and were inversely proportional to the percentage of genomic correction **(Table 1).** Wild-type, *mdx,* and *mdx-C* mice were also subjected to grip strength testing to measure muscle performance, and the *mdx-C* mice showed enhanced muscle performance compared to *mdx* mice **(Table 1).**

**Permanent exon skipping via CRISPR/Cas9-mediated genome editing (Myo-editing).** A challenge to genomic editing in postnatal tissues is that HDR does not occur in postmitotic cells, such as myofibers and cardiomyocytes. However, NHEJ does occur and can be used to destroy mutations without the need for precision of mutagenesis. Exon skipping is a strategy in which sections of genes are "skipped", allowing the creation of partially functional dystrophin (Aartsma-Rus, 2012). However, traditional antisense oligonucleotide (AON)-mediated transient exon skipping suffers from inefficiency of oligonucleotide tissue uptake, requirement for lifelong delivery of oligonucleotides and incomplete exon skipping. To circumvent this challenge, the inventors used CRISPR/Cas9 system to destroy exon splice sites preceding DMD mutations or to delete mutant or out-of-frame exons, thereby allowing splicing between surrounding exons to recreate an in-frame dystrophin protein that lack the mutations. By permanently correcting the genetic lesion responsible for DMD, genomic editing requires only one-time delivery of the editing components to heart or skeletal muscle. Moreover, the progressive improvement of muscle function over time, allows for continued restoration of muscle function long after the genomic editing has occurred.

A schematic diagram of the dystrophin protein is shown in FIG. 12. This large protein of 3685 amino acids contains several well characterized domains, including an actin-binding domain at the N-terminus, a central rod domain with a series of spectrin-like repeats and actin-binding repeats, and WW and Cysteine-rich domains at the C-terminus that mediate binding to dystroglycan, dystrobrevin and syntrophin. Importantly, many regions of the protein are dispensable for function, which allows therapeutic efficacy of exon skipping strategies. The C-terminus of dystrophin is essential for function, thus, exon skipping strategies that restore the C-terminus can convert DMD to Becker Muscular Dystrophy (BMD) a relatively mild form of the disease that is does not cause premature death or severe loss of mobility, allowing for dramatic functional improvement.

Given the thousands of individual DMD mutations that have been identified in humans, an obvious question is how such a large number of mutations might be readily corrected by CRISPR/Cas9-mediated genome editing. To circumvent this challenge, the inventors propose to use CRISPR/Cas9 to destroy splice acceptor/donor sites preceding DMD mutations or to delete mutant exons, thereby allowing splicing between surrounding exons to recreate the in-frame dystrophin protein lacking the mutations. A schematic diagram of this approach is shown in **FIG. 13****,** in which NHEJ can either create internal genomic deletions to correct the open reading frame or can disrupt splice acceptor sites. The example shows how this approach can be applied to bypass the exon 23 mutation responsible for the dystrophic phenotype of *mdx* mice, but in principle, can be applied to numerous types of mutations within the gene. It has been estimated that as many as 80% of DMD patients could potentially benefit from exon skipping strategies to partially restore dystrophin expression.

**CRISPR/Cas9-mediated permanent Dmd exon skipping on mdx mice germline.** To begin to test Myo-editing of the exon 23 mutation in *mdx* mice, the inventors first generated a pool of sgRNAs (sgRNA-L and R) that target the 5' end and 3' end of exon 23 **(****FIG. 14A****).** NHEJ-mediated indel can abolish the conserved RNA splice site or delete exon 23.These guide RNAs were cloned in plasmid spCas9-2A-GFP (Addgene #48138). Initially, the inventors evaluated the efficiency of guide RNAs in the mouse 10T½ cells. Myo-editing efficiency was detected by the T7E1 assay as describe before. sgRNA-R3 target 3' end of exon 23 showed a high activity. They then co-injected Cas9 and guide RNA mdx and R3 into mdx zygotes without HDR template **(****FIG. 14B****).** Strikingly, 7 out of 9 progeny mice contained indel at the 3' donor site or deleted the whole exon 23 **(****FIG. 14C****).** In previous work, only ~8% of *mdx* pups contain an HDR-mediated correction. Using a new method significantly increased the efficiency by ten-fold. PCR products from the target sites of exon 23 were cloned and sequenced. The results showed that Myo-editing can efficiently generate NHEJ-mediated indel mutation to rescue the open reading frame of the dystrophin gene.

A subset of NHEJ gene-edited *mdx* mice harbored a genetic deletion that abolished the splice site in exon 23. The inventors analyzed these mice for possible exon skipping by sequencing the generated RT-PCR products using primers in exon 22 and 24. Sequencing results showed that exon 22 spliced directly to exon 24, excluding exon 23 **(****FIG. 15****).** The result of skipping exon 23 maintains the open reading frame of dystrophin and restores protein expression **(****FIG. 16****).** Sequencing of RT-PCR products of exon 23 "skipper" mice (also called mdx-ΔE23 mice) confirmed the 213 nucleotide deletion corresponding to the absence of exon 23 sequence and a 71 amino acid in-frame deletion of dystrophin. These findings establish important proof of concept for the proposed studies to use NHEJ-mediated genomic editing to bypass numerous different mutations in the *Dmd* gene.

***In vivo* rescue of muscular dystrophy in mice by AAV-mediated Myo-editing.** AAV is one of the most promising and appropriate vehicles for safe delivery of the Cas9 protein and guide RNAs for precise Myo-editing to human skeletal muscle and heart. It is important to emphasize that the problem of delivering CRISPR/Cas9 precision Myo-editing therapy by AAV goes hand-in-hand with optimizing the efficiency of both viral delivery and the process of genome engineering. The inventors focused on developing the highest titer AAV9 preparations for delivering the CRISPR/Cas9 genome editing machinery to muscle cells *in vivo.* This is an area of intensive international research (Senis *et al.,* 2014; Schmidt & Grimm, 2015).

The inventors used the verified guide RNA-mdx/R3 to generate AAV9-guide RNAs **(****FIG. 17A****).** They obtained a unique AAV9 CRISPR/Cas9 vector (miniCMV-Cas9-shortPolyA plasmid) **(****FIG. 17B****).** This viral construct employs the shortest possible "mini"-CMV promoter/enhancer sequence to drive expression of the Cas9 protein. The inventors evaluated these recombinant viruses in *mdx* mouse models *in vivo.* They are systematically testing different modes of AAV9 delivery as well as variations in timing of expression to identify the optimal method to achieve maximal *Dmd* editing. They administered four types of injection routes at various ages: (i) intra-peritoneal (IP) at P1, (ii) intra-muscular (IM) at P10, (iii) retro-orbital (RO) at P14 and (iv) intra-cardiac (IC) at P28. Heart and skeletal muscle were harvested at time points shown in **FIG. 17C****.**

In a proof of concept experiment, the inventors injected recombinant AAVs by IM injection of P10 mice or IC injection of P28. Muscle tissues were analyzed by immunostaining for dystrophin protein expression 3-weeks post-injection, as shown in **FIG. 18A****.** Native green fluorescent protein (GFP) indicates the AAV-mediated gene expression in myofibers. Skeletal muscle from the injected mouse has a unique pattern of clusters of dystrophin-positive fibers adjacent to clusters of dystrophin-negative fibers. A transduction frequency or rescue of 7.7%±3.1% of myofibers is estimated in the tibialis anterior muscle of treated *mdx* mouse, 3-weeks post-IM-AAV. **(****FIG. 18B****)** Native GFP and dystrophin immunostaining from serial sections of mdx mouse heart showing dystrophin protein expression in cardiomyocyte (4-weeks post-IC-AAV). Transduction frequency (rescue) increases to an estimated 25.5%±2.9% of myofibers by 6-weeks post-IM-AAV **(****FIG. 19****)** Progressive improvement with age is seen from 3-weeks to 6-weeks post-IM-AAV, which is consistent with results from germline editing **(****FIG. 10A****).**

Muscle tissues from mice injected with recombinant AAVs by retro-orbital injection (RO-AAV) at P14 were examined by immunohistochemistry at 4 and 8-weeks post injection **(****FIG. 20****).** The percentage of dystrophin positive fibers or myocytes were calculated as a function total estimated fibers. At 4-weeks post-RO injection in *mdx* mice, 1.9%±0.51% of myofibers are dystrophin positive, while 1.3%±0.05% of cardiac myocytes are dystrophin positive. Progressive improvement with age is observed from 4-weeks to 8-weeks post-RO-AAV. Rescue increases to an estimated 6.1±3.2% of myofibers in tibialis anterior muscle, and 5.0%±2.1% of cardiomyocytes by 8-weeks post-RO-AAV

At 4-weeks post-IP injection of P1 *mdx* pup, skeletal muscle and heart were examined by immunohistochemistry **(****FIG. 21****).** A transduction frequency (rescue) of 3.0% of myofibers is estimated in tibialis anterior muscle and 2.4% of cardiomyocytes in treated *mdx* mice. The inventors expect higher percent of muscle correcting progressive with time. In fact, in the inventors' previous germline study *in vivo* editing improved progressively with time (Long *et al.,* 2014). It has been reported that even low level expression of dystrophin (4-15%) in the heart can partially ameliorate cardiomyopathy in *mdx* mice (van Putten *et al.,* 2014).

Morphometric analysis of dystrophin-positive and total myofibers and cardiomyocytes were carried out on replicates of whole step-sections of tibialis anterior muscles and hearts scanned at 20x objective magnification. Scanned images, ranging in size from 7889 x 7570 pixels to 27518 x 18466 pixels, were parsed using Nikon Imaging Solution Elements v4.20.00 Software's Annotations and Measurements functions (NIS/AM). Enumeration of dystrophin positive myofibers and cardiomyocytes were individually counted and recorded using NIS/AM, while enumeration of total myofibers and cardiomyocytes were estimated from cell-counts per field area made from the mean of eight 20x objective images and extrapolated to the whole scanned section area.

The results indicate that AAV-mediated Myo-editing can efficiently rescue the reading frame of dystrophin in *mdx* mice *in vivo.* Different AAV delivery methods have different impact on tissues. IM has the highest rescue percentage myofibers in the injected skeletal muscle (TA), while RO shows the best performance in heart.

**Rescue of DMD cardiomyocyte function by Myo-editing.** A long-term goal is to adapt Myo-editing to postnatal cardiac and skeletal muscle cells and to leverage this approach to correct *DMD* mutations in humans. The inventors have now advanced Myo-editing from mice to cells from human DMD patients by engineering the skipping of mutant exons in the genome of DMD patient-derived iPSCs. *DMD* mutations in patients are clustered in specific areas of the gene ("hot spot" mutations) **(****FIG. 22****).** They have optimized Myo-editing of "hot spot" *DMD* mutations using pools of sgRNAs to target the top 12 hot spot mutant exons, potentially applicable to 80% of DMD patients. They selected three to six PAM sequences to target the 5' or 3' ends of each exon **(Table 2).** Myo-editing-mediated indels abolish the conserved RNA splice acceptor/donor sites and rescue the out-of-frame exons. Based on the known *DMD* mutations, the inventors are establishing an online resource (Duchenne Skipper Database) for selecting the optimal target *DMD* sequences for Myo-editing, which will rescue dystrophin function in the majority of DMD patients.

For instance, the inventors designed three guide RNAs to target 5' of exon 51 **(****FIG. 23A****).** These guide RNAs were cloned in plasmid spCas9-2A-GFP (Addgene #48138). Initially, the inventors evaluated the efficiency of guide RNAs in the human 293T cells and normal human iPSCs by transfection and nucleofection. The transfected 293T cell and nucleofected iPSCs were sorted by GFP reporter. Myo-editing efficiency were detected by the T7E1 assay as describe before **(****FIG. 23B****).** In GFP+ sorted 293T cells, guide RNA #3 shown a high activity, while guide RNA #1 and 2 had no detectable activity. The results highlight the importance of the optimization of target sequences. The inventors then applied guide RNA #3 in human iPSCs and observed the same results. PCR products from target sites of Exon 51 were cloned and sequenced. Results show that Myo-editing can efficiently abolish the splicing acceptor site (Δag) or generate indel to rescue reading frame.

Next, the inventors performed Myo-editing on an iPSC line (aka Riken HPS0164) from a DMD patient with a deletion (exons 48-50), which creates a frame-shift mutation, as visualized in **FIG. 24A****.** Destruction of the splice acceptor in exon 51 will, in principle, allow for splicing of exon 47 to exon 52, thereby reconstituting the open reading frame. Using guide RNA (Exon 51-sgRNA #3, **(****FIG. 23B****),** the inventors successfully destroyed the splice acceptor in Exon 51 in iPSCs from this patient, restoring the open reading frame by NHEJ mutation **(****FIG. 24B****).** Taking the pool of Myo-edited DMD-iPSCs, the inventors differentiated them into cardiomyocytes (iCM) using standardized conditions and confirmed rescue of dystrophin protein expression by immunocytochemistry in a subset of these cells **(****FIG. 25****).**

To further extend the Myo-editing concept, the Myo-editing Core at UTSW generated additional DMD iPSC cell lines, which were used to test the permanent exon skipping strategy **(****FIG. 26****).** The inventors made DMD-iPSCs from patients' blood samples instead of skin cells since blood cells are more accessible with minimal risk to the patient. PBMCs (peripheral blood mononuclear cells) obtained from whole blood was cultured and then reprogrammed into iPSCs using recombinant Sendai viral vectors expressing reprogramming factors (Cytotune 2.0, Life Technologies). iPSC colonies are validated by immunocytochemistry, mycoplasma testing, and teratoma formation.

A 22-year old male patient has a spontaneous mutation in intron 47 (c.6913-4037T>G) which generates a novel RNA splicing acceptor site (YnNYAG) and results in a pseudoexon of exon 47A **(****FIG. 27****).** This pseudoexon encodes a premature stop signal. The inventors designed two guide RNAs which precisely target the mutation sites **(****FIG. 28****).** Myo-editing can abolish the novel splice acceptor site and permanently skip the pseudoexon. It is worth to point out that guide RNA #2 will only target the mutation allele, because the wide type *DMD* does not contain the PAM sequence (AGG), which is important for the potential Myo-editing in female DMD carriers who have both mutation and wild-type alleles. In addition, the Myo-editing-mediated indel mutation is in the middle of the intron region which will not affect normal function of the encoded dystrophin. Theoretically, by skipping the pseudogene the resulting dystrophin gene can generate a full length mRNA and dystrophin protein. Specific guide RNAs were cloned and nucleofected into iPSCs as described. The inventors tested the efficacy of exon skipping by RT-PCR in these DMD-iCMs **(****FIG. 29****)**. Muscle cells derived from corrected iPSCs were assayed for dystrophin protein expression by immunocytochemistry which showed dystrophin expression in myo-edited DMD cardiomyocytes **(****FIG. 30****).**

In conclusion, precision Myo-editing allows us not only to target on DMD "hot spots" (*e.g.,* Riken HPS0164 DMD-iPSCs), but also to easily correct any other rare mutations (*e.g.,* DC0160 DMD). Myo-editing represents a new and powerful approach to permanently eliminate the genetic cause of DMD. Given the potential for durable and progressive therapeutic response in post-mitotic adult tissue, the inventors feel this is an opportune time to apply Myo-editing to permanently correct the muscle abnormalities associated with DMD.

### EXAMPLE 3 - Discussion

These results show that CRISPR/Cas9-mediated genomic editing is capable of correcting the primary genetic lesion responsible for muscular dystrophy (DMD) and preventing development of characteristic features of this disease in *mdx* mice. Because genome editing in the germline produced genetically corrected animals with a wide range of mosaicism (2 to 100%), the inventors were able to compare the percent genomic correction with the extent of rescue of normal muscle structure and function. The inventors observed that only a subset of corrected cells *in vivo* is sufficient for complete phenotypic rescue. As schematized in **FIG. 3C****,** histological analysis of partially corrected *mdx* mice revealed three types of myofibers: 1) Normal dystrophin-positive myofibers; 2) dystrophic dystrophin-negative myofibers; and 3) mosaic dystrophin-positive myofibers containing centralized nuclei, indicative of muscle regeneration. The inventors propose that the latter type of myofiber arises from the recruitment of corrected satellite cells into damaged myofibers, forming mosaic myofibers with centralized nuclei. Efforts to expand satellite cells *ex vivo* as a source of cells for *in vivo* engraftment have been hindered by the loss of proliferative potential and regenerative capacity of these cells in culture (Montarras *et al.,* 2005). Thus, direct editing of satellite cells *in vivo* by CRISPR/Cas9 system represents a potentially promising alternative approach to promote muscle repair in DMD.

Genomic editing could, in principle, be envisioned within postnatal cells *in vivo* if certain technical challenges can be overcome. For example, there is a need for appropriate somatic cell delivery systems capable of directing the components of the CRISPR/Cas9 system to dystrophic muscle or satellite cells *in vivo.* In this regard, the non-pathogenic adeno-associated virus (AAV) delivery system has proven to be safe and effective and has already been advanced in clinical trials for gene therapy (Nathwani *et al.,* 2011 and Peng *et al.,* 2005). Moreover, the AAV9 serotype has been shown to provide robust expression in skeletal muscle, heart and brain, the major tissues affected in DMD patients. Other non-viral gene delivery methods, including injection of naked plasmid DNA (Peng *et al.,* 2005), chemically modified mRNA (Kormann *et al.,* 2011 and L. Zangi *et al.,* 2013), and nanoparticles containing nucleic acid (Harris *et al.,* 2010) also warrant consideration. Another challenge with respect to the feasibility of clinical application of the CRISPR/Cas9 system is the increase in body size between rodents and humans, requiring substantial scale-up. More efficient genome editing in post-natal somatic tissues is also needed for the advancement of the CRISPR/Cas9 system into clinical use. Although CRISPR/Cas9 can effectively generate NHEJ-mediated indel mutations in somatic cells, HDR-mediated correction is relatively ineffective in post-mitotic cells, such as myofibers and cardiomyocytes, because these cells lack the proteins essential for homologous recombination (Hsu *et al.,* 2014). Co-expression of components of the HDR pathway with the CRISPR/Cas9 system might enhance HDR-mediated gene repair. Finally, safety issues of the CRISPR/Cas9 system, especially for long-term use, need to be evaluated in preclinical studies in large animal models of disease. Despite the challenges listed above, with rapid technological advances of gene delivery systems and improvements to the CRISPR/Cas9 editing system (Hsu *et al.,* 2014), the approach the inventors describe could ultimately offer therapeutic benefit to DMD and other human genetic diseases in the foreseeable future.

In sum, the approach here uses the CRISPR/Cas9 system to delete the exon splice acceptor upsteam of the exon containing the mutation of the dystrophin gene. This approach makes only a minor change (a few nucleotides) on the genome which will avoid disrupting other functional elements in the intron (enhancer, alternative promoter and microRNA, *etc.*)*.* This mechanism for gene correction is different than that reported by others. The major spliceosome splices introns containing GU at the 5' splice site and AG at the 3' splice site. Cas9, guided by single-guide RNA (sgRNA), binds to a targeted genomic locus next to the protospacer adjacent motif (PAM) and generates a double-strand break (DSB). The PAM sequence for the classical Cas9 (from *Streptococcus pyogenes*) is N**AG** or NGG, which means that in principle one can target any of exon with mutations and rescue the gene expression by exon-skipping. In addition, the approach here is to direct genomic editing of satellite cells or myofibers *in vivo* via delivery of CRISPR/Cas9 system using AAV9 (and other delivery methods). Direct genomic editing in humans has not been reported to date. This direct approach represents a potentially promising alternative method to promote muscle repair in DMD.

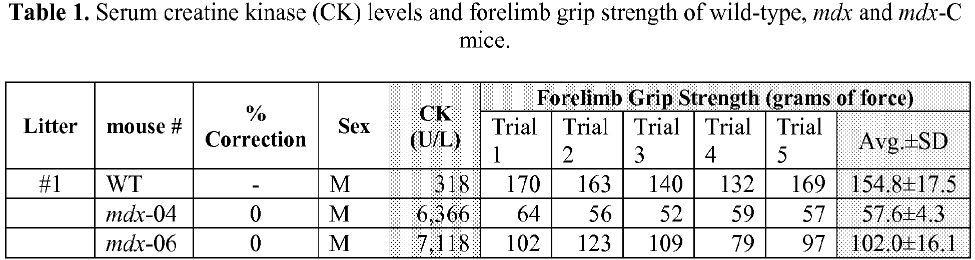

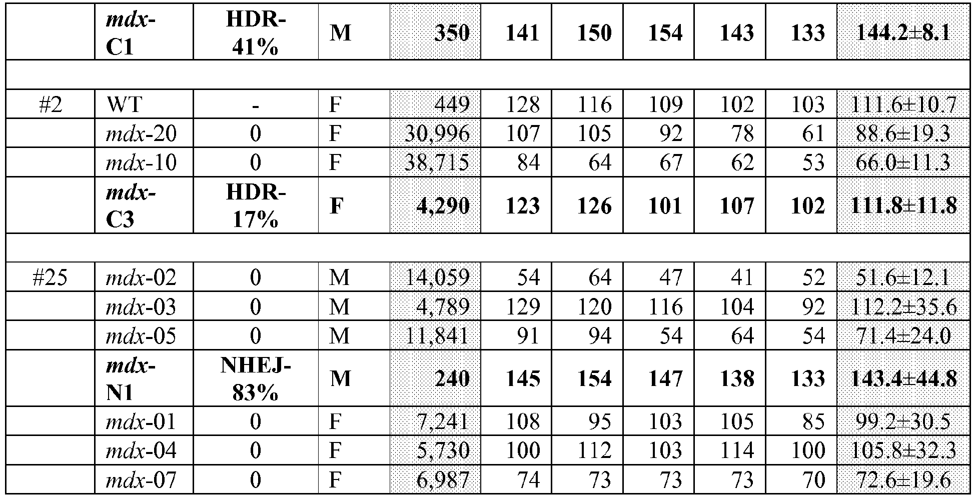

**Table 2. Sequence of guide RNA for 12 exons of DMD gene.**

| **Exon** | **gRNA at 5'acceptor site** | | **SEQ ID** | **gRNA at 3' donor site** | | **SEQ ID** |
|---|---|---|---|---|---|---|
| **51** | #1: | TGCAAAAACCCAAAATATTT | 33 | | | |
| | #2: | AAAATATTTTAGCTCCTACT | 34 | | | |
| | **#3:** | **CAGAGTAACAGTCTGAGTAG** | 35 | | | |
| **52** | **#1:** | **TAAGGGATATTTGTTCTTAC** | 36 | | | |
| | #2: | CTAAGGGATATTTGTTCTTA | 37 | | | |
| | #3: | TGTTCTTACAGGCAACAATG | 38 | | | |
| **50** | **#1:** | **TGTATGCTTTTCTGTTAAAG** | 39 | | | |
| | #2: | ATGTGTATGCTTTTCTGTTA | 40 | | | |
| | #3: | GTGTATGCTTTTCTGTTAAA | 41 | | | |
| **45** | #1: | TTGCCTTTTTGGTATCTTAC | 42 | | | |
| | #2: | TTTGCCTTTTTGGTATCTTA | 43 | | | |
| | **#3:** | **CGCTGCCCAATGCCATCCTG** | 44 | | | |
| **53** | #1: | ATTTATTTTTCCTTTTATTC | 45 | **#4:** | **AAAGAAAATCACAGAAACCA** | 69 |
| | #2: | TTTCCTTTTATTCTAGTTGA | 46 | #5: | AAAATCACAGAAACCAAGGT | 70 |
| | #3: | TGATTCTGAATTCTTTCAAC | 47 | **#6:** | **GGTATCTTTGATACTAACCT** | 71 |
| **44** | **#1:** | **ATCCATATGCTTTTACCTGC** | 48 | | | |
| | #2: | GATCCATATGCTTTTACCTG | 49 | | | |
| | #3: | CAGATCTGTCAAATCGCCTG | 50 | | | |
| **46** | #1: | TTATTCTTCTTTCTCCAGGC | 51 | | | |
| | **#2:** | **AATTTTATTCTTCTTTCTCC** | 52 | | | |
| | #3: | CAATTTTATTCTTCTTTCTC | 53 | | | |
| **43** | #1: | GTTTTAAAATTTTTATATTA | 54 | **#4:** | **TATGTGTTACCTACCCTTGT** | 72 |
| | #2: | TTTTATATTACAGAATATAA | 55 | #5: | AAATGTACAAGGACCGACAA | 73 |
| | #3: | ATATTACAGAATATAAAAGA | 56 | **#6:** | **GTACAAGGACCGACAAGGGT** | 74 |
| 7 | #1: | TGTGTATGTGTATGTGTTTT | 57 | | | |
| | #2: | TATGTGTATGTGTTTTAGGC | 58 | | | |
| | **#3:** | **CTATTCCAGTCAAATAGGTC** | 59 | | | |
| 8 | #1: | GTGTAGTGTTAATGTGCTTA | 60 | #4: | TGCACTATTCTCAACAGGTA | 75 |
| | #2: | GGACTTCTTATCTGGATAGG | 61 | **#5:** | **TCAAATGCACTATTCTCAAC** | 76 |
| | #3: | TAGGTGGTATCAACATCTGT | 62 | #6: | CTTTACACACTTTACCTGTT | 77 |
| 6 | #1: | TGAAAATTTATTTCCACATG | 63 | **#4:** | **ATGCTCTCATCCATAGTCAT** | 78 |
| | #2: | GAAAATTTATTTCCACATGT | 64 | #5: | TCTCATCCATAGTCATAGGT | 79 |
| | #3: | TTACATTTTTGACCTACATG | 65 | #6: | CATCCATAGTCATAGGTAAG | 80 |
| **55** | #1: | TGAACATTTGGTCCTTTGCA | 66 | | | |
| | #2: | TCTGAACATTTGGTCCTTTG | 67 | | | |
| | **#3:** | **TCTCGCTCACTCACCCTGCA** | 68 | | | |

| | | | | | | |
|---|---|---|---|---|---|---|
| Note: **BOLD** indicates the best guide for Myo-editing | | | | | | |

**Table S1. Oligonucleotides and primer sequences.**

| **ssODN used for HDR-medicated editing via embryo micro-injection** | | **SEQ ID NO:** |
|---|---|---|
| **Dmd_donor_TseI-s180** | | 81 |
| | | |

| **Primers for genotyping** | | **SEQ ID NO:** |
|---|---|---|
| Dmd 729F | gagaaacttctgtgatgtgaggacata | 82 |
| Dmd 729R | caatatctttgaaggactctgggtaaa | 83 |
| | | |

| **Primers for OT analysis** | | **SEQ ID NO:** |
|---|---|---|
| DMD232_f | cttctatttaattttgaggctctgc | 84 |
| DMD232_r | cctgaaattttcgaagtttattcat | 85 |
| DS-OT-01_f | tatgccacttcttcaaagagatgat | 86 |
| DS-OT-01_r | aacaagcaaacaattcaaaggatag | 87 |
| DS-OT-02_f | aagaagatatggcattgctggta | 88 |
| DS-OT-02_r | tctggaaacaaaaaggcaatg | 89 |
| DS-OT-03_f | taagagttctgacatgatttccaca | 90 |
| DS-OT-03_r | tggaacactactctctacactgtgc | 91 |
| DS-OT-04_f | ctatgagtttaccaccctaatgtgc | 92 |
| DS-OT-04_r | cttatgcttgttcaggcaaatacc | 93 |
| DS-OT-05_f | ttttgagttgtgttcattttctgag | 94 |
| DS-OT-05_r | taggagtacagctgcttcttcagac | 95 |
| DS-OT-06_f | gaaaaacaaaattactgaggcatgt | 96 |
| DS-OT-06_r | cctccaagttcttatcttgtttgaa | 97 |
| DS-OT-07_f | agtgattttctgatgacccaaatta | 98 |
| DS-OT-07_r | tgtttttaatggctaggtgctaatc | 99 |
| DS-OT-08_f | tttcttggagctgta gtgtgtactg | 100 |
| DS-OT-08_r | ggaatagagtgagcattgttctgat | 101 |
| DS-OT-09_f | tgtcacagttgcaattcttagtgtt | 102 |
| DS-OT-09_r | cttagaaaaacaaggttcctgacaa | 103 |
| DS-OT-10_f | caataaggacaagtgaaggctaaaa | 104 |
| DS-OT-10_r | aggtctccacacatattcactcttc | 105 |
| DS-OTE-01_f | agatctgggagcttctatcaactg | 106 |
| DS-OTE-01_r | gggtagaagtgaatcaataagtgga | 107 |
| DS-OTE-02 _f | gaacacttctttgcttctcatcact | 108 |
| DS-OTE-02_r | gctgagactactgtagccctttaga | 109 |
| DS-OTE-03_f | tagtttttcacattcagtccagctt | 110 |
| DS-OTE-03_r | gctttcaaaactacaccaaacctac | 111 |
| DS-OTE-04_f | ctttaaaatacaagcctccagttcc | 112 |
| DS-OTE-04_r | tatttgtttctcaaatttccagacc | 113 |
| DS-OTE-05_f | attttctagaggtggtctcacacac | 114 |
| DS-OTE-05_r | gaaaagtggatagacagtttcagga | 115 |
| DS-OTE-06_f | aacctaaaagaaaggacaaggagaa | 116 |
| DS-OTE-06_r | acatgactcggtaataaaccttgag | 117 |
| DS-OTE-07_f | ttgtaaaagttccaactcccagtag | 118 |
| DS-OTE-07_r | tttaaaatctatttccccagagagg | 119 |
| DS-OTE-08_f | tgtccatttttaacctgtgttctg | 120 |
| DS-OTE-08_r | ccctaactcagtttctcttgttctg | 121 |
| DS-OTE-09_f | atctgtgttttcaatgtggaatctt | 122 |
| DS-OTE-09_r | agaaagcgaataggatttcttgttt | 123 |
| DS-OTE-10_f | tcgaatcttctacaatatgcaatca | 124 |
| DS-OTE-10_r | gtgggaaatgtttcaagtatcacat | 125 |
| DS-OTE-11_f | gcaaaatacaacttctaagcaaacc | 126 |
| DS-OTE-11_r | ccagaccagaggtagagtgtttcta | 127 |
| DS-OTE-12 _f | caggagtcagcctcttactttacaa | 128 |
| DS-OTE-12 r | gctagatgacaaagccacttaactc | 129 |
| DS-OTE-13 _f | gctacagaaaagaggctaggaaagt | 130 |
| DS-OTE-13 r | gctttgaagatgccctagaaatact | 131 |
| DS-OTE-14 _f | taatacataaggggacatcacgagt | 132 |
| DS-OTE-14_r | gatctttgtagtggtttttctcctg | 133 |
| DS-OTE-15_f | ttaagcggaaagataagctgaagta | 134 |
| DS-OTE-15 _r | ggaccaatgttactggaacacatac | 135 |
| DS-OTE-16_f | cttctacattcacctccctgtgtt | 136 |
| DS-OTE-16 _r | cccagcatctaagaaaggagtaata | 137 |
| DS-OTE-17_f | aaatttttagtcaaaagtgcttgga | 138 |
| DS-OTE-17_r | caataaacctttcagacttcattgg | 139 |
| DS-OTE-18_f | tatgatttccagggtaagtccacta | 140 |
| DS-OTE-18_r | gcacttttgctaacatctaaattcc | 141 |
| DS-OTE-19_f | aaagtatatctgagaatgccactgc | 142 |
| DS-OTE-19_r | gtagctgtaggaatgtctgtcctgt | 143 |
| DS-OTE-2_f | tgtaataaaatgagaatttgcacca | 144 |
| DS-OTE-20_r | aatgaagccaaggtacatacaaaga | 145 |
| DS-OTE-21_f | catgaagatacagaaacatcccagt | 146 |
| DS-OTE-21_r | ggagtggcaccctccttac | 147 |
| DS-OTE-22_f | ataccccaagccatacttgtatcat | 148 |
| DS-OTE-22_r | cacttatccatctaggaaagcagag | 149 |

**Table S2. Efficiency of CRISPR/Cas9-mediated genomic editing by cytoplasm and pronuclear injection.**

| **Strain** | **Dose of Cas9/sgRNA /ssODN (ng/µl)** | **Injection Methods** | **No.of Transferred Zygotes** | **No. of Pups /Zygotes (%)** | **No. of Mutant Founders/ Pups (%)** | **No. of HDR /Pups (%)** |
|---|---|---|---|---|---|---|
| C57BL6/ C3H | 5/2.5/5 | Nuc | 60 | 29 (48%) | 9 (31%) | 1 (3.4%) |
| | | Nuc+Cyt | 60 | 27 (45%) | 5 (19%) | 1 (3.7%) |
| | 10/5/5 | Nuc | 30 | 13 (43%) | 1(7.7%) | 1 (7.7%) |
| | | Nuc+Cyt | 30 | 17 (57%) | 6(35%) | 3 (18%) |
| | | | | | | |
| C57BL/6 | 10/5/10 | Nuc | 48 | 9 (18%) | 3 (33%) | 1 (11%) |
| | 50/20/10 | Nuc+Cyt | 30 | 12 (40%) | 1 (8.3%) | 0 |
| | | | | | | |
| *mdx* | 10/10/10 | Nuc | 103 | 29 (28%) | 4 (14%) | 1 (3.4%) |
| | | Nuc+Cyt | 150 | 58 (39%) | 7 (12%) | 4 (6.9%) |
| | 50/20/10 | Nuc | 30 | 14 (47%) | 2 (6.7%) | 0 |
| | | Nuc+Cyt | 120 | 23 (19%) | 9 (39%) | 2(8.9%) |

**Table S3. Sequences of the target site (Dmd exon 23) and 32 potential off-target (OT) sites in the mouse genome.**

| **#** | **Target(20nt)**-PAM(3nt) | **SEQ ID NO:** | **locus (mm10)** | **score** | **mismatches** | **UCSC gene** |
|---|---|---|---|---|---|---|
| **DMD** | | 150 | **chrX:83803318-83803340** | 37 | | |
| **OT-01** | | 151 | **chr16:53976196-53976218** | 5.5 | 2MMs [2:19] | |
| **OT-02** | | 152 | **chr16:58084165-58084187** | 4.2 | 2MMs [2:12] | |
| **OT-03** | | 153 | **chr2: 2606863 7-26068659** | 3.1 | 2MMs [4:13] | |
| **OT-04** | | 154 | **chr17:85542328-85542350** | 2.6 | 2MMs [3:18] | |
| **OT-05** | | 155 | **chr5:28127468-28127490** | 2.3 | 3MMs [4:5:10] | |
| **OT-06** | | 156 | **chr2:44769953-44769975** | 2.1 | 2MMs [6:19] | |
| **OT-07** | | 157 | **chr14:93068307-93068329** | 2 | 2MMs [10:13] | |
| **OT-08** | | 158 | **chr9:95136798-95136820** | 1.5 | 3MMs [1:4:20] | |
| **OT-09** | | 159 | **chrX:45387898-45387920** | 1.5 | 3MMs [2:6:10] | |
| **OT-10** | | 160 | **chr5:38571962-38571984** | 1.4 | 2MMs [11:12] | |
| **OTE-01** | | 161 | **chrX:169303124-169303146** | 0.6 | 3MMs [1:10:18] | NM_178754 |
| **OTE-02** | | 162 | **chr6:78381061-78381083** | 0.6 | 3MMs [8:12:20] | NM_011259 |
| **OTE-03** | | 163 | **chr16:10960046-10960068** | 0.5 | 3MMs [8:13:20] | NM_019980 |
| **OTE-04** | | 164 | **chr6:129053832-129053854** | 0.5 | 4MMs [1:2:6:9] | NR_024262 |
| **OTE-05** | | 165 | **chr2:118748097-118748119** | 0.4 | 4MMs [1:2:6:13] | NR_030716 |
| **OTE-06** | | 166 | **chr1:90830366-90830388** | 0.4 | 4MMs [2:9:10:20] | NM_001243008 |
| **OTE-07** | | 167 | **chr3:28668648-28668670** | 0.3 | 3MMs [2:13:15] | NM_026910 |
| **OTE-08** | | 168 | **chr8:109728362-109728384** | 0.3 | 4MMs [1:8:12:20] | NM_001080930 |
| **OTE-09** | | 169 | **chr2:76705331-76705353** | 0.3 | 4MMs [2:4:10:18] | NM 028004 |
| **OTE-10** | | 170 | **chr2:126908236-126908258** | 0.3 | 4MMs [2:5:9:19] | NM_023220 |
| **OTE-11** | | 171 | **chr9:88581220-88581242** | 0.3 | 4MMs [2:6:10:13] | NM_001034906 |
| **OTE-12** | | 172 | **chr4:32723618-32723640** | 0.3 | 4MMs [3:5:17:20] | NM_001081392 |
| **OTE-13** | | 173 | **chr19:26696234-26696256** | 0.2 | 3MMs [5:13:16] | NM_011416 |
| **OTE-14** | | 174 | **chr16:10170610-10170632** | 0.2 | 4MMs [2:7:11:13] | NM_026594 |
| **OTE-15** | | 175 | chr1:139447127-139447149 | 0.2 | 4MMs [6:9:10:13] | NM_172643 |
| **OTE-16** | | 176 | **chr5:134295459-134295481** | 0.2 | 4MMs [4:10:13:17] | NM_001080748 |
| **OTE-17** | | 177 | **chr2:79672854-79672876** | 0.1 | 3MMs [13:15:17] | NM_080558 |
| **OTE-18** | | 178 | **chr10:20988803-20988825** | 0.1 | 4MMs [2:4:14:19] | NM_026203 |
| **OTE-19** | | 179 | **chr1:161837651-161837673** | 0.1 | 4MMs [2:5:13:16] | NM_172645 |
| **OTE-20** | | 180 | **chr16:48977882-48977904** | 0.1 | 4MMs [3:4:16:17] | NM_001110017 |
| **OTE-21** | | 181 | **chr7:45425042-45425064** | 0.1 | 4MMs [4:9:12:19] | NM_011304 |
| **OTE-22** | | 182 | **chr11:60875710-60875732** | 0.1 | 4MMs [6:7:12:15] | NM_001168507 |

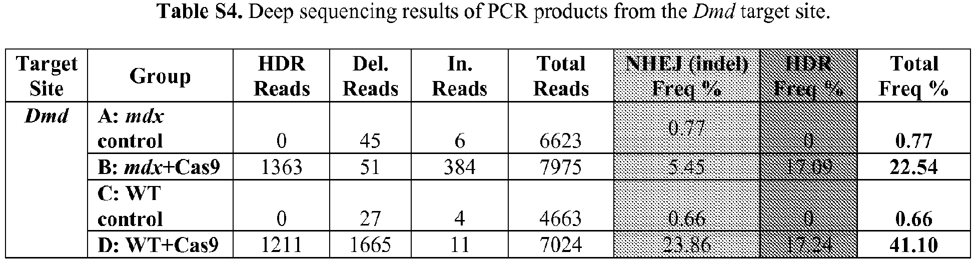

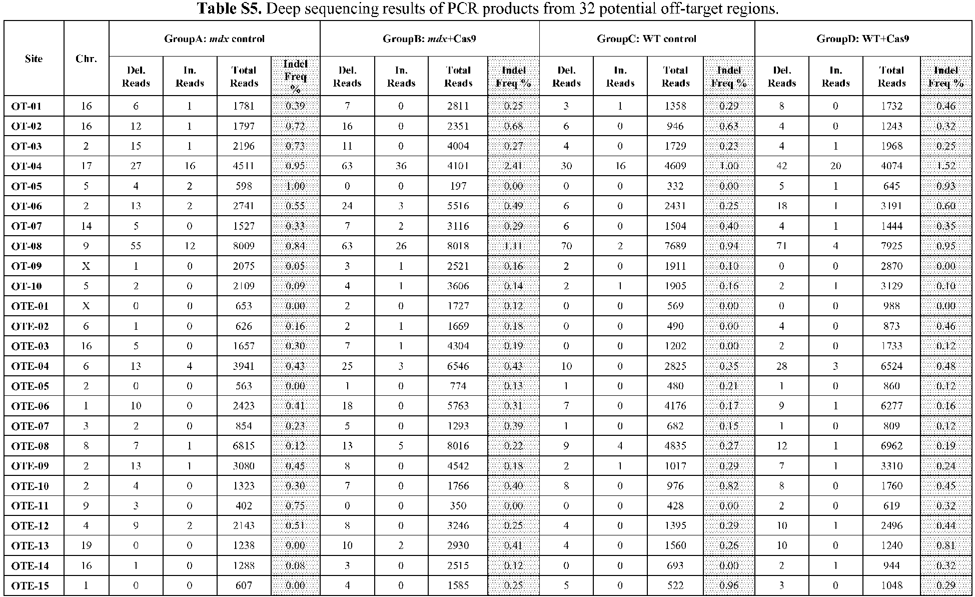

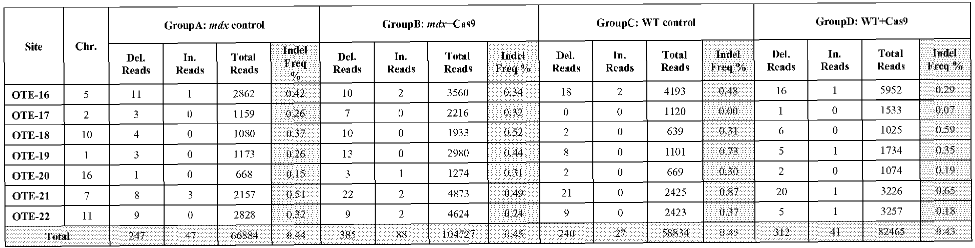

### VI. References

Aartsma-Rus, Methods MolBiol 867:97-116, 2012.
Angel et al., Mol. Cell. Biol., 7:2256, 1987a.
Angel et al., Cell, 49:729, 1987b.
Baichwal and Sugden, In: Gene Transfer, Kucherlapati (Ed), NY, Plenum Press, 117-148, 1986.
Banerji et al., Cell, 27(2 Pt 1):299-308, 1981.
Banerji et al., Cell, 33(3):729-740, 1983.
Barnes et al., J. Biol. Chem., 272(17):11510-7, 1997.
Benvenisty and Neshif, Proc. Natl. Acad. Sci. USA, 83:9551-9555, 1986.
Berkhout et al., Cell, 59:273-282, 1989.
Bhavsar et al., Genomics, 35(1):11-23, 1996.
Bikard et al., Nucleic Acids Res. 41(15): 7429-7437, 2013.
Blanar et al., EMBO J., 8:1139, 1989.
Bodine and Ley, EMBO J., 6:2997, 1987.
Boshart et al., Cell, 41:521, 1985.
Bosze et al., EMBO J., 5(7):1615-1623, 1986.
Braddock et al., Cell, 58:269, 1989.
Bulla and Siddiqui, J. Virol., 62:1437, 1986.
Campbell and Villarreal, Mol. Cell. Biol., 8:1993, 1988.
Campere and Tilghman, Genes and Dev., 3:537, 1989.
Campo et al., Nature, 303:77, 1983.
Celander and Haseltine, J. Virology, 61:269, 1987.
Celander et al., J. Virology, 62:1314, 1988.
Chandler et al., Cell, 33:489, 1983.
Chang et al., Mol. Cell. Biol., 9:2153, 1989.
Chang et al., Stem Cells., 27:1042-1049, 2009.
Chatterjee et al., Proc. Natl. Acad. Sci. USA, 86:9114, 1989.
Chen and Okayama, Mol. Cell Biol., 7:2745-2752, 1987.
Cho et al., Nat. Biotechnol. 31(3): 230-232, 2013.
Choi et al., Cell, 53:519, 1988.
Coffin, In: Virology, Fields et al. (Eds.), Raven Press, NY, 1437-1500, 1990.
Cohen et al., J. Cell. Physiol., 5:75, 1987.
Cong et al., Science 339, 819-823, 2013.
Cong et al., Sciencexpress, 2013.
Costa et al., Mol. Cell. Biol., 8:81, 1988.
Couch et al., Am. Rev. Resp. Dis., 88:394-403, 1963.
Coupar et al., Gene, 68:1-10, 1988.
Cripe et al., EMBO J., 6:3745, 1987.
Culotta and Hamer, Mol. Cell. Biol., 9:1376, 1989.
Dandolo et al., J. Virology, 47:55-64, 1983.
De Villiers et al., Nature, 312(5991):242-246, 1984.
Deans et al., Cell., 130:363-372, 2007.
Deschamps et al., Science, 230:1174-1177, 1985.
Donnelly et al., J. Gen. Virol. 82, 1027-1041, 2001.
Duan et al., Cell Res, published online (10.1038/cr.2014.87), 2014.
Dubensky et al., Proc. Natl. Acad. Sci. USA, 81:7529-7533, 1984.
Edbrooke et al., Mol. Cell. Biol., 9:1908, 1989.
Edlund et al., Science, 230:912-916, 1985.
EP 0273085
Fairclough et al., Nat Rev Genet 14, 373-378, 2013.
Fechheimer et al., Proc Natl. Acad. Sci. USA, 84:8463-8467, 1987.
Feng and Holland, Nature, 334:6178, 1988.
Ferkol et al., FASEB J., 7:1081-1091, 1993.
Firak and Subramanian, Mol. Cell. Biol., 6:3667, 1986.
Foecking and Hofstetter, Gene, 45(1):101-105, 1986.
Fraley et al., Proc Natl. Acad. Sci. USA, 76:3348-3352, 1979
Franz et al., Cardoscience, 5(4):235-43, 1994.
Friedmann, Science, 244:1275-1281, 1989.
Fu et al., Nat Biotechnol 31, 822-826, 2013.
Fu et al., Nat. Biotechnol., 2013.
Fujita et al., Cell, 49:357, 1987.
Ghosh and Bachhawat, In: Liver Diseases, Targeted Diagnosis and Therapy Using Specific
Receptors and Ligands, Wu et al. (Eds.), Marcel Dekker, NY, 87-104, 1991. Ghosh-Choudhury et al., EMBO J., 6:1733-1739, 1987.
Gibson et al., Nature methods. 6:343-345, 2009.
Gilles et al., Cell, 33:717, 1983.
Gjerdrum et al., J Mol Diagn 3, 105-110, 2001.
Gloss et al., EMBO J., 6:3735, 1987.
Godbout et al., Mol. Cell. Biol., 8:1169, 1988.
Gomez-Foix et al., J. Biol. Chem., 267:25129-25134, 1992.
Goodbourn and Maniatis, Proc. Natl. Acad. Sci. USA, 85:1447, 1988.
Goodbourn et al., Cell, 45:601, 1986.
Gopal, Mol. Cell Biol., 5:1188-1190, 1985.
Gopal-Srivastava et al., J. Mol. Cell. Biol., 15(12):7081-90, 1995.
Graham and Prevec, In: *Methods in Molecular Biology: Gene Transfer and Expression*
*Protocol,* Murray (Ed.), Humana Press, Clifton, NJ, 7:109-128, 1991.
Graham and van der Eb, Virology, 52:456-467, 1973.
Graham et al., J. Gen. Virol., 36:59-72, 1977.
Greene et al., Immunology Today, 10:272, 1989
Grosschedl and Baltimore, Cell, 41:885, 1985.
Grunhaus and Horwitz, Seminar in Virology, 3:237-252, 1992.
Harland and Weintraub, J. Cell Biol., 101:1094-1099, 1985.
Harris et al., Biomaterials 31, 998-1006, 2010.
Haslinger and Karin, Proc. Natl. Acad. Sci. USA, 82:8572, 1985.
Hauber and Cullen, J. Virology, 62:673, 1988.
Hen et al., Nature, 321:249, 1986.
Hensel et al., Lymphokine Res., 8:347, 1989.
Hermonat and Muzycska, Proc. Nat'l Acad. Sci. USA, 81:6466-6470, 1984.
Herr and Clarke, Cell, 45:461, 1986.
Hersdorffer et al., DNA Cell Biol., 9:713-723, 1990.
Herz and Gerard, Proc. Nat'l. Acad. Sci. USA 90:2812-2816, 1993.
Hirochika et al., J. Virol., 61:2599, 1987.
Holbrook et al., Virology, 157:211, 1987.
Horlick and Benfield, Mol. Cell. Biol., 9:2396, 1989.
Horwich et al., J. Virol., 64:642-650, 1990.
Hsu et al., Cell 157, 1262-1278, 2014.
Hsu et al., Nat. Biotechnol., 2013.
Hsu et al., Natl Biotechnol. 31:827-832, 2013
Huang et al., Cell, 27:245, 1981*.*
Hug et al., Mol. Cell. Biol., 8:3065, 1988.
Hwang et al., Mol. Cell. Biol., 10:585, 1990.
Imagawa et al., Cell, 51:251, 1987.
Imbra and Karin, Nature, 323:555, 1986.
Imler et al., Mol. Cell. Biol., 7:2558, 1987.
Imperiale and Nevins, Mol. Cell. Biol., 4:875, 1984.
Jakobovits et al., Mol. Cell. Biol., 8:2555, 1988.
Jameel and Siddiqui, Mol. Cell. Biol., 6:710, 1986.
Jaynes et al., Mol. Cell. Biol., 8:62, 1988.
Jinek et al., Science 337, 816-821, 2012.
Johnson et al., Mol. Cell. Biol., 9:3393, 1989.
Jones and Shenk, Cell, 13:181-188, 1978.
Kadesch and Berg, Mol. Cell. Biol., 6:2593, 1986.
Kaneda et al., Science, 243:375-378, 1989.
Karin et al., Mol. Cell. Biol., 7:606, 1987.
Karlsson et al., EMBO J., 5:2377-2385, 1986.
Katinka et al., Cell, 20:393, 1980.
Kato et al., J Biol Chem., 266(6):3361-3364, 1991.
Kawamoto et al., Mol. Cell. Biol., 8:267, 1988.
Kelly et al., J. Cell Biol., 129(2):383-96, 1995.
Kiledjian et al., Mol. Cell. Biol., 8:145, 1988.
Kimura et al., Dev. Growth Differ., 39(3):257-65, 1997.
Klamut et al., Mol. Cell. Biol., 10:193, 1990.
Klein et al., Nature, 327:70-73, 1987.
Koch et al., Mol. Cell. Biol., 9:303, 1989.
Kodippili et al., PLos One9, e88280, 2014.
Kormann et al., Nat Biotechnol 29, 154-157, 2011.
Kriegler and Botchan, In: Eukaryotic Viral Vectors, Gluzman (Ed.), Cold Spring Harbor: Cold Spring Harbor Laboratory, NY, 1982.
Kriegler and Botchan, Mol. Cell. Biol., 3:325, 1983.
Kriegler et al., Cell, 38:483, 1984.
Kriegler et al., Cell, 53:45, 1988.
Kuhl et al., Cell, 50:1057, 1987.
Kunz et al., Nucl. Acids Res., 17:1121, 1989.
Kuscu et al., NatBiotechnol 32, 677-683, 2014.
L. Zangi et al., Nat Biotechnol 31, 898-907, 2013.
LaPointe et al., Hypertension, 27(3):715-22, 1996
LaPointe et al., J. Biol. Chem., 263(19):9075-8, 1988.
Larsen et al., Proc. Natl. Acad. Sci. USA., 83:8283, 1986.
Laspia et al., Cell, 59:283, 1989.
Latimer et al., Mol. Cell. Biol., 10:760, 1990.
Le Gal La Salle et al., Science, 259:988-990, 1993.
Lee et al., Nature, 294:228, 1981.
Levinson et al., Nature, 295:79, 1982.
Levrero et al., Gene, 101:195-202, 1991.
Li et al., J. Biol. Chem., 273:34970-34975, 1998.
Lin et al., Mol. Cell. Biol., 10:850, 1990.
Long et al., Science 345:1184-1188, 2014.
Luria et al., EMBO J., 6:3307, 1987.
Lusky and Botchan, Proc. Natl. Acad. Sci. USA, 83:3609, 1986.
Lusky et al., Mol. Cell. Biol., 3:1108, 1983.
Majors and Varmus, Proc. Natl. Acad. Sci. USA, 80:5866, 1983.
Mali et al., Science 339, 823-826, 2013a.
Mali et al., Nat Methods 10, 957-963, 2013b.
Mali et al, Nat. Biotechnol. 31:833-838, 2013b.
Mann et al., Cell, 33:153-159, 1983.
Markowitz et al., J. Virol., 62:1120-1124, 1988.
McNeall et al., Gene, 76:81, 1989.
Miksicek et al., Cell, 46:203, 1986.
Montarras et al., Science 309, 2064-2067, 2005.
Mordacq and Linzer, Genes and Dev., 3:760, 1989.
Moreau et al., Nucl. Acids Res., 9:6047, 1981.
Moss et al., J. Gen. Physiol., 108(6):473-84, 1996.
Muesing et al., Cell, 48:691, 1987.
Murphy et al., Development 138, 3625-3637, 2011.
Nathwani et al., Mol Ther 19, 876-885, 2011.
Nicholson et al., J Neuro Sci 94, 1250136, 1989.
Nicolas and Rubinstein, In: Vectors: A survey of molecular cloning vectors and their uses, Rodriguez and Denhardt (Eds.), Stoneham: Butterworth, 494-513, 1988.
Nicolau and Sene, Biochim. Biophys. Acta, 721:185-190, 1982.
Nicolau et al., Methods Enzymol., 149:157-176, 1987.
Ondek et al., EMBO J., 6:1017, 1987.
Ornitz et al., Mol. Cell. Biol., 7:3466, 1987.
Osborn et al., Mol Ther 21, 1151-1159, 2013.
Ousterout et al., Mol Ther 21, 1718-1726, 2013.
Palmiter et al., Cell, 29:701, 1982.
Palmiter et al., Nature, 300:611, 1982.
Paskind et al., Virology, 67:242-248, 1975.
Pattanayak et al., Nat Biotechnol 31, 839-843, 2013.
Pech et al., Mol. Cell. Biol., 9:396, 1989.
Peng et al., Biochem Biophys Res Commun 338, 1490-1498, 2005.
Perales et al., Proc. Natl. Acad. Sci. USA, 91(9):4086-4090, 1994.
Perez-Stable and Constantini, Mol. Cell. Biol., 10:1116, 1990.
Picard and Schaffner, Nature, 307:83, 1984.
Pigozzo et al., PLoS One 8, e72147, 2013.
Pinkert et al., Genes and Dev., 1:268, 1987.
Ponta et al., Proc. Natl. Acad. Sci. USA, 82:1020, 1985.
Porton et al., Mol. Cell. Biol., 10:1076, 1990.
Potter et al., Proc. Natl. Acad. Sci. USA, 81:7161-7165, 1984.
Queen and Baltimore, Cell, 35:741, 1983.
Quinn et al., Mol. Cell. Biol., 9:4713, 1989.
Racher et al., Biotech. Techniques, 9:169-174, 1995.
Ragot et al., Nature, 361:647-650, 1993.
Redondo et al., Science, 247:1225, 1990.
Reisman and Rotter, Mol. Cell. Biol., 9:3571, 1989.
Renan, Radiother. Oncol., 19:197-218, 1990.
Resendez Jr. et al., Mol. Cell. Biol., 8:4579, 1988.
Rich et al., Hum. Gene Ther., 4:461-476, 1993.
Ridgeway, In: Vectors: A survey of molecular cloning vectors and their uses, Stoneham: Butterworth, 467-492, 1988.
Ripe et al., Mol. Cell. Biol., 9:2224, 1989.
Rippe et al., Mol. Cell Biol., 10:689-695, 1990.
Rittling et al., Nuc. Acids Res., 17:1619, 1989.
Rosen et al., Cell, 41:813, 1988.
Rosenfeld et al., Cell, 68:143-155,1992.
Rosenfeld et al., Science, 252:431-434,1991.
Roux et al., Proc. Natl. Acad. Sci. USA, 86:9079-9083, 1989.
Sage et al., Cell. Div., 5:17-1028-5-17, 2010.
Sakai et al., Genes and Dev., 2:1144, 1988.
Satake et al., J. Virology, 62:970, 1988.
Schaffner et al., J. Mol. Biol., 201:81, 1988.
Schmidt & Grimm, Biotechnology Journal 10:258-272, 2015.
Schwank et al., Cell Stem Cell 13, 653-658, 2013.
Searle et al., Mol. Cell. Biol., 5:1480, 1985.
Senis et al., Biotechnology Journal 9:1402-1412, 2014.
Sharp and Marciniak, Cell, 59:229, 1989.
Shaul and Ben-Levy, EMBO J., 6:1913, 1987.
Sherman et al., Mol. Cell. Biol., 9:50, 1989.
Sleigh and Lockett, J. EMBO, 4:3831, 1985.
Spalholz et al., Cell, 42:183, 1985.
Spandau and Lee, J. Virology, 62:427, 1988.
Spandidos and Wilkie, EMBO J., 2:1193, 1983.
Stephens and Hentschel, Biochem. J., 248:1, 1987.
Sternberg et al., Nature, 2014.
Stratford-Perricaudet and Perricaudet, In: Human Gene Transfer, Cohen-Haguenauer and Boiron (Eds.), John Libbey Eurotext, France, 51-61, 1991.
Stratford-Perricaudet et al., Hum. Gene. Ther., 1:241-256, 1990.
Stuart et al., Nature, 317:828, 1985.
Sullivan and Peterlin, Mol. Cell. Biol., 7:3315, 1987.
Swartzendruber and Lehman, J. Cell. Physiology, 85:179, 1975.
Takebe et al., Mol. Cell. Biol., 8:466, 1988.
Tavemier et αl., Nature, 301:634, 1983.
Taylor and Kingston, Mol. Cell. Biol., 10:165, 1990a.
Taylor and Kingston, Mol. Cell. Biol., 10:176, 1990b.
Taylor et al., J. Biol. Chem., 264:15160, 1989.
Temin, In: Gene Transfer, Kucherlapati (Ed.), NY, Plenum Press, 149-188, 1986.
Thiesen et al., J. Virology, 62:614, 1988.
Top et al., J. Infect. Dis., 124:155-160, 1971.
Tronche et al., Mol. Biol. Med., 7:173, 1990.
Trudel and Constantini, Genes and Dev. 6:954, 1987.
Tur-Kaspa et al., Mol. Cell Biol., 6:716-718, 1986.
Tyndell et al., Nuc. Acids. Res., 9:6231, 1981.
Urnov et al., Nature 435, 646-651, 2005.
Van Deutekom and Van Ommen, Nat Rev Genet 4, 774-783, 2003.
van Putten et al., J Mol Cell Cardiol 69:17-23, 2014.
Vannice and Levinson, J. Virology, 62:1305, 1988.
Varmus et al., Cell, 25:23-36, 1981.
Vasseur et al., Proc Natl. Acad. Sci. U.S.A., 77:1068, 1980.
Wagner et al., Proc. Natl. Acad. Sci. USA, 87(9):3410-3414, 1990.
Wang and Calame, Cell, 47:241, 1986.
Wang et al., Cell, 153:910-910, 2013.
Weber et al., Cell, 36:983, 1984.
Weinberger et al. Mol. Cell. Biol., 8:988, 1984.
Winoto and Baltimore, Cell, 59:649, 1989.
Wong et al., Gene, 10:87-94, 1980.
Worton et al., Annu Rev Genet 22, 601-629, 1988.
Wu and Wu, Adv. Drug Delivery Rev., 12:159-167, 1993.
Wu and Wu, Biochemistry, 27:887-892, 1988.
Wu and Wu, J. Biol. Chem., 262:4429-4432, 1987.
Wu et al., Cell Stem Cell 13, 659-662, 2013.
Wu et al., Nat Biotechnol 32, 670-676, 2014.
Yamauchi-Takihara et al., Proc. Natl. Acad. Sci. USA, 86(10):3504-3508, 1989.
Yang et al., Proc. Natl. Acad. Sci. USA, 87:9568-9572, 1990.
Yen et al., Dev Biol, published online (10.1016/j.ydbio.2014.06.017), 2014.
Yin et al., Nat Biotechnol 32, 551-553, 2014.
Yin et al., Physiol Rev 93, 23-67, 2013.
Yutzey et al. Mol. Cell. Biol., 9:1397, 1989.
Zelenin et al., FEBS Lett., 280:94-96, 1991.
Ziober and Kramer, J. Bio. Chem., 271(37):22915-22922, 1996.

## Claims

1. Cas9 or an expression vector, naked plasmid DNA or chemically-modified mRNA, coding therefor; and a single DMD guide RNA or an expression vector coding therefor; for use in a method of treating Duchenne muscular dystrophy (DMD) in a subject, the method comprising contacting a cell in said subject with the Cas9 and the single DMD guide RNA (gRNA), that, once expressed, form a Cas9-gRNA complex to destroy a splice acceptor site upstream of exon 51 of the DMD gene, wherein the single DMD gRNA comprises a spacer RNA and a tracrRNA; wherein the spacer RNA consists of 20 nucleotides; wherein the splice acceptor site comprises the sequence of SEQ ID NO: 28; and thereby rescuing the expression of the DMD gene by exon skipping; wherein the Cas9 and and/or DMD guide RNA are delivered using an adeno-associated viral (AAV) vector.

2. The Cas9 or an expression vector, naked plasmid DNA or chemically-modified mRNA, coding therefor; and the single DMD guide RNA or an expression vector coding therefor; for use in a method according to claim 1, wherein said cell is a muscle cell, a satellite cell, or an iPSC/iCM.

3. The Cas9 or an expression vector, naked plasmid DNA or chemically-modified mRNA, coding therefor; and the single DMD guide RNA or an expression vector coding therefor; for use in a method according to claim 1, wherein the method further comprises contacting said cell with a single-stranded DMD oligonucleotide to effect homology directed repair.

4. The Cas9 or an expression vector, naked plasmid DNA or chemically-modified mRNA, coding therefor; and the single DMD guide RNA or an expression vector coding therefor; for use in a method according to any of claims 1-3, wherein said Cas9 and/or DMD guide RNA are:
(a) delivered directly to a muscle tissue, and optionally wherein said muscle tissue is tibialis anterior, quadricep, soleus, diaphragm or heart; or
(b) delivered systemically.

5. The Cas9 or an expression vector, naked plasmid DNA or chemically-modified mRNA, coding therefor; and the single DMD guide RNA or an expression vector coding therefor; for use in a method according to claim 1, wherein:
(a) said subject exhibits normal dystrophin-positive myofibers and/or mosaic dystrophin positive myofibers containing centralized nuclei after said contacting occurs;
(b) said subject exhibits a decreased serum CK level as compared to a serum CK level prior to contacting; or
(c) said subject exhibits improved grip strength as compared to grip strength prior to contacting.

6. The Cas9 or an expression vector, naked plasmid DNA or chemically-modified mRNA, coding therefor; and the single DMD guide RNA or an expression vector coding therefor; for use in a method according to claim 1, wherein the correction is permanent skipping of a mutant exon.

7. The Cas9 or an expression vector, naked plasmid DNA or chemically-modified mRNA, coding therefor; and the single DMD guide RNA or an expression vector coding therefor; for use in a method according to claim 1, wherein the method further comprises designing a dystrophin gene target based on reference to a Duchenne mutation database.

8. The Cas9 or an expression vector, naked plasmid DNA or chemically-modified mRNA, coding therefor; and the single DMD guide RNA or an expression vector coding therefor; for use in a method according to claim 1, wherein the DMD guide RNA targets a genomic locus next to a protospacer adjacent motif associated with the exon containing a mutation in the dystrophin gene.

9. The Cas9 or an expression vector, naked plasmid DNA or chemically-modified mRNA, coding therefor; and the single DMD guide RNA or an expression vector coding therefor; for use in a method according to claim 1, wherein the exon is exon 51.

10. The Cas9 or an expression vector, naked plasmid DNA or chemically-modified mRNA, coding therefor; and the single DMD guide RNA or an expression vector coding therefor; for use in a method according to claim 1, wherein the AAV vector is an AAV9 vector.

## Patentansprüche

1. Cas9 oder ein Expressionsvektor, nackte Plasmid-DNA oder chemisch modifizierte mRNA, die dafür kodieren; und eine einzelne DMD-Leit-RNA oder ein Expressionsvektor, die dafür kodieren; zur Verwendung in einem Verfahren zum Behandeln von Duchenne-Muskeldystrophie (DMD) bei einem Subjekt, wobei das Verfahren ein Inberührungbringen einer Zelle in dem Subjekt mit dem Cas9 und der einzelnen DMD-Leit-RNA (guide RNA - gRNA) umfasst, die, sobald sie exprimiert sind, einen Cas9-gRNA-Komplex ausbilden, um eine Spleiß-Akzeptorstelle stromaufwärts von Exon 51 des DMD-Gens zu zerstören, wobei die einzelne DMD-gRNA eine Spacer-RNA und eine tracrRNA umfasst; wobei die Spacer-RNA aus 20 Nukleotiden besteht; wobei die Spleiß-Akzeptorstelle die Sequenz von SEQ ID NO: 28 umfasst; und wobei dadurch die Expression des DMD-Gens durch Exon-Überspringen gerettet wird; wobei das Cas9 und/oder die DMD-Leit-RNA unter Verwendung eines Adeno-assoziierten-viralen(AAV)-Vektors abgegeben wird.

2. Cas9 oder ein Expressionsvektor, nackte Plasmid-DNA oder chemisch modifizierte mRNA, die dafür kodieren; und die einzelne DMD-Leit-RNA oder ein Expressionsvektor, die dafür kodieren; zur Verwendung in einem Verfahren nach Anspruch 1, wobei die Zelle eine Muskelzelle, eine Satellitenzelle oder eine iPSC/iCM ist.

3. Cas9 oder ein Expressionsvektor, nackte Plasmid-DNA oder chemisch modifizierte mRNA, die dafür kodieren; und die einzelne DMD-Leit-RNA oder ein Expressionsvektor, die dafür kodieren; zur Verwendung in einem Verfahren nach Anspruch 1, wobei das Verfahren ferner das Inberührungbringen der Zelle mit einem einzelsträngigen DMD-Oligonukleotid umfasst, um eine homologiegerichtete Reparatur zu bewirken.

4. Cas9 oder ein Expressionsvektor, nackte Plasmid-DNA oder chemisch modifizierte mRNA, die dafür kodieren; und die einzelne DMD-Leit-RNA oder ein Expressionsvektor, die dafür kodieren; zur Verwendung in einem Verfahren nach einem der Ansprüche 1-3, wobei das Cas9 und/oder die DMD-Leit-RNA:
(a) an ein Muskelgewebe direkt abgegeben werden, und optional wobei das Muskelgewebe Tibialis anterior, Quadrizeps, Soleus, Diaphragma oder Herz ist; oder
(b) systemisch abgegeben werden.

5. Cas9 oder ein Expressionsvektor, nackte Plasmid-DNA oder chemisch modifizierte mRNA, die dafür kodieren; und die einzelne DMD-Leit-RNA oder ein Expressionsvektor, die dafür kodieren; zur Verwendung in einem Verfahren nach Anspruch 1, wobei:
(a) das Subjekt normale Dystrophin-positive Muskelfasern und/oder Mosaik-Dystrophin-positive Muskelfasern vorweist, die zentralisierte Kerne enthalten, nachdem das Inberührungbringen erfolgt;
(b) das Subjekt einen verringerten Serum-CK-Spiegel im Vergleich zu einem Serum-CK-Spiegel vor dem Inberührungbringen vorweist; oder
(c) das Subjekt eine verbesserte Griffstärke im Vergleich zu der Griffstärke vor dem Inberührungbringen vorweist.

6. Cas9 oder ein Expressionsvektor, nackte Plasmid-DNA oder chemisch modifizierte mRNA, die dafür kodieren; und die einzelne DMD-Leit-RNA oder ein Expressionsvektor, die dafür kodieren; zur Verwendung in einem Verfahren nach Anspruch 1, wobei die Korrektur permanentes Überspringen eines mutierten Exons ist.

7. Cas9 oder ein Expressionsvektor, nackte Plasmid-DNA oder chemisch modifizierte mRNA, die dafür kodieren; und die einzelne DMD-Leit-RNA oder ein Expressionsvektor, die dafür kodieren; zur Verwendung in einem Verfahren nach Anspruch 1, wobei das Verfahren ferner ein Aufbauen eines Dystrophin-Gen-Ziels basierend auf einer Bezugnahme auf eine Duchenne-Mutationsdatenbank umfasst.

8. Cas9 oder ein Expressionsvektor, nackte Plasmid-DNA oder chemisch modifizierte mRNA, die dafür kodieren; und die einzelne DMD-Leit-RNA oder ein Expressionsvektor, die dafür kodieren; zur Verwendung in einem Verfahren nach Anspruch 1, wobei die DMD-Leit-RNA auf einen genomischen Locus neben einem Protospacer-Nachbarmotiv zielt, das mit dem Exon verknüpft ist, das eine Mutation in dem Dystrophin-Gen enthält.

9. Cas9 oder ein Expressionsvektor, nackte Plasmid-DNA oder chemisch modifizierte mRNA, die dafür kodieren; und die einzelne DMD-Leit-RNA oder ein Expressionsvektor, die dafür kodieren; zur Verwendung in einem Verfahren nach Anspruch 1, wobei das Exon Exon 51 ist.

10. Cas9 oder ein Expressionsvektor, nackte Plasmid-DNA oder chemisch modifizierte mRNA, die dafür kodieren; und die einzelne DMD-Leit-RNA oder ein Expressionsvektor, die dafür kodieren; zur Verwendung in einem Verfahren nach Anspruch 1, wobei der AAV-Vektor ein AAV9-Vektor ist.

## Revendications

1. Cas9 ou un vecteur d'expression, un ADN plasmidique nu ou un ARNm chimiquement modifié, codant pour celui-ci ; et un seul ARN guide DMD ou un vecteur d'expression codant pour celui-ci ; destiné à être utilisé dans un procédé de traitement de la maladie de Duchenne de Boulogne (DMD) chez un sujet, le procédé comprenant la mise en contact d'une cellule dudit sujet avec le Cas9 et l'ARN guide DMD unique (ARNg), qui, une fois exprimés, forment un complexe Cas9-ARNg pour détruire un site accepteur d'épissage en amont de l'exon 51 du gène DMD, l'ARNg DMD unique comprenant un ARN espaceur et un ARNtracr ; l'ARN espaceur consistant en 20 nucléotides ; le site accepteur d'épissage comprenant la séquence de SEQ ID NO : 28 ; et ainsi sauvant l'expression du gène DMD par saut d'exon ; l'ARN guide Cas9 et/ou DMD étant administré à l'aide d'un vecteur viral adéno-associé (AAV).

2. Cas9 ou un vecteur d'expression, un ADN plasmidique nu ou un ARNm chimiquement modifié, codant pour celui-ci ; et l'ARN guide DMD unique ou un vecteur d'expression codant pour celui-ci ; destiné à être utilisé dans un procédé selon la revendication 1, ladite cellule étant une cellule musculaire, une cellule satellite ou une CSPi/MCi.

3. Cas9 ou un vecteur d'expression, un ADN plasmidique nu ou un ARNm chimiquement modifié, codant pour celui-ci ; et l'ARN guide DMD unique ou un vecteur d'expression codant pour celui-ci ; destiné à être dans un procédé selon la revendication 1, le procédé comprenant en outre la mise en contact de ladite cellule avec un oligonucléotide DMD monocaténaire afin d'effectuer une réparation dirigée par homologie.

4. Cas9 ou un vecteur d'expression, un ADN plasmidique nu ou un ARNm chimiquement modifié, codant pour celui-ci ; et l'ARN guide DMD unique ou un vecteur d'expression codant pour celui-ci ; destiné à être utilisé dans un procédé selon l'une quelconque des revendications 1 à 3, lesdits ARN guide Cas9 et/ou DMD étant :
(a) administrés directement à un tissu musculaire, et éventuellement ledit tissu musculaire étant le tibial antérieur, le quadriceps, le soléaire, le diaphragme ou le cœur ; ou
(b) administrés de manière systémique.

5. Cas9 ou un vecteur d'expression, un ADN plasmidique nu ou un ARNm chimiquement modifié, codant pour celui-ci ; et l'ARN guide DMD unique ou un vecteur d'expression codant pour celui-ci ; destiné à être utilisé dans un procédé selon la revendication 1 :
(a) ledit sujet présentant des myofibres positives à la dystrophine normales et/ou des myofibres positives à la dystrophine en mosaïque contenant des noyaux centralisés après que ladite mise en contact s'est produite ;
(b) ledit sujet présentant un niveau de CK sérique diminué par rapport à un niveau de Ck sérique avant la mise en contact ; ou
(c) ledit sujet présentant une force de préhension améliorée par rapport à la force de préhension avant la mise en contact.

6. Cas9 ou un vecteur d'expression, un ADN plasmidique nu ou un ARNm chimiquement modifié, codant pour celui-ci ; et l'ARN guide DMD unique ou un vecteur d'expression codant pour celui-ci ; destiné à être utilisé dans un procédé selon la revendication 1, la correction étant le saut permanent d'un exon mutant.

7. Cas9 ou un vecteur d'expression, un ADN plasmidique nu ou un ARNm chimiquement modifié, codant pour celui-ci ; et l'ARN guide DMD unique ou un vecteur d'expression codant pour celui-ci ; destiné à être utilisé dans un procédé selon la revendication 1, le procédé comprenant en outre la conception d'un gène cible de la dystrophine en fonction d'une référence à une base de données de mutations de Duchenne.

8. Cas9 ou un vecteur d'expression, un ADN plasmidique nu ou un ARNm chimiquement modifié, codant pour celui-ci ; et l'ARN guide DMD unique ou un vecteur d'expression codant pour celui-ci ; destiné à être utlisé dans un procédé selon la revendication 1, l'ARN guide DMD ciblant un locus génomique à côté d'un motif adjacent au protospacer associé à l'exon contenant une mutation dans le gène de la dystrophine.

9. Cas9 ou un vecteur d'expression, un ADN plasmidique nu ou un ARNm chimiquement modifié, codant pour celui-ci ; et l'ARN guide DMD unique ou un vecteur d'expression codant pour celui-ci ; destiné à être utlisé dans un procédé selon la revendication 1, l'exon étant l'exon 51.

10. Cas9 ou un vecteur d'expression, un ADN plasmidique nu ou un ARNm chimiquement modifié, codant pour celui-ci ; et l'ARN guide DMD unique ou un vecteur d'expression codant pour celui-ci ; destiné à être utlisé dans un procédé selon la revendication 1, le vecteur AAV étant un vecteur AAV9.
